# EUROPEAN PATENT APPLICATION

(11) **EP 3 919 517 A1**
(43) Date of publication of application: **08.12.2021**
(21) Application number: 20749765.2
(22) Date of filing: 02.02.2020
(51) Int. Cl.: C07K 16/28, A61P 35/00, C07K 16/30, C12N 15/13, C12N 5/10, C12N 7/01

(54) **TCR FUSION PROTEIN AND CELL EXPRESSING TCR FUSION PROTEIN**

(30) Priority: 01.02.2019 CN 201910105233; 05.09.2019 CN 201910838439
(71) Applicant: CAFA THERAPEUTICS LIMITED, Dublin D01 AE27 (IE)
(72) Inventor: LI, Zonghai, Shanghai 200231 (CN); JIANG, Hua, Shanghai 200032 (CN); WANG, Peng, Shanghai 200231 (CN)
(74) Representative: dompatent von Kreisler Selting Werner - Partnerschaft von Patent- und Rechtsanwälten mbB
(86) International application number: PCT/CN2020/074169
(87) International publication number: WO 2020/156554

(57) **Abstract**

Disclosed is a T cell receptor (TCR) fusion protein (TFP). The fusion protein comprises a TCR subunit (or referred to as a TCR unit) and an antigen recognition unit that recognizes an antigen. The antigen is GPC3 or claudin 18.2. Also disclosed is a T cell containing the fusion protein, a pharmaceutical composition and an application method of using the fusion protein or the T cell to treat diseases such as cancer. The use of TFP or T cells not only inhibits the growth of tumor cells, but also releases fewer cytokines, thereby effectively reducing the possibility of cytokine storms.

## Description

### Technical field

The present invention relates to the field of immunotherapy. More particular, the present invention relates to a TCR fusion protein and cells expressing the TCR fusion protein.

### Background

Currently, immunotherapy has become indispensable in the clinical treatment of tumors. Drugs and solutions used in immunotherapy involve various stages of the body's immune system to recognize and attack cancer cells. Existing tumor immune drugs include following types: antibodies targeting cancer cells, adoptive cell therapy, oncolytic viruses, dendritic cell-related therapies, tumor vaccines at the DNA and protein levels, immune-activating cytokines, and other immune regulatory compounds, among which, antibody drugs against T cell checkpoint-inhibiting proteins and tumor antigen-specific T cell adoptive therapy have achieved breakthroughs in recent years and have attracted wide attention.

Genetic engineering based on T cells includes CAR-T and TCR-T. The former requires the construction of a chimeric antigen receptor, usually by connecting a single-chain antibody to the intracellular segment of CD3ζ through a hinge region and a transmembrane segment, and then the chimeric antigen receptor will be transduced into T cells through virus, the single-chain antibody binds to the antigen on the tumor cell surface so as to activate the intracellular signal of CD3ζ, which in turn causes the transduced T cells to kill tumor cells. The latter usually requires the transduction of a TCR that can specifically recognize the tumor antigen peptide/MHC complex into T cells, and then use these TCRs to form a new TCR complex with the CD3 subunit inside the T cell, so that the T cell can specifically target tumor cells, activate the signal pathway of the entire TCR complex, and achieve the purpose of killing tumor cells.

The method described herein is a new modification for T cells, which fuses a single-chain antibody with CD3ε or CD3γ, and then forms a new TCR complex with other subunits of the endogenous TCR complex, thereby both using the targeting properties of the single-chain antibody and the entire signaling pathway of the TCR complex.

### Summary of the invention

The purpose of the present invention is to provide a TCR fusion protein and T cells expressing the TCR fusion protein, so as to improve application effects of tumor immunotherapy in solid tumors.

The technical solution adopted in the present invention is as follows:
In the first aspect, the present invention provides a T cell receptor (TCR) fusion protein (TFP), the fusion protein comprising:
(a) a TCR subunit (or a TCR unit); and
(b) an antigen recognition unit that recognizes the antigen;
   the antigen is GPC3 or claudin 18.2;
   wherein the TCR subunit and the antigen recognition unit are operably connected.

In a specific embodiment, the TCR subunit comprises:
(i) at least a part of the extracellular domain of TCR, and
(ii) a TCR intracellular domain comprising a stimulatory domain derived from an intracellular signaling domain of CD3ε, CD3γ, CD3δ, TCRα, or TCRβ.

In a preferred embodiment, the TFP is incorporated into the TCR when expressed in T cells.

In a specific embodiment, the light chain LCDR1, LCDR2, and LCDR3 of the amino acid sequence of the antigen recognition unit that recognizes GPC3 are independently selected from or have 70-100% sequence identity with the light chain LCDR1, LCDR2, and LCDR3 shown in the following table, and/or the heavy chain HCDR1, HCDR2 and HCDR3 of the amino acid sequence of the antigen recognition unit that recognizes GPC3 are independently selected from or have 70-100% sequence identity with the heavy chain HCDR1, HCDR2 and HCDR3 shown in the following table;

| LCDR1 | LCDR2 | LCDR3 | HCDR1 | HCDR2 | HCDR3 |
|---|---|---|---|---|---|
| TGTSSDVGGYNYVS | GNSNRP S | QSYDSSLRV V | GFTFSSYAM H | AISGSGGSTYYADSVK G | DRRGSHADAFD V |
| TGTSSDVGGYNYVS | GNSNRP S | QSYDSSLRV V | GFTFSTYAMT | SISSSGESTYY ADSVKG | DRRGSHADAFD V |
| TGTSSDVGGYNYVS | GNSNRP S | QSYDSSLRV V | GFTFSTYAM A | EISSSGSRTYY ADSVKG | DRRGSHADAFD V |
| TGTSSDVGGYNYVS | GNSNRP S | QSYDSSLRV V | GFTFSTYAM A | AISMSGESTYYADSVK G | DRRGSHADAFD V |
| TGTSSDVGHKFPVS | KNLLRP S | QSYDSSLRV V | GFTFSSYAM H | AISSSGGSTYY ADSVKG | DRRGSHADAFD V |
| TGTSSDVGLMHNVS | KSSSRPS | QSYDSSLRV V | GFTFSSYAM H | AISSSGGSTYY ADSVKG | DRRGSHADAFD V |
| TGTSSDVGGYNYVS | KSSSRPS | QSYDSSLRV V | GFTFSSYAM H | AISSSGRSTYY ADSVEG | DRRGSHADALN V |
| RSSQSLVHSNGNTYL Q | KVSNRF S | SQSIYVPYT | DYEMH | AIHPGSGDTAYNQRFK G | FYSYAY |
| RSSQSLVHSNGNTYL Q | KVSNRF S | SQSIYVPYTF | DYEMH | AIHPGSGDTAYNQRFK G | FYSYAY |

or
the light chain LCDR1, LCDR2, and LCDR3 of the amino acid sequence of the antigen recognition unit that recognizes claudin 18.2 are independently selected from or have 70-100% sequence identity with the light chain LCDR1, LCDR2, and LCDR3 shown in the following table, and/or the heavy chain HCDR1, HCDR2 and HCDR3 of the amino acid sequence of the antigen recognition unit that recognizes claudin 18.2 are independently selected from or have 70-100% sequence identity with the heavy chain HCDR1, HCDR2 and HCDR3 shown in the following table;

| LCDR1 | LCDR2 | LCDR3 | HCDR1 | HCDR2 | HCDR3 |
|---|---|---|---|---|---|
| KSSQSLLNSGNQKNYLT | WASTRES | QNDYSYPL T | SYTMH | YINPSSGYTNYNQKFKD | IYYGNSF AY |
| SASSSISYMH | DTSKLAS | HQRSSYPYT | SYDIN | WIYPGDGSTKYNEKFKG | GGYRYDEAMDY |
| KSSQSLLNSGNQKNYLA | GASTRES | QNDHSYPLT | NYGMN | WINTNTGEPTY AEEFKG | FSYGNSF AY |
| KSSQSLFNSGNQKNYLT | WASTRES | QNAYSFPYT | SGYNWH | YIHYTGSTNYNPSLRS | IYNGNSFPY |
| KSSQSLLNSGNQKNYLT | WASTRES | QNDYSYPL T | SYTMH | YIDPSSGYTNYNQKFKD | IYYGNSF AY |
| KSSQSLLNSGNQKNYLT | WASTRES | QNDYSYPL T | SYTMH | YINP AS GYTNYNQKFKD | IYYGNSF AY |
| KSSQSLLNSGNQKNYLT | WASTRES | QNDYSYPL T | SYTMH | YINP AS GYTNYNQKFKD | IYYGNSF AY |
| KSSQSLLNSGNQKNYLT | WASTRES | QNDYSYPL T | SYTMH | YINP AS GYTNYNQKFKD | IYYGNSF AY |
| KSSQSLFNSGNQKNYLT | WASTRES | QNAYSFPYT | SGYNWH | YIHYTGSTNYNP ALRS | IYNGNSFPY |
| KSSQSLFNSGNQKNYLT | WASTRES | QNAYSFPYT | SGYNWH | YIHYTGSTNYNP ALRS | IYNGNSFPY. |

In a specific embodiment, the light chain variable region of the amino acid sequence of the antigen recognition unit that recognizes GPC3 is independently selected from or has 70-100% sequence identity with the light chain variable regions shown in the following table, and/or the heavy chain variable region of the amino acid sequence of the antigen recognition unit that recognizes GPC3 is independently selected from or has 70-100% sequence identity with the heavy chain variable region shown in the following table;

| VH | VL |
|---|---|
| QVQLQESGGGLVQPGRSLRLSCAASGFTFSSYAMHWVRQA PGKGLEWVSAISGSGGSTYYADSVKGRFTISRDNSKNTLYL QMNSLRAEDTAVYYCAKDRRGSHADAFDVWGQGTLVTVS S | QSALTQPPSASGSPGQSVTISCTGTSSDVGGYNYVSW YQQYPGKAPKLLIYGNSNRPSGVPDRFSGSKSGTSAS LAITGLQAEDGADYYCQSYDSSLRVVFGGGTKVTVL G |
| QVQLQESGGGLVQPGRSLRLSCAASGFTFSTYAMTWVRQA PGKGLEWVSSISSSGESTYYADSVKGRFTISRDNSKNTLYLQ MNSLRAEDTAVYYCAKDRRGSHADAFDVWGQGTLVTVSS | QSALTQPPSASGSPGQSVTISCTGTSSDVGGYNYVSW YQQYPGKAPKLLIYGNSNRPSGVPDRFSGSKSGTSAS LAITGLQAEDGADYYCQSYDSSLRVVFGGGTKVTVL G |
| QVQLQESGGGLVQPGRSLRLSCAASGFTFSTYAMAWVRQA PGKGLEWVSEISSSGSRTYYADSVKGRFTISRDNSKNTLYLQ MNSLRAEDTAVYYCAKDRRGSHADAFDVWGQGTLVTVSS | QSALTQPPSASGSPGQSVTISCTGTSSDVGGYNYVSW YQQYPGKAPKLLIYGNSNRPSGVPDRFSGSKSGTSAS LAITGLQAEDGADYYCQSYDSSLRVVFGGGTKVTVL G |
| QVQLQESGGGLVQPGRSLRLSCAASGFTFSTYAMNWVRQA PGKGLEWVSAISMSGESTYYADSVKGRFTISRDNSKNTLYL QMNSLRAEDTAVYYCAKDRRGSHADAFDVWGQGTLVTVS S | QSALTQPPSASGSPGQSVTISCTGTSSDVGGYNYVSW YQQYPGKAPKLLIYGNSNRPSGVPDRFSGSKSGTSAS LAITGLQAEDGADYYCQSYDSSLRVVFGGGTKVTVL G |
| QVQLQESGGGLVQPGRSLRLSCAASGFTFSSYAMHWVRQA PGKGLEWVSAISSSGGSTYYADSVKGRFTISRDNSKNTLYLQ MNSLRAEDTAVYYCAKDRRGSHADAFDVWGQGTLVTVSS | QSALTQPPSASGSPGQSVTISCTGTSSDVGHKFPVSWY QQYPGKAPKLLIYKNLLRPSGVPDRFSGSKSGTSASLA ITGLQAEDGADYYCQSYDSSLRVVFGGGTKVTVLG |
| QVQLQESGGGLVQPGRSLRLSCAASGFTFSSYAMHWVRQA PGKGLEWVSAISSSGGSTYYADSVKGRFTISRDNSKNTLYLQ MNSLRAEDTAVYYCAKDRRGSHADAFDVWGQGTLVTVSS | QSALTQPPSASGSPGQSVTISCTGTSSDVGLMHNVSW YQQYPGKAPKLLIYKSSSRPSGVPDRFSGSKSGTSASL AITGLQAEDGADYYCQSYDSSLRVVFGGGTKVTVLG |
| QVQLQESGGGLVQPGRSLRLSCAASGFTFSSYAMHWVRQA PGKGLEWVSAISSSGRSTYYADSVEGRFTISRDNSKNTLYLQ MNSLRAEDTAVYYCAKDRRGSHADALNVWGQGTLVTVSS | QSALTQPPSASGSPGQSVTISCTGTSSDVGGYNYVSW YQQYPGKAPKLLIYGNSNRPSGVPDRFSGSKSGTSAS LAITGLQAEDGADYYCQSYDSSLRVVFGGGTKVTVL G |
| EVQLVQSGAEVKKPGASVKVSCKASGYTFSDYEMHWVRQ APGQGLEWMGAIHPGSGDTAYNQRFKGRVTITADKSTSTAY MELSSLRSEDTAVYYCARFYSYAYWGQGTLVTVSA | DIVMTQTPLSLPVTPGEPASISCRSSQSLVHSNGNTYL QWYLQKPGQSPQLLIYKVSNRFSGVPDRFSGSGSGTD FTLKISRVEAEDVGVYYCSQSIYVPYTFGQGTKLEIKR |
| QVQLQQSGTELVRPGASVKLSCKALGYTFTDYEMHWVKQT PVHGLEWIGAIHPGSGDTAYNQRFKGKATLTADKSSSTAYM EYSSLTSEDSAVYYCTRFYSYAYWGQGTLVTVSA | DVVMTQTPLSLPVSLGDQASISCRSSQSLVHSNGNTY LQWYLQKPGQSPKLLIYKVSNRFSGVPDRFSGRGSGT DFTLKISRVEAEDLGVYFCSQSIYVPYTFGGGTKLEIK R |

or,
the light chain variable region of the amino acid sequence of the antigen recognition unit that recognizes claudin18.2 is independently selected from or has 70-100% sequence identity with the light chain variable regions shown in the following table, and/or the heavy chain variable region of the amino acid sequence of the antigen recognition unit that recognizes claudin18.2 is independently selected from or has 70-100% sequence identity with the heavy chain variable region shown in the following table;

| VH | VL |
|---|---|
| QVQLQQSGAELARPGASVKMSCKASGYTFTSYTMHWVKQ RPGQGLEWIGYINPSSGYTNYNQKFKDKATLTADKSSSTA YMQLSSLTSEDSAVYYCARIYYGNSFAYWGQGTTVTVSS | DIVMTQSPSSLTVTAGEKVTMSCKSSQSLLNSGNQKNY LTWYQQKPGQPPKLLIYWASTRESGVPDRFTGSGSGTD FTLTISSVQAEDLAVYYCQNDYSYPLTFGAGTKLELKR |
| QVQLQQSGPELVKPGALVKISCKASGYTFTSYDINWVKQR PGQGLEWIGWIYPGDGSTKYNEKFKGKATLTADKSSSTAY MQLSSLTSENSAVYFCARGGYRYDEAMDYWGQGTTVTVS S | QIVLTQSPAIMSASPGEKVTMTCSASSSISYMHWYQQK PGTSPKRWIYDTSKLASGVPARFSGSGSGTSYSLTISSM EAEDAATYYCHQRSSYPYTFGGGTKLEIKR |
| QIQLVQSGPELKKPGETVKISCKASGYTFTNYGMNWVKQA PGKGLKWMGWINTNTGEPTYAEEFKGRFAFSLETSASTAY LQINNLKNEDTATYFCARFSYGNSFAYWGQGTTVTVSS | DIVMTQSPSSLSVSAGEKVTMSCKSSQSLLNSGNQKNY LAWYQQKPGQPPKLLIYGASTRESGVPDRFTGSGSGTD FTLTISSVQAEDLAVYYCQNDHSYPLTFGAGTKLELKR |
| DVQLQESGPDLVKPSQSLSLTCTVTGYSITSGYNWHWIRQF PGNKMEWMGYIHYTGSTNYNPSLRSRISITRDTSKNQFFLQ LNSVTTDDTATYYCTRIYNGNSFPYWGQGTSVTVSS | DIVMTQSPSSLTVTPGEKVTMTCKSSQSLFNSGNQKNY LTWYQQRPGQPPKMLIYWASTRESGVPDRFTGSGSGT DFTLTISSVQAEDLAVFYCQNAYSFPYTFGGGTKLEIKR |
| QVQLQQSGAELARPGASVKMSCKASGYTFTSYTMHWVKQ RPGQGLEWIGYIDPSSGYTNYNQKFKDKATLTADKSSSTA YMQLSSLTSEDSAVYYCARIYYGNSFAYWGQGTTVTVSS | DIVMTQSPSSLTVTAGEKVTMSCKSSQSLLNSGNQKNY LTWYQQKPGQPPKLLIYWASTRESGVPDRFTGSGSGTD FTLTISSVQAEDLAVYYCQNDYSYPLTFGAGTKLELKR |
| QVQLQQSGAELARPGASVKMSCKASGYTFTSYTMHWVKQ RPGQGLEWIGYINPASGYTNYNQKFKDKATLTADKSSSTA YMQLSSLTSEDSAVYYCARIYYGNSFAYWGQGTTVTVSS | DIVMTQSPSSLTVTAGEKVTMSCKSSQSLLNSGNQKNY LTWYQQKPGQPPKLLIYWASTRESGVPDRFTGSGSGTD FTLTISSVQAEDLAVYYCQNDYSYPLTFGAGTKLELKR |
| QVQLQQSGAELARPGASVKMSCKASGYTFTSYTMHWVKQ RPGQGLEWIGYINPASGYTNYNQKFKDKATLTADKSSSTA YMQLSSLTSEDSAVYYCARIYYGNSFAYWGQGTTVTVSS | DIVMTQSPSSLTVTAGEKVTMSCKSSQSLLNSGNQKNY LTWYQQKPGQPPKLLIYWASTRESGVPDRFTGSGSGTD FTLTISSVQAEDLAVYYCQNDYSYPLTFGAGTKLELKR |
| QVQLVQSGAEVKKPGASVKVSCKASGYTFTSYTMHWVRQ APGQGLEWMGYINPASGYTNYNQKFKDRVTMTRDTSTST AYMELSSLRSEDTAVYYCARIYYGNSFAYWGQGTLVTVSS | DIVMTQSPDSLAVSLGERATINCKSSQSLLNSGNQKNY LTWYQQKPGQPPKLLIYWASTRESGVPDRFSGSGSGTD FTLTISSLQAEDVAVYYCQNDYSYPLTFGGGTKVEIKR |
| QVQLQESGPGLVKPSQTLSLTCTVSGGSISSGYNWHWIRQP PGKGLEWIGYIHYTGSTNYNPALRSRVTISVDTSKNQFSLK LSSVTAADTAVYYCARIYNGNSFPYWGQGTTVTVSS | DIVMTQSPDSLAVSLGERATINCKSSQSLFNSGNQKNY LTWYQQKPGQPPKLLIYWASTRESGVPDRFSGSGSGTD FTLTISSLQAEDVAVYYCQNAYSFPYTFGGGTKLEIKR |
| QVQLQESGPGLIKPSQTLSLTCTVSGGSISSGYNWHWIRQPP GKGLEWIGYIHYTGSTNYNPALRSRVTISVDTSKNQFSLKL SSVTAADTAIYYCARIYNGNSFPYWGQGTTVTVSS | DIVMTQSPDSLAVSLGERATINCKSSQSLFNSGNQKNY LTWYQQKPGQPPKLLIYWASTRESGVPDRFSGSGSGTD FTLTISSLQAEDVAVYYCQNAYSFPYTFGGGTKLEIKR. |

In a specific embodiment, the light chain LCDR1, LCDR2, and LCDR3 of the amino acid sequence of the antigen recognition unit that recognizes GPC3 are or have 70-100% sequence identity with a combination of the light chain LCDR1, LCDR2, and LCDR3 shown in any row of the following table, and/or the heavy chain HCDR1, HCDR2, and HCDR3 of the amino acid sequence of the antigen recognition unit that recognizes GPC3 are or have 70-100% sequence identity with a combination of the heavy chain HCDR1, HCDR2, and HCDR3 shown in any row of the following table;

| LCDR1 | LCDR2 | LCDR3 | HCDR1 | HCDR2 | HCDR3 |
|---|---|---|---|---|---|
| TGTSSDVGGYNYVS | GNSNRP S | QSYDSSLRV V | GFTFSSYAM H | AISGSGGSTYYADSVK G | DRRGSHADAFD V |
| TGTSSDVGGYNYVS | GNSNRP S | QSYDSSLRV V | GFTFSTYAMT | SISSSGESTYY ADSVKG | DRRGSHADAFD V |
| TGTSSDVGGYNYVS | GNSNRP S | QSYDSSLRV V | GFTFSTYAM A | EISSSGSRTYY ADSVKG | DRRGSHADAFD V |
| TGTSSDVGGYNYVS | GNSNRP S | QSYDSSLRV V | GFTFSTYAM A | AISMSGESTYYADSVK G | DRRGSHADAFD V |
| TGTSSDVGHKFPVS | KNLLRP S | QSYDSSLRV V | GFTFSSYAM H | AISSSGGSTYY ADSVKG | DRRGSHADAFD V |
| TGTSSDVGLMHNVS | KSSSRPS | QSYDSSLRV V | GFTFSSYAM H | AISSSGGSTYY ADSVKG | DRRGSHADAFD V |
| TGTSSDVGGYNYVS | KSSSRPS | QSYDSSLRV V | GFTFSSYAM H | AISSSGRSTYY ADSVEG | DRRGSHADALN V |
| RSSQSLVHSNGNTYL Q | KVSNRF S | SQSIYVPYT | DYEMH | AIHPGSGDTAYNQRFK G | FYSYAY |
| RSSQSLVHSNGNTYL Q | KVSNRF S | SQSIYVPYTF | DYEMH | AIHPGSGDTAYNQRFK G | FYSYAY; |

or
the light chain LCDR1, LCDR2, and LCDR3 of the amino acid sequence of the antigen recognition unit that recognizes claudin18.2 are or have 70-100% sequence identity with a combination of the light chain LCDR1, LCDR2, and LCDR3 shown in any row of the following table, and/or the heavy chain HCDR1, HCDR2, and HCDR3 of the amino acid sequence of the antigen recognition unit that recognizes claudin18.2 are or have 70-100% sequence identity with a combination of the heavy chain HCDR1, HCDR2, and HCDR3 shown in any row of the following table;

| LCDR1 | LCDR2 | LCDR3 | HCDR1 | HCDR2 | HCDR3 |
|---|---|---|---|---|---|
| KSSQSLLNSGNQKNYLT | WASTRES | QNDYSYPL T | SYTMH | YINPSSGYTNYNQKFKD | IYYGNSF AY |
| SASSSISYMH | DTSKLAS | HQRSSYPYT | SYDIN | WIYPGDGSTKYNEKFKG | GGYRYDEAMDY |
| KSSQSLLNSGNQKNYLA | GASTRES | QNDHSYPLT | NYGMN | WINTNTGEPTY AEEFKG | FSYGNSF AY |
| KSSQSLFNSGNQKNYLT | WASTRES | QNAYSFPYT | SGYNWH | YIHYTGSTNYNPSLRS | IYNGNSFPY |
| KSSQSLLNSGNQKNYLT | WASTRES | QNDYSYPL T | SYTMH | YIDPSSGYTNYNQKFKD | IYYGNSF AY |
| KSSQSLLNSGNQKNYLT | WASTRES | QNDYSYPL T | SYTMH | YINP AS GYTNYNQKFKD | IYYGNSF AY |
| KSSQSLLNSGNQKNYLT | WASTRES | QNDYSYPL T | SYTMH | YINP AS GYTNYNQKFKD | IYYGNSF AY |
| KSSQSLLNSGNQKNYLT | WASTRES | QNDYSYPL T | SYTMH | YINP AS GYTNYNQKFKD | IYYGNSF AY |
| KSSQSLFNSGNQKNYLT | WASTRES | QNAYSFPYT | SGYNWH | YIHYTGSTNYNP ALRS | IYNGNSFPY |
| KSSQSLFNSGNQKNYLT | WASTRES | QNAYSFPYT | SGYNWH | YIHYTGSTNYNP ALRS | IYNGNSFPY. |

In a specific embodiment, the light chain variable region and the heavy chain variable region of the amino acid sequence of the antigen recognition unit that recognizes GPC3 are or have 70-100% sequence identity with a combination of the light chain variable region and the heavy chain variable region shown in any row of the following table;

| VH | VL |
|---|---|
| QVQLQESGGGLVQPGRSLRLSCAASGFTFSSYAMHWVRQA PGKGLEWVSAISGSGGSTYYADSVKGRFTISRDNSKNTLYL QMNSLRAEDTAVYYCAKDRRGSHADAFDVWGQGTLVTVS S | QSALTQPPSASGSPGQSVTISCTGTSSDVGGYNYVSWY QQYPGKAPKLLIYGNSNRPSGVPDRFSGSKSGTSASLA ITGLQAEDGADYYCQSYDSSLRVVFGGGTKVTVLG |
| QVQLQESGGGLVQPGRSLRLSCAASGFTFSTYAMTWVRQA PGKGLEWVSSISSSGESTYYADSVKGRFTISRDNSKNTLYLQ MNSLRAEDTAVYYCAKDRRGSHADAFDVWGQGTLVTVSS | QSALTQPPSASGSPGQSVTISCTGTSSDVGGYNYVSWY QQYPGKAPKLLIYGNSNRPSGVPDRFSGSKSGTSASLA ITGLQAEDGADYYCQSYDSSLRVVFGGGTKVTVLG |
| QVQLQESGGGLVQPGRSLRLSCAASGFTFSTYAMAWVRQA PGKGLEWVSEISSSGSRTYYADSVKGRFTISRDNSKNTLYLQ MNSLRAEDTAVYYCAKDRRGSHADAFDVWGQGTLVTVSS | QSALTQPPSASGSPGQSVTISCTGTSSDVGGYNYVSWY QQYPGKAPKLLIYGNSNRPSGVPDRFSGSKSGTSASLA ITGLQAEDGADYYCQSYDSSLRVVFGGGTKVTVLG |
| QVQLQESGGGLVQPGRSLRLSCAASGFTFSTYAMNWVRQA PGKGLEWVSAISMSGESTYYADSVKGRFTISRDNSKNTLYL QMNSLRAEDTAVYYCAKDRRGSHADAFDVWGQGTLVTVS S | QSALTQPPSASGSPGQSVTISCTGTSSDVGGYNYVSWY QQYPGKAPKLLIYGNSNRPSGVPDRFSGSKSGTSASLA ITGLQAEDGADYYCQSYDSSLRVVFGGGTKVTVLG |
| QVQLQESGGGLVQPGRSLRLSCAASGFTFSSYAMHWVRQA PGKGLEWVSAISSSGGSTYYADSVKGRFTISRDNSKNTLYL QMNSLRAEDTAVYYCAKDRRGSHADAFDVWGQGTLVTVS S | QSALTQPPSASGSPGQSVTISCTGTSSDVGHKFPVSWY QQYPGKAPKLLIYKNLLRPSGVPDRFSGSKSGTSASLA ITGLQAEDGADYYCQSYDSSLRVVFGGGTKVTVLG |
| QVQLQESGGGLVQPGRSLRLSCAASGFTFSSYAMHWVRQA PGKGLEWVSAISSSGGSTYYADSVKGRFTISRDNSKNTLYL QMNSLRAEDTAVYYCAKDRRGSHADAFDVWGQGTLVTVS S | QSALTQPPSASGSPGQSVTISCTGTSSDVGLMHNVSW YQQYPGKAPKLLIYKSSSRPSGVPDRFSGSKSGTSASL AITGLQAEDGADYYCQSYDSSLRVVFGGGTKVTVLG |
| QVQLQESGGGLVQPGRSLRLSCAASGFTFSSYAMHWVRQA PGKGLEWVSAISSSGRSTYYADSVEGRFTISRDNSKNTLYLQ MNSLRAEDTAVYYCAKDRRGSHADALNVWGQGTLVTVSS | QSALTQPPSASGSPGQSVTISCTGTSSDVGGYNYVSWY QQYPGKAPKLLIYGNSNRPSGVPDRFSGSKSGTSASLA ITGLQAEDGADYYCQSYDSSLRVVFGGGTKVTVLG |
| EVQLVQSGAEVKKPGASVKVSCKASGYTFSDYEMHWVRQ APGQGLEWMGAIHPGSGDTAYNQRFKGRVTITADKSTSTA YMELSSLRSEDTAVYYCARFYSYAYWGQGTLVTVSA | DIVMTQTPLSLPVTPGEPASISCRSSQSLVHSNGNTYLQ WYLQKPGQSPQLLIYKVSNRFSGVPDRFSGSGSGTDFT LKISRVEAEDVGVYYCSQSIYVPYTFGQGTKLEIKR |
| QVQLQQSGTELVRPGASVKLSCKALGYTFTDYEMHWVKQ TPVHGLEWIGAIHPGSGDTAYNQRFKGKATLTADKSSSTAY MEYSSLTSEDSAVYYCTRFYSYAYWGQGTLVTVSA | DVVMTQTPLSLPVSLGDQASISCRSSQSLVHSNGNTYL QWYLQKPGQSPKLLIYKVSNRFSGVPDRFSGRGSGTD FTLKISRVEAEDLGVYFCSQSIYVPYTFGGGTKLEIKR; |

or,
the light chain variable region and the heavy chain variable region of the amino acid sequence of the antigen recognition unit that recognizes claudin 18.2 are or have 70-100% sequence identity with a combination of the light chain variable region and the heavy chain variable region shown in any row of the following table;

| VH | VL |
|---|---|
| QVQLQQSGAELARPGASVKMSCKASGYTFTSYTMHWVKQ RPGQGLEWIGYINPSSGYTNYNQKFKDKATLTADKSSSTA YMQLSSLTSEDSAVYYCARIYYGNSFAYWGQGTTVTVSS | DIVMTQSPSSLTVTAGEKVTMSCKSSQSLLNSGNQK NYLTWYQQKPGQPPKLLIYWASTRESGVPDRFTGSG SGTDFTLTISSVQAEDLAVYYCQNDYSYPLTFGAGT KLELKR |
| QVQLQQSGPELVKPGALVKISCKASGYTFTSYDINWVKQR PGQGLEWIGWIYPGDGSTKYNEKFKGKATLTADKSSSTAY MQLSSLTSENSAVYFCARGGYRYDEAMDYWGQGTTVTVS S | QIVLTQSPAIMSASPGEKVTMTCSASSSISYMHWYQQ KPGTSPKRWIYDTSKLASGVPARFSGSGSGTSYSLTIS SMEAEDAATYYCHQRSSYPYTFGGGTKLEIKR |
| QIQLVQSGPELKKPGETVKISCKASGYTFTNYGMNWVKQA PGKGLKWMGWINTNTGEPTYAEEFKGRFAFSLETSASTAY LQINNLKNEDTATYFCARFSYGNSFAYWGQGTTVTVSS | DIVMTQSPSSLSVSAGEKVTMSCKSSQSLLNSGNQK NYLAWYQQKPGQPPKLLIYGASTRESGVPDRFTGSG SGTDFTLTISSVQAEDLAVYYCQNDHSYPLTFGAGT KLELKR |
| DVQLQESGPDLVKPSQSLSLTCTVTGYSITSGYNWHWIRQF PGNKMEWMGYIHYTGSTNYNPSLRSRISITRDTSKNQFFLQ LNSVTTDDTATYYCTRIYNGNSFPYWGQGTSVTVSS | DIVMTQSPSSLTVTPGEKVTMTCKSSQSLFNSGNQK NYLTWYQQRPGQPPKMLIYWASTRESGVPDRFTGS GSGTDFTLTISSVQAEDLAVFYCQNAYSFPYTFGGGT KLEIKR |
| QVQLQQSGAELARPGASVKMSCKASGYTFTSYTMHWVKQ RPGQGLEWIGYIDPSSGYTNYNQKFKDKATLTADKSSSTA YMQLSSLTSEDSAVYYCARIYYGNSFAYWGQGTTVTVSS | DIVMTQSPSSLTVTAGEKVTMSCKSSQSLLNSGNQK NYLTWYQQKPGQPPKLLIYWASTRESGVPDRFTGSG SGTDFTLTISSVQAEDLAVYYCQNDYSYPLTFGAGT KLELKR |
| QVQLQQSGAELARPGASVKMSCKASGYTFTSYTMHWVKQ RPGQGLEWIGYINPASGYTNYNQKFKDKATLTADKSSSTA YMQLSSLTSEDSAVYYCARIYYGNSFAYWGQGTTVTVSS | DIVMTQSPSSLTVTAGEKVTMSCKSSQSLLNSGNQK NYLTWYQQKPGQPPKLLIYWASTRESGVPDRFTGSG SGTDFTLTISSVQAEDLAVYYCQNDYSYPLTFGAGT KLELKR |
| QVQLQQSGAELARPGASVKMSCKASGYTFTSYTMHWVKQ RPGQGLEWIGYINPASGYTNYNQKFKDKATLTADKSSSTA YMQLSSLTSEDSAVYYCARIYYGNSFAYWGQGTTVTVSS | DIVMTQSPSSLTVTAGEKVTMSCKSSQSLLNSGNQK NYLTWYQQKPGQPPKLLIYWASTRESGVPDRFTGSG SGTDFTLTISSVQAEDLAVYYCQNDYSYPLTFGAGT KLELKR |
| QVQLVQSGAEVKKPGASVKVSCKASGYTFTSYTMHWVRQ APGQGLEWMGYINPASGYTNYNQKFKDRVTMTRDTSTST AYMELSSLRSEDTAVYYCARIYYGNSFAYWGQGTLVTVSS | DIVMTQSPDSLAVSLGERATINCKSSQSLLNSGNQKN YLTWYQQKPGQPPKLLIYWASTRESGVPDRFSGSGS GTDFTLTISSLQAEDVAVYYCQNDYSYPLTFGGGTK VEIKR |
| QVQLQESGPGLVKPSQTLSLTCTVSGGSISSGYNWHWIRQP PGKGLEWIGYIHYTGSTNYNPALRSRVTISVDTSKNQFSLK LSSVTAADTAVYYCARIYNGNSFPYWGQGTTVTVSS | DIVMTQSPDSLAVSLGERATINCKSSQSLFNSGNQKN YLTWYQQKPGQPPKLLIYWASTRESGVPDRFSGSGS GTDFTLTISSLQAEDVAVYYCQNAYSFPYTFGGGTK LEIKR |
| QVQLQESGPGLIKPSQTLSLTCTVSGGSISSGYNWHWIRQPP GKGLEWIGYIHYTGSTNYNPALRSRVTISVDTSKNQFSLKL SSVTAADTAIYYCARIYNGNSFPYWGQGTTVTVSS | DIVMTQSPDSLAVSLGERATINCKSSQSLFNSGNQKN YLTWYQQKPGQPPKLLIYWASTRESGVPDRFSGSGS GTDFTLTISSLQAEDVAVYYCQNAYSFPYTFGGGTK LEIKR. |

In a specific embodiment, the amino acid sequence of the antigen recognition unit that recognizes GPC3 is selected from or has 70-100% sequence identity with the sequence shown in the following table;

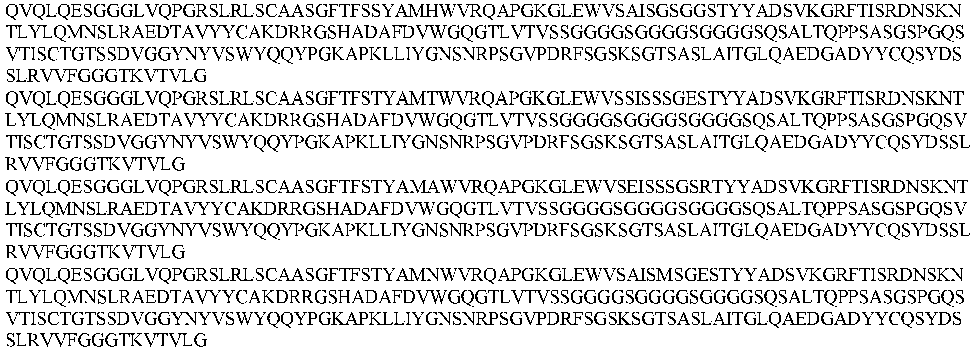

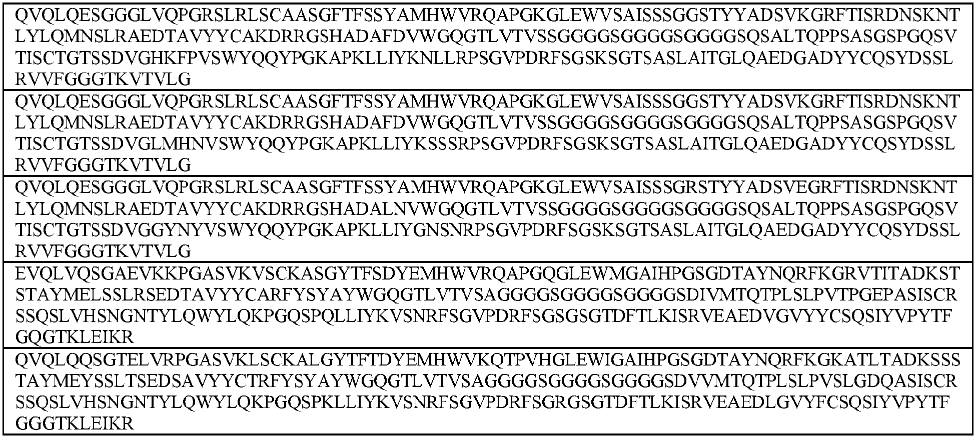

or,
the amino acid sequence of the antigen recognition unit that recognizes claudin18.2 is selected from or has 70-100% sequence identity with the sequence shown in the following table;

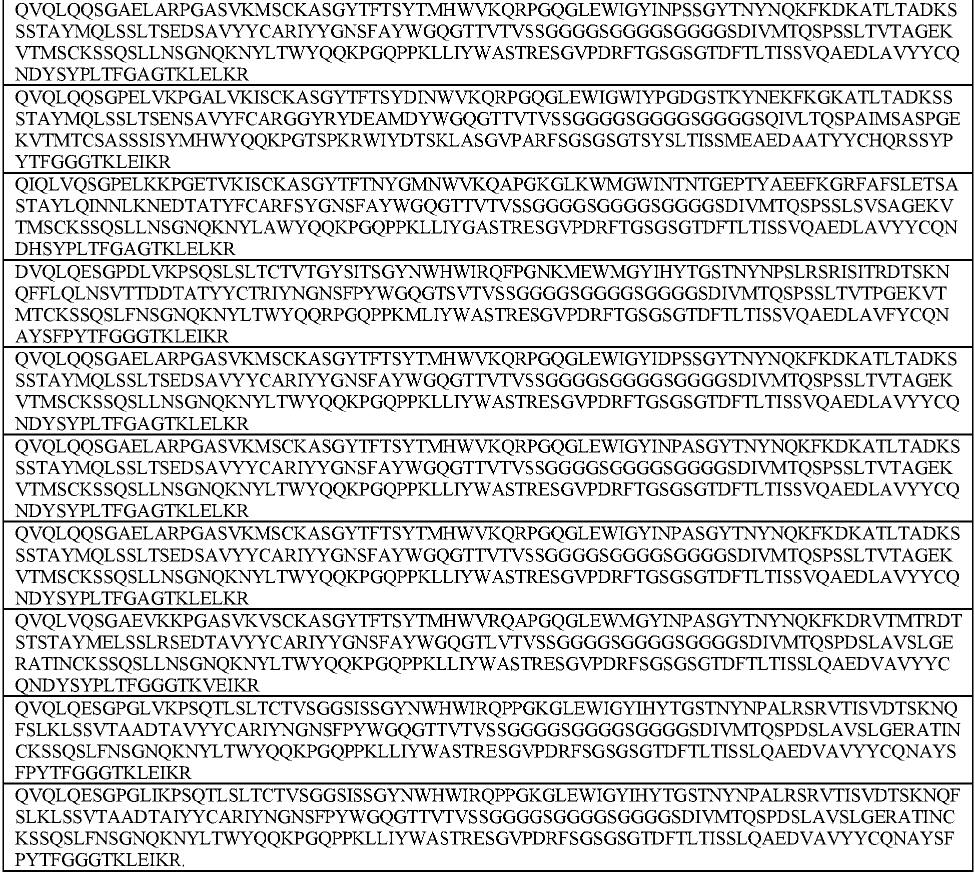

In a preferred embodiment, the antigen recognition unit is connected to the extracellular domain of the TCR via a linker sequence.

In a preferred embodiment, the linker sequence includes (G₄S)ₙ, where n = 1 to 4; or the encoding sequence of the linker has the nucleic acid sequence shown in SEQ ID NO: 5, 11 or 12.

In a preferred embodiment, the TCR subunit comprises an extracellular domain, and/or transmembrane domain, and/or intracellular domain; preferably, at least two of the extracellular domain, transmembrane domain and intracellular domain are from the same TCR subunit.

In a preferred embodiment, the TCR subunit comprises a TCR intracellular domain containing a stimulatory domain, and the stimulatory domain is selected from the intracellular signaling domain of CD3ε, CD3γ, or CD3δ, or an amino acid sequence thereof having at least one modification.

In a preferred embodiment,
(i) a light chain variable region of the amino acid sequence of the antigen recognition unit that binds to GPC3, wherein the light chain variable region comprises an amino acid sequence having at least one but not more than 30 mofifications as compared with amino acid sequence of the light chain variable region of the anti-GPC3 antibody provided herein, or a sequence having 90-99% identity with the amino acid sequence of the light chain variable region of the anti-GPC3 antibody provided herein; or
(ii) a light chain variable region of the amino acid sequence of the antigen recognition unit that binds to claudin18.2, wherein the light chain variable region comprises an amino acid sequence having at least one but not more than 30 mofifications as compared with amino acid sequence of the light chain variable region of the anti-claudin18.2 antibody provided herein, or a sequence having 90-99% identity with the amino acid sequence of the light chain variable region of the anti-claudin18.2 antibody provided herein.

In a preferred example,
(i) a heavy chain variable region of the amino acid sequence of the antigen recognition unit that binds to GPC3, wherein the heavy chain variable region comprises an amino acid sequence having at least one but not more than 30 mofifications as compared with amino acid sequence of the heavy chain variable region of the anti-GPC3 antibody provided herein, or a sequence having 90-99% identity with the amino acid sequence of the heavy chain variable region of the anti-GPC3 antibody provided herein; or
(ii) a heavy chain variable region of the amino acid sequence of the antigen recognition unit that binds to claudin18.2, wherein the heavy chain variable region comprises an amino acid sequence having at least one but not more than 30 mofifications as compared with amino acid sequence of the heavy chain variable region of the anti-claudin18.2 antibody provided herein, or a sequence having 90-99% identity with the amino acid sequence of the heavy chain variable region of the anti-claudin18.2 antibody provided herein.

In a preferred example, the TFP comprises the extracellular domain of the TCR subunit, and the extracellular domain includes the extracellular domain of a protein or a part thereof selected from the group consisting of: TCRα chain, TCRβ chain, CD3εTCR subunit, CD3γTCR subunit, CD3δTCR subunit, functional fragments thereof, and an amino acid sequence thereof with at least one but no more than 20 modifications.

In a preferred example, the TFP includes a transmembrane domain, and the transmembrane domain includes a transmembrane domain of a protein selected from the group consisting of: TCRα chain, TCRβ chain, CD3εTCR subunit, CD3γTCR subunit, The CD3δTCR subunit, functional fragments thereof, and an amino acid sequence thereof with at least one but no more than 20 modifications.

In a preferred example, the TFP includes a transmembrane domain, and the transmembrane domain includes a transmembrane domain of a protein selected from the group consisting of: TCRα chain, TCRβ chain, TCRζ chain, CD3εTCR subunit, CD3γTCR subunits, CD3δTCR subunits, CD45, CD4, CD5, CD8, CD9, CD16, CD22, CD33, CD28, CD37, CD64, CD80, CD86, CD134, CD137, CD154, functional fragments thereof, and an amino acid sequence thereof with at least one but not more than 20 modifications.

In a preferred example, the antigen recognition unit includes an antibody or a fragment thereof. Preferably, the antibody fragment is Fab, Fab', F(ab')2, Fv fragment, scFv, sdFv, Fd fragment composed of VH and CH1 domains, linear antibody, single domain antibody, or camelid VHH domain, and more preferably, the antibody is a scFv.

In a preferred example, the TFP further includes a costimulatory domain.

In a preferred example, the costimulatory domain is a functional signaling domain obtained from a protein selected from the group consisting of: OX40, CD2, CD27, CD28, CDS, ICAM-1, LFA-1 (CD11a/CD18), ICOS (CD278) and 4-1BB (CD137), and an amino acid sequence thereof with at least one but no more than 20 modifications.

In a second aspect, the present invention provides a combination of the fusion protein and other factors, the other factors including cytokines, transcription factors, chemokines, and/or combinations thereof, and the expression cassette is constitutively or inducibly expressed; preferably, the promoter of the expression cassette is an immune cell inducible promoter; preferably, the immune cell inducible promoter is NFAT6 promoter; and preferably, the NFAT6 promoter is reversely regulated.

In a preferred example, the fusion protein and other factors are expressed by the same nucleic acid molecule, or by different nucleic acid molecules.

In a preferred example, the fusion protein and other factors are expressed by the same nucleic acid molecule, and the expression cassettes of the other factors and the expression cassettes of TFP, and the expression cassettes of the other factors are directly connected or connected by tandem fragments, and the tandem fragments are selected from F2A, PA2, T2A, and/or E2A.

In a preferred example, the cytokine is selected from IL-7 or IL-12, the chemokine is CCL19 or CCL21; the transcription factor is RUNX3; preferably, the other factors are a combination of cytokines and chemokines; and preferably, the other factors are a combination of IL7 and CCL21, or a combination of IL7 and CCL19.

In a preferred example, the at least one but no more than 20 modifications include amino acid modifications that mediate cell signal transduction, or amino acid modifications that are phosphorylated in response to the binding of a ligand to TFP.

In a preferred example, the TFP includes the immunoreceptor tyrosine activation motif (ITAM) of the TCR subunit, and the ITAM includes the ITAM or a part thereof of a protein selected from the group consisting of: CD3ζTCR subunit, CD3εTCR subunit, CD3γTCR subunit, CD3δTCR subunit, TCRζ chain, Fcε receptor 1 chain, Fcε receptor 2 chain, Fcγ receptor 1 chain, Fcγ receptor 2a chain, Fcγ receptor 2b1 chain, Fcγ receptor 2b2 chain, Fcγ receptor 3a chain, Fcγ receptor 3b chain, Fcβ receptor 1 chain, TYROBP (DAP12), CD5, CD16a, CD16b, CD22, CD23, CD32, CD64, CD79a, CD79b, CD89, CD278, CD66d, and functional fragments thereof, and an amino acid sequence thereof with at least one but no more than 20 modifications.

In a preferred example, the ITAM replaces the ITAM of CD3γ, CD3δ or CD3ε.

In a preferred example, the ITAM is selected from CD3ζTCR subunit, CD3εTCR subunit, CD3γTCR subunit, or CD3δTCR subunit, and replaces different ITAMs selected from CD3ζTCR subunit, CD3εTCR subunit, CD3γTCR subunit or CD3δTCR subunit.

In a preferred example, the TFP molecule further includes a leader sequence.

In a third aspect, the present invention provides a nucleic acid molecule expressing the fusion protein described in the first aspect or the combination described in the second aspect.

In a preferred example, the nucleic acid is composed of DNA and/or RNA.

In a preferred example, the nucleic acid is mRNA.

In a preferred example, the nucleic acid comprises nucleotide analogs.

In the fourth aspect, the present invention provides a vector comprising the nucleic acid molecule described in the third aspect.

In a preferred example, the vector is selected from the group consisting of DNA, RNA, plasmid, lentiviral vector, adenoviral vector, Rous sarcoma virus (RSV) vector and retroviral vector.

In the fifth aspect, the present invention provides a cell comprising the vector of the fourth aspect or having the nucleic acid molecule of the third aspect integrated into its genome.

In a preferred example, the cells are human T cells, preferably allogeneic T cells.

In a sixth aspect, the present invention provides a protein complex comprising:
i) the fusion protein TFP molecule of the first aspect; and
ii) at least one endogenous TCR subunit or endogenous TCR complex.

In a seventh aspect, the present invention provides a method for preparing cells, comprising transducing T cells with the vector of the fourth aspect or the nucleic acid molecule of the third aspect.

In an eighth aspect, the present invention provides a method for producing an RNA-engineered cell population, comprising introducing *in vitro* transcribed RNA or synthetic RNA into the cell,
(i) wherein the RNA includes the nucleic acid of the third aspect.

In a ninth aspect, the present invention provides a method for providing anti-tumor immunities in a mammal, comprising administering to the mammal an effective amount of the fusion protein of the first aspect and the combination of the second aspect, the nucleic acid molecule of the third aspect, the vector of the fourth aspect, or the cell of the fifth aspect.

In a preferred example, the mammal is a human.

In a tenth aspect, the present invention provides a method for treating a mammal suffering from a disease related to the expression of GPC3 or claudin 18.2, comprising administering to the mammal an effective amount of the fusion protein of the first aspect, the combination of the second aspect, the nucleic acid molecule of the third aspect, the vector of the fourth aspect, or the cell of the fifth aspect.

In a preferred example, the disease related to the expression of GPC3 or claudin 18.2 is selected from colon cancer, rectal cancer, renal cell carcinoma, liver cancer, lung cancer, small intestine cancer, esophageal cancer, melanoma, bone cancer, pancreatic cancer, skin cancer, head and neck cancer, skin or intraocular malignant melanoma, uterine cancer, ovarian cancer, rectal cancer, anal cancer, stomach cancer, testicular cancer, uterine cancer, fallopian tube cancer, endometrial cancer, cervical cancer, vaginal cancer, endocrine system cancer, thyroid cancer, parathyroid cancer, adrenal cancer, soft tissue sarcoma, urethral cancer, penile cancer, bladder cancer, kidney or ureter cancer, renal pelvis cancer, central nervous system (CNS) tumors, tumor angiogenesis, spinal tumors, brainstem glioma, pituitary adenoma, Kaposi's sarcoma, epidermoid carcinoma, squamous cell carcinoma, non-cancer related indications related to the expression of GPC3 or claudin18.2; and preferably, selected from liver cancer, lung cancer, breast cancer, ovarian cancer, kidney cancer, thyroid cancer, stomach cancer, colorectal cancer, pancreatic cancer, esophageal cancer.

In a preferred example, the cells expressing TFP molecules are administered in combination with an agent that increases the efficacy of the cells expressing TFP molecules.

In a preferred example, compared with a mammal that is administered with an effective amount of T cells expressing GPC3 chimeric antigen receptor (CAR) or claudin18.2 chimeric antigen receptor (CAR), there are fewer cytokines released in the mammal.

In a preferred example, the cells expressing TFP molecules are administered in combination with an agent that improves one or more side effects associated with the administration of cells expressing TFP molecules.

In a preferred example, the cell expressing the TFP molecule is administered in combination with an agent for treating the disease related to GPC3 or claudin 18.2.

In the eleventh aspect, the present invention provides the fusion protein of the first aspect, the combination of the second aspect, the nucleic acid molecule of the third aspect, the vector of the fourth aspect, or the cell of the fifth aspect as a medicament.

In a preferred example, the medicament is a medicament for preventing or treating a disease related to the expression of GPC3 or claudin 18.2.

In a preferred example, the disease related to the expression of GPC3 or claudin 18.2 is selected from colon cancer, rectal cancer, renal cell carcinoma, liver cancer, lung cancer, small intestine cancer, esophageal cancer, melanoma, bone cancer, pancreatic cancer, skin cancer, head and neck cancer, skin or intraocular malignant melanoma, uterine cancer, ovarian cancer, rectal cancer, anal cancer, stomach cancer, testicular cancer, uterine cancer, fallopian tube cancer, endometrial cancer, cervical cancer, vaginal cancer, endocrine system cancer, thyroid cancer, parathyroid cancer, adrenal cancer, soft tissue sarcoma, urethral cancer, penile cancer, bladder cancer, kidney or ureter cancer, renal pelvis cancer, central nervous system (CNS) tumors, tumor angiogenesis, spinal tumors, Brainstem glioma, pituitary adenoma, Kaposi's sarcoma, epidermoid carcinoma, squamous cell carcinoma, non-cancer related indications related to GPC3 or claudin18.2 expression; and preferably, selected from liver cancer, lung cancer, breast cancer, ovarian cancer, kidney cancer, thyroid cancer, stomach cancer, colorectal cancer, pancreatic cancer, esophageal cancer.

In one aspect of the present invention, a TCR fusion protein is provided. The TCR fusion protein comprises a tumor antigen recognition unit and a TCR unit, the tumor antigen recognition unit is an antibody that recognizes a solid tumor antigen, and the TCR unit contains at least a portion of CD3 extracellular domain, CD3 transmembrane domain and CD3 intracellular signal domain.

In a preferred embodiment, the solid tumor antigen is selected from GPC3, claudin 6, EGFR, EGFRvIII, claudin 18.2. More preferably, the solid tumor antigen is GPC3 or claudin 18.2.

In a preferred embodiment, the solid tumor is selected from the group consisting of colon cancer, rectal cancer, renal cell carcinoma, liver cancer, lung cancer, small bowel cancer, esophageal cancer, melanoma, bone cancer, pancreatic cancer, skin cancer, head and neck cancer, skin or intraocular melanoma, uterine cancer, ovarian cancer, rectal cancer, anal cancer, stomach cancer, testicular cancer, uterine cancer, fallopian tube cancer, endometrial cancer, cervical cancer, vagina cancer, vaginal cancer, endocrine system cancer, thyroid cancer, parathyroid cancer, adrenal cancer, soft tissue sarcoma, urethral cancer, penile cancer, bladder cancer, kidney or ureter cancer, renal pelvis cancer, central nervous system (CNS) tumor, tumor angiogenesis, spine tumor, brainstem glioma, pituitary adenoma, Kaposi's sarcoma, epidermoid carcinoma, squamous cell carcinoma; and preferably, selected from liver cancer, lung cancer, breast cancer, ovarian cancer, kidney cancer, thyroid cancer, stomach cancer, colorectal cancer, stomach cancer, pancreatic cancer, esophageal cancer.

In a preferred embodiment, the antibody is a complete immunoglobulin or antibody fragment; preferably, the antibody fragment is Fab, Fab', F(ab')2, Fv fragment, scFv, sdFv, Fd fragments composed of VH and CH1 domains, linear antibodies, single domain antibodies, or camelid VHH domains; and more preferably, the antibody is a scFv.

In a preferred embodiment, the antibody has HCDR1 shown in SEQ ID NO: 15, HCDR2 shown in SEQ ID NO: 16, HCDR3 shown in SEQ ID NO: 17, and LCDR1 SEQ ID NO: 18 , LCDR2 shown in SEQ ID NO: 19, and LCDR3 shown in SEQ ID NO: 20;

In a preferred embodiment, the antibody has the VH shown in SEQ ID NO: 21 and the VL shown in SEQ ID NO: 22.

In a preferred embodiment, the CD3 intracellular signal domain is the intracellular signal domain of CD3ε, CD3γ, CD3ζ, or CD3δ, preferably, the intracellular signal domain of CD3ε and CD3γ, and more preferably, the intracellular signal domain of CD3ε.

In a preferred embodiment, the tumor antigen recognition unit is operably connected to the TCR unit.

In a preferred embodiment, the tumor antigen recognition unit is connected to the extracellular domain of the TCR via a linker.

Preferably, the linker sequence is (G₄S)ₙ, n = any natural number between 1 and 4, or the linker has the nucleic acid sequence shown in SEQ ID NO: 5, 11 or 12.

In a preferred embodiment, the TCR unit contains at least a part of the TCR extracellular domain, TCR transmembrane domain and CD3 intracellular signal domain from the same TCR subunit.

In a preferred embodiment, the TCR unit has the amino acid sequence shown in SEQ ID NO: 3 or 8.

In a preferred embodiment, the nucleic acid encoding the TCR fusion protein has the sequence shown in SEQ ID NO: 7, 8, 13, 14.

In one aspect of the present invention, a nucleic acid encoding the TCR fusion protein of the present invention is provided.

In one aspect of the present invention, an expression vector is provided, which contains the nucleic acid encoding the TCR fusion protein of the present invention.

In one aspect of the present invention, a virus is provided, which comprises the expression vector of the present invention.

In one aspect of the present invention, T cells expressing the TCR fusion protein of the present invention are provided.

In a preferred embodiment, the T cell also expresses an cytokine, transcription factor, another chimeric receptor, chemokine, chemokine receptor, siRNA reducing PD-1 expression, or a protein blocking PD-L1, TCR, or safety switch.

In another preferred embodiment, the factor is a fusion protein expressing a cytokine.

In another preferred embodiment, the cytokine is selected from IL-12, IL-18, IL-21, or type I interferon.

In another preferred embodiment, the fusion protein contains cytokines and chemokines.

In another preferred embodiment, the chemokine is CCL19 or CCL21; preferably, the fusion protein contains IL7 and CCL21, or IL7 and CCL19.

In another preferred embodiment, the chemokine receptor includes CCR2, CCR5, CXCR2, or CXCR4;

In another preferred embodiment, the safety switch includes iCaspase-9, Truncated EGFR or RQR8.

In another preferred embodiment, the other chimeric receptor is selected from the group consisting of chimeric antigen receptor (CAR), modified T cell (antigen) receptor (TCR), T cell fusion protein (TFP), T Cell Antigen Coupler (TAC).

In another preferred embodiment, the transcription factor is RUNX3.

In another preferred embodiment, the T cells also express a fusion protein inhibiting the tumor microenvironment or a fusion protein stimulating T cells.

In another preferred embodiment, the chemokine is a lymphocyte chemokine, and preferably, the chemokine is CCL21.

In one aspect of the present invention, there is provided the use of the expression vector of the present invention, or the virus of the present invention, or the cell of the present invention for the preparation of a medicament for inhibiting tumors.

It should be understood that within the scope of the present invention, the above-mentioned technical features of the present invention and the technical features specifically described in the following (such as the embodiments) can be combined with each other to form a new or preferred technical solution, which will not be repeated herein one by one.

### Description of drawings

Figure 1A shows the constructed TCR fusion protein vector targeting Claudin 18.2 and GPC3; and Figure 1B shows the result of the positive rate of T cells expressing the TCR fusion protein;
Figure 2 shows the *in vitro* killing results of T cells expressing TCR fusion protein on pancreatic cancer cells and gastric cancer cells;
Figure 3 shows the detection results of cytokine secretion after co-incubation of T cells expressing TCR fusion protein with pancreatic cancer cells and gastric cancer cells;
Figure 4 shows the detection results of the positive rate of different TCR fusion proteins;
Figure 5 shows the cell killing effects of T cells expressing TCR fusion protein targeting GPC3;
Figure 6 shows the secretion of different cytokines in T cells expressing TCR fusion protein detected by ELISA;
Figure 7 shows the *in vivo* anti-tumor results of T cells expressing TCR fusion protein targeting GPC3;
Figure 8 shows the body weight of mice in each group after treated with T cells expressing TCR fusion protein targeting GPC3.

### Modes for carrying out the invention

The present application provides a technical solution for treating a solid tumor, especially for treating gastric cancer and pancreatic cancer by using cells expressing TCR fusion protein.

Unless specifically defined, all technical and scientific terms used herein have the same meanings commonly understood by those skilled in the fields of gene therapy, biochemistry, genetics, and molecular biology. All methods and materials similar or equivalent to those described herein can be used in the practice or testing of the present invention, among which suitable methods and materials are described herein. All publications, patent applications, patents and other references mentioned in this article are incorporated herein by reference in their entirety. In case of conflict, the specification, including definitions, will control. In addition, unless otherwise specified, the materials, methods, and examples are illustrative only and not intended to be limiting.

Unless otherwise specified, the practice of the present invention will use traditional techniques of cell biology, cell culture, molecular biology, transgenic biology, microbiology, recombinant DNA and immunology, all of which fall within the technical scope of the art. These techniques are fully explained in the literature. See, for example, Current Protocols in Molecular Biology (FrederickM.AUSUBEL, 2000, Wileyand sonInc, Library of Congress, USA); Molecular Cloning: A Laboratory Manual, Third Edition, (Sambrooketal, 2001, Cold Spring Harbor, NewYork: Cold Spring Harbor Laboratory Press); Oligonucleotide Synthesis (M.J.Gaited., 1984); Mullis et al. U.S. Pat. No. 4,683,195; Nucleic Acid Hybridization (B. D. Harries & S. J. Higginseds. 1984); Transcription And Translation (B. D. Hames & S. J. Higginseds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson 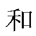 M. Simon, eds.-in-chief, Academic Press, Inc., New York), especially Vols. 154 and 155 (Wuetal. eds.) and Vol.185, "Gene Expression Technology" (D. Goeddel, ed.); Gene Transfer Vectors For Mammalian Cells (J. H. Miller 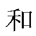 M. P. Caloseds., 1987, Cold Spring Harbor Laboratory); Immunochemical Methods In Cell And Molecular Biology (Mayer and Walker, eds., Academic Press, London, 1987); Hand book Of Experimental Immunology, Vol I-IV (D. M. Weir and C. C. Blackwell, eds., 1986); and Manipulating the Mouse Embryo (Cold Spring Harbor Laboratory Press, Cold Spring Harbor, N.Y., 1986).

To facilitate a better understanding of the present invention, relevant terms are defined as follows:
As used herein, "about" may mean, depending on the specific circumstances and known by a skilled person in the art, plus or minus less than 1% or 1%, 2%, 3%, 4%, 5%, 6%, 7%, 8%, 9%, 10%, 11%, 12%, 13%, 14%, 15%, 16%, 17%, 18%, 19%, 20%, 25%, 30% or more than 30 %.

The term "T cell (antigen) receptor (TCR), also known as TCR subunit, or TCR unit" is a characteristic mark on the surface of all T cells, which binds to CD3 by a non-covalent bond to form TCR- CD3 complex. TCR is responsible for recognizing antigens bound to major histocompatibility complex molecules. TCR is a heterodimer composed of two different peptide chains, composed of two peptide chains, α and β. Each peptide chain can be divided into several parts, such as variable region (V region), constant region (C region), transmembrane region and cytoplasmic region, characterized in that the cytoplasmic region is very short. TCR molecules belong to the immunoglobulin superfamily, and their antigen specificity exists in the V region; each of V regions (Vα, Vβ) has three hypervariable regions CDR1, CDR2, and CDR3, in which CDR3 has the largest variation and directly determines the binding specificity of TCR to an antigen. When TCR recognizes the MHC-antigen peptide complex, CDR1 and CDR2 recognize and bind to the side wall of the antigen binding groove of the MHC molecule, and CDR3 directly binds to the antigen peptide. TCR is divided into two categories: TCR1 and TCR2; TCR1 is composed of two chains, γ and δ, and TCR2 is composed of two chains, α and β. The recognition ability of these natural (or manufactured by other means) "anti-cancer" T cells is generally weak, therefore they cannot favorably attack cancer cells. In this case, a partial genetic modification method can be used to improve the "affinity" and effectiveness of these TCRs to the corresponding TAA, that is, high-affinity TCR. "Genetically modified TCR" technology is therefore called "affinity-enhanced TCR" technology. The gene modified T cell receptor uses the constant region domains of the heavy and light chains of antibodies that belong to the same immunoglobulin superfamily with the TCR molecule to replace the constant region domains of the B chain and a chain, respectively, to form a chimeric TCR molecule (chim-TCR).

The term "TCR fusion protein" or "TFP" includes recombinant proteins derived from various TCR proteins, which are generally capable of: i) binding to the surface antigen on target cells; ii) interacting with other polypeptide components of an intact TCR complex when localized to T cells. A "TFP T cell" is a T cell that has been transduced with (for example, according to the methods disclosed herein) and expresses TFP, for example, a T cell introduced with a natural TCR. In some embodiments, the T cell is a CD4+ T cell, CD8+ T cell, or CD4+/CD8+ T cell. In some embodiments, the TFP T cell is a NK cell. In some embodiments, the TFP T cell is a γδ T cell.

The "TCR fusion protein" of the present invention includes an extracellular antigen-binding domain (also called an antigen recognition unit), TCR transmembrane domain, and intracellular domain. The antigen-binding domain is a continuous polypeptide chain, including, for example, a single domain antibody fragment (sdAb), a part of single-chain antibody (scFv) derived from murine, humanized or human antibodies (Harlow et al., 1999, Using Antibodies: A Laboratory Manual, Cold Spring Harbor Laboratory Press, NY; Harlow et al. Human, 1989, Antibodies: A Laboratory Manual, Cold Spring Harbor, NY; Houston et al., 1988, Proc. Natl. Acad. Sci. USA 85: 5879-5883; Bird et al., 1988, Science 242: 423-426) . In one aspect, the antigen binding domain of the TFP composition of the invention includes an antibody fragment. In another aspect, TFP includes an antibody fragment containing scFv or sdAb.

The present invention includes a recombinant DNA construct encoding TFP, wherein the TFP comprises an antibody fragment specifically binding to GPC3 or Claudin 18.2, and the sequence of the antibody fragment is adjacent to the nucleic acid sequence encoding the TCR unit or a part thereof and in the same reading frame. The TFP provided herein can associate with one or more endogenous (alternatively, one or more exogenous, or endogenous and exogenous combinations) TCR units to form a functional TCR complex.

In one aspect, the TFP of the present invention comprises a target-specific binding element, which is also referred to as an antigen recognition unit. The choice of a part depends on the type and number of target antigens that define the surface of the target cell. For example, the antigen recognition unit can be selected to recognize the target antigen as a cell surface marker associated with a specific disease state on the target cell. Therefore, examples of cell surface markers that can be used as target antigens of the antigen recognition unit in the TFP of the present invention include markers related to viral, bacterial and parasitic infections, autoimmune diseases, and cancerous diseases (e.g., malignant diseases).

The extracellular domain of TFP of the present invention can be derived from natural sources or from recombinant sources. In the case of natural sources, the domain can be derived from any protein, especially a membrane-bound or transmembrane protein. In one aspect, the extracellular domain can associate with the transmembrane domain. Extracellular domains particularly useful in the present invention may include at least the following extracellular regions: for example, the α, β, or ζ chains of the T cell receptor, or CD3ε, CD3γ, or CD3δ, or in alternative embodiments, include CD28, CD45 , CD4, CD5, CD8, CD9, CD16, CD22, CD33, CD37, CD64, CD80, CD86, CD134, CD137, CD154.

The transmembrane domain of the TFP of the present invention can be derived from natural sources or recombinant sources. In the case of natural sources, the domain can be derived from any membrane-bound or transmembrane protein. In one aspect, the transmembrane domain can signal to the intracellular domain when TFP binds to a target. Transmembrane domains particularly useful in the present invention may include at least the following transmembrane regions: for example, the α, β, or ζ chains of T cell receptors, or CD28, CD3ε, CD45, CD4, CD5, CD8, CD9, CD16, CD22 , CD33, CD37, CD64, CD80, CD86, CD134, CD137, CD154. In some cases, the transmembrane domain may be connected to the extracellular region of TFP (such as the antigen binding domain of TFP) via a hinge (such as a hinge from a human protein). For example, in one embodiment, the hinge may be a human immunoglobulin (Ig) hinge, such as an IgG4 hinge or CD8a hinge.

The linker of the present invention is optionally a short oligopeptide of 2 to 10 amino acids in length, or a polypeptide linker can form a connection between the transmembrane domain and the cytoplasmic region of TFP. The glycine-serine doublet provides a particularly suitable linker.

If TFP contains CD3γ, δ, or ε polypeptides, the cytoplasmic domain of TFP may include intracellular signaling domains; and TCRα and TCRβ subunits usually lack signaling domains. The intracellular signaling domain is generally responsible for activating at least one normal effector function of immune cells into which TFP has been introduced. The term "effector function" refers to the specialized function of a cell. For example, the effector function of T cells can be cytolytic activity or auxiliary activity, including the secretion of cytokines. In one example, after co-incubated with tumor cells overexpressing TFP targeting antigens, the TFP-T cells can secrete large amounts of IFN-γ, Granzyme-B, IL2, TNF-α and GM-CSF; and compared with CAR T cells constructed with the same antigen recognition unit, TCR-T maintains the same significant cytotoxicity, while the cytokine secretion is significantly reduced, thereby effectively reducing the possibility of cytokine storm.

Therefore, the term "intracellular signaling domain" refers to a part of a protein that transduces effector function signals and guides cells to perform specialized functions. Although the entire intracellular signaling domain can usually be used, in many cases it is not necessary to use the entire chain. If the truncated part of the intracellular signaling domain is used, such a truncated part can be used instead of the complete chain as long as it transduces the effector function signal. Therefore, the term "intracellular signaling domain" is intended to include any truncated portion of the intracellular signaling domain sufficient to transduce effector function signals.

Examples of intracellular signaling domains used in TFPs of the present invention include cytoplasmic sequences of T cell receptors (TCRs) and co-receptors that act synergistically to initiate signal transduction after antigen receptor engagement, and derivatives or variants of any of these sequences and any recombinant sequence with the same functional capabilities. T cell activation can be mediated by two different types of cytoplasmic signaling sequences: the signaling sequence (primary intracellular signal transduction domain) that initiates antigen-dependent primary activation through TCR and the signaling sequence acts in an antigen-independent manner to provide secondary or costimulatory signals (secondary cytoplasmic domains, such as costimulatory domains). The primary signaling domain modulates the primary activation of the TCR complex in a stimulating manner or in an inhibitory manner. Primary intracellular signaling domains that act in a stimulating manner may contain signaling motifs, which are called immunoreceptor tyrosine-based activation motifs (ITAM).

Examples of ITAM-containing primary intracellular signaling domains that are particularly useful in the present invention include intracellular signaling domains of CD3ζ, FcRγ, FcRβ, CD3γ, CD3δ, CD3ε, CD5, CD22, CD79a, CD79b, and CD66d. In one embodiment, the TFP of the invention comprises an intracellular signaling domain, such as the primary signaling domain of CD3ε. In one embodiment, the primary signaling domain comprises a modified ITAM domain, such as a mutant ITAM domain having altered (e.g., increased or decreased) activity compared with the natural ITAM domain. In one embodiment, the primary signaling domain includes a primary intracellular signaling domain containing a modified ITAM, for example, a primary intracellular signaling domain containing an optimized and/or truncated ITAM. In one embodiment, the primary signaling domain comprises one, two, three, four or more ITAM motifs.

The intracellular signaling domain of TFP may comprise the CD3ζ signaling domain alone, or it may be combined with any other desired intracellular signaling domain useful in the TFP of the present invention. For example, the intracellular signaling domain of TFP may include a CD3ε chain portion and a co-stimulatory signaling domain. The costimulatory signaling domain refers to a part of TFP that contains the intracellular domain of a costimulatory molecule. Co-stimulatory molecules are cell surface molecules other than antigen receptors or ligands thereof, which are required for effective response of lymphocytes to antigens. Examples of such molecules include CD27, CD28, 4-1BB (CD137), OX40, DAP10, DAP12, CD30, CD40, PD1, ICOS, lymphocyte function associated antigen-1 (LFA-1), CD2, CD7, LIGHT, NKG2C, B7-H3 and ligands that specifically bind to CD83, etc.

The intracellular signaling sequences in the cytoplasmic portion of the TFP of the present invention can be connected to each other in a random or specified order. Optionally, a short oligopeptide or polypeptide linker, for example, 2 to 10 amino acids in length (for example, 2, 3, 4, 5, 6, 7, 8, 9 or 10 amino acids), can form a connection between the intracellular signaling sequences.

In another aspect, the TFP-expressing cells described herein can further express another factor, such as cytokines, transcription factors, chemokines, and/or combinations thereof, to increase T cell proliferation, cell survival, anti-apoptosis effect, tumor infiltration and other functions to improve anti-tumor activity. In one embodiment, T cells expressing the TFP also express cytokine IL-7 and chemokine CCL21, cytokine IL-12 or transcription factor RUNX3, and when co-incubated with tumor cells expressing TFP targeting antigens, the TFP-T cells can significantly increase the *in vitro* killing toxicity to the tumor cells, and significantly inhibit the formation of the tumor cells *in vivo* subcutaneously xenograft tumors; and when the *in vitro* cytokine secretion was tested, it is found that the TFP-T cells can secrete a large amount of IFN-γ and Granzyme-B, in which TFP-T cells expressing IL-12 exhibit the largest secretion.

The present invention also includes RNA constructs encoding TFP that can be directly transfected into cells. The method for producing mRNA for transfection can involve *in vitro* transcription (IVT) of the template with specially designed primers, and then adding poly A to produce a construct containing 3' and 5' untranslated sequences ("UTR"), 5' cap and/or the internal ribosome entry site (IRES), the nucleic acid to be expressed and poly-A tail, usually 50-2000 bases in length. The RNA thus produced can effectively transfect different types of cells. In one aspect, the template contains the sequence of TFP. In one aspect, the anti-mesothelin TFP is encoded by messenger RNA (mRNA). In one aspect, mRNA encoding TFP against GPC3 or Claudin 18.2 is introduced into T cells to generate TFP-T cells. In one embodiment, *in vitro* transcribed RNA TFP can be introduced into cells by transient transfection. RNA is produced by *in vitro* transcription using a template generated by polymerase chain reaction (PCR). Appropriate primers and RNA polymerase can be used to directly convert DNA of interest from any source into a template for *in vitro* mRNA synthesis by PCR. The source of DNA can be, for example, genomic DNA, plasmid DNA, phage DNA, cDNA, synthetic DNA sequence or any other suitable DNA source. The required template for *in vitro* transcription is the TFP of the present invention. In one embodiment, the DNA to be used for PCR contains an open reading frame. The DNA may be derived from a naturally occurring DNA sequence in the genome of an organism. In one embodiment, the nucleic acid may comprise some or all of the 5' and/or 3' untranslated region (UTR). The nucleic acid may include exons and introns. In one embodiment, the DNA to be used for PCR is a human nucleic acid sequence. In another embodiment, the DNA to be used for PCR is a human nucleic acid sequence comprising 5' and 3' UTR. Alternatively, the DNA may be an artificial DNA sequence that is not normally expressed in a naturally occurring organism. An exemplary artificial DNA sequence is a sequence that contains portions of a gene that are joined together to form an open reading frame encoding a fusion protein. The DNA parts that are linked together can be from a single organism or from more than one organism.

PCR is used to generate a template for *in vitro* transcription of mRNA, which is used for transfection. Methods of performing PCR are well known in the art. The primer used for PCR is designed to have a region that is substantially complementary to the DNA region to be used as a PCR template. As used herein, "substantially complementary" refers to a nucleotide sequence in which most or all of the bases in the primer sequence are complementary, or one or more bases are non-complementary or mismatched. The primers that can be used for PCR can be produced by synthetic methods known in the art.

The invention also provides nucleic acid molecules encoding one or more of the TFP constructs described herein. The invention also provides a vector into which the DNA of the invention is inserted. Vectors derived from retroviruses such as lentiviruses are suitable tools to achieve long-term gene transfer since they allow long-term, stable integration of transgenes and their propagation in daughter cells. For example, nucleic acids can be cloned into vectors including but not limited to plasmids, phagemids, phage derivatives, animal viruses, and cosmids. Viruses that can be used as vectors include, but are not limited to, retrovirus, adenovirus, adeno-associated virus, herpes virus, and lentivirus. The present invention is not limited to the use of constitutive promoters, while inducible promoters are also considered. The use of an inducible promoter provides a molecular switch that can initiate the expression of an operably linked polynucleotide sequence when expression is required, or close the expression when expression is not required. Examples of inducible promoters include, but are not limited to, NFAT6 promoter, metallothionein promoter, glucocorticoid promoter, progesterone promoter, and tetracycline-regulated promoter.

Biological methods for introducing polynucleotides of interest into host cells include the use of DNA and RNA vectors. Viral vectors, especially retroviral vectors, have become the most widely used method for inserting genes into mammalian, such as human cells. Other viral vectors can be derived from lentivirus, poxvirus, herpes simplex virus I, adenovirus and adeno-associated virus.

Before expansion and genetic modification, a source of T cells is obtained from a subject. Examples of subjects include humans, dogs, cats, mice, rats, and transgenic species thereof. T cells can be obtained from many sources, including peripheral blood mononuclear cells, bone marrow, lymph node tissue, umbilical cord blood, thymus tissue, tissue from the site of infection, ascites, pleural effusion, spleen tissue, and tumors. In certain aspects of the invention, any number of T cell lines available in the art can be used. In certain aspects of the present invention, any number of techniques known to a skilled person, such as FicollTM separation technology, can be used to obtain T cells from blood units collected from a subject. In a preferred aspect, cells from the circulating blood of an individual are obtained by apheresis. Products obtained by apheresis blood apheresis usually contain lymphocytes, including T cells, monocytes, granulocytes, B cells, other nucleated white blood cells, red blood cells and platelets. In one aspect, the cells collected by apheresis can be washed to remove the plasma fraction and placed in an appropriate buffer or medium for subsequent processing steps. Multiple rounds of selection can also be used in the context of the invention. In some aspects, it may be necessary to perform a selection procedure and use "unselected" cells during activation and expansion. "Unselected" cells can also undergo other rounds of selection.

Generally, the T cell of the present invention can be expanded by contacting with a surface to which an agent that stimulates the CD3/TCR complex-related signal and a ligand that stimulates a costimulatory molecule on the surface of the T cell are attached. In particular, the T cell population can be stimulated as described herein, for example by contacting with an anti-CD3 antibody or antigen-binding fragments thereof or an anti-CD2 antibody immobilized on a surface, or by contacting a protein kinase C activator (for example, bryostatin) and calcium ionophore.

There are two splice variants for Claudin18 (CLDN18, CLD18), respectively splice variant 1 (CLDN18A1), gene registration number: NP_057453, NM016369; and splice variant 2 (CLD18A2), gene registration number : NM_001002026, NP_001002026. Claudin18 is an intrinsic transmembrane protein located in the tight junction between epithelium and endothelium. In some embodiments, the claudin 18A2 or claudin 18A2 peptide or Claudin 18.2 is a peptide comprising the amino acid sequence of SEQ ID NO: 54 or a protein/peptide of a variant of the amino acid sequence. The term "variant" refers to a mutant, splice variant, conformation, isoform, allelic variant, species variant and species homolog, especially naturally occurring variant. The allelic variant involves changes in the normal sequenc of a gene, the significance of which is usually not obvious. Whole-gene sequencing usually identifies a large number of allelic variants of a given gene. An interspecies homolog is a nucleic acid or amino acid sequence that has a different species origin from a given nucleic acid or amino acid sequence.

CLD18A2 is strongly expressed in several cancer types, including gastric cancer, esophageal cancer, pancreatic cancer and lung cancer such as non-small cell lung cancer, ovarian cancer, colon cancer, liver cancer, head and neck cancer and gallbladder cancer, gastric cancer metastasis such as Krukenberg tumor, peritoneal metastasis and lymph node metastatic to lung tumors and human cancer cell lines, and mainly expressed in the adenocarcinoma subtypes of these indications, therefore CLDN18A2 is particularly suitable for antibody-mediated prevention and/or treatment of primary tumors and metastasis targets thereof. In one example, the antigen binding portion of TFP recognizes and binds to gastric cancer, esophageal cancer, pancreatic cancer, and lung cancer, such as non-small cell lung cancer, ovarian cancer, colon cancer, liver cancer, head and neck cancer, and gallbladder cancer, and gastric cancer metastasis such as Krukenberg tumor, peritoneal metastasis and lymph node metastasis to lung tumors and epitopes in the extracellular domain of CLD18A2 expressed on human cancer cell lines.

The term "GPC3" or "Glypican 3" is a member of the Glypican family, which plays an important role in regulating cell growth and differentiation. An abnormal expression of GPC3 is closely related to the occurrence and development of a variety of tumors, such as liver cancer, lung cancer, breast cancer, ovarian cancer, kidney cancer, thyroid cancer, gastric cancer, colorectal cancer, and so on.

The term "immune effector cell" refers to a cell that exerts an effector function during an immune response, including, for example, immune cells secreting cytokines and/or chemokines, killing microorganisms, secreting antibodies, and recognizing or eliminating tumor cells. In some embodiments, immune effector cells include T cells (cytotoxic T cells, helper T cells, tumor-infiltrating T cells), B cells, natural killer cells, neutrophils, macrophages, and dendritic cells.

The term "immune effector function" includes any function mediated by the composition of the immune system, which can lead to inhibition of tumor growth and/or inhibition of tumorigenesis, including inhibition the spread and metastasis of a tumor. Preferably, the immune effector function kills tumor cells. Preferably, the immune effector function in the present invention is antibody-mediated, including complement-dependent cytotoxicity (CDC), antibody-dependent cell-mediated cytotoxicity (ADCC), antibody-dependent cell-mediated phagocytosis (ADCP), inducing apoptosis in cells carrying tumor-associated antigens (for example, through the binding of antibodies to surface antigens), inhibiting CD40L-mediated signaling (for example, binding to CD40 receptors or CD40 ligands (CD40L) through the antibodies), and/or inhibiting the proliferation of cells carrying tumor-associated antigens, preferably ADCC and/or CDC. Therefore, antibodies capable of mediating one or more immune effector functions are preferably capable of inducing CDC-mediated lysis, ADCC-mediated lysis, apoptosis, homo-adhesion and/or phagocytosis (preferably by inducing CDC-mediated Lysis and/or ADCC-mediated lysis) to mediate the killing of tumor cells. Antibodies can also function simply by binding to tumor-associated antigens on the surface of cancer cells. For example, antibodies can block the function of tumor-associated antigens or induce apoptosis by binding to tumor-associated antigens on the surface of tumor cells.

The term "antigen presenting cell" or "APC" refers to a cell in the immune system that display a complex of foreign antigens and major histocompatibility complex (MHC) on the surface, such as helper cells (such as B cells, dendritic cells, etc.). T cells can recognize these complexes using T cell receptor (TCR) thereof. APC processes the antigen and presents it to T cells.

The term "anti-tumor effect" refers to a biological effect that can be manifested in various ways, including but not limited to, for example, reduction in tumor volume, reduction in the number of tumor cells, reduction in the number of metastases, increase in life expectancy, reduction in tumor cell proliferation, and reduction in tumor cell survival rate, or improvement in various physiological symptoms related to cancerous conditions. The "anti-tumor effect" can also be expressed by the ability of the peptides, polynucleotides, cells and antibodies of the present invention to prevent tumorigenesis.

The term "autologous" refers to any material derived from an individual that will later be reintroduced into that same individual.

The term "allogeneic" refers to any material derived from a different animal or a different patient of the same species as the individual into which the material is introduced. When the genes at one or more loci are different, two or more individuals are considered to be allogeneic to each other. In some aspects, allogeneic materials from individuals of the same species may be genetically different enough for antigenic interaction to occur.

The term "xenogeneic" refers to animals in which the grafts are derived from different species.

The term "genetically engineered cell" refers to a cell modified by means of genetic engineering.

The terms "therapeutically effective amount", "therapeutically effective", "effective amount" or "in an effective amount" are used interchangeably herein and refer to the amount of a compound, preparation, substance or composition that is effective to achieve a specific biological result as described herein, such as but not limited to an amount or dose sufficient to promote T cell response. When indicating "immunologically effective amount", "anti-tumor effective amount", "tumor-suppressing effective amount" or "therapeutically effective amount", the precise number of immune effector cells and therapeutic agents of the present invention to be administered can be determined by a physician in consideration of the individual's age, weight, tumor size, degree of infection or metastasis, and the condition of a patient (subject). An effective amount of immune effector cells refers to, but is not limited to, the number of immune effector cells which can increase, enhance or prolong the anti-tumor activity of immune effector cells; increase the number of anti-tumor immune effector cells or activated immune effector cells; promote IFN-γ secretion, tumor regression and tumor shrinkage and tumor necrosis.

CD3 (Cluster of Differentiation 3) T cell co-receptor is a protein complex composed of four different chains. In mammals, the complex contains one CD3γ chain, CD3δ chain, and two CD3ε chains. These chains have a molecule of accessory T cell receptor (TCR) and zeta-chain to generate activation signals for T lymphocytes. The TCR, ζ chain and CD3 molecule together constitute a T cell receptor complex. The CD3 molecule is connected to the T cell receptor (TCR) through a salt bridge to form a TCR-CD3 complex, which participates in the signaling of T cells, and is mainly used to label thymocytes, T lymphocytes and T cell lymphomas. The cytoplasmic segment of CD3 contains immunoreceptor tyrosine-based activation motif (ITAM). TCR recognizes and binds to the antigen peptide presented by the MHC (major histo-compatibility complex) molecule, resulting in the tyrosine residues in the conserved sequenceof ITAM of CD3. being phosphorylated by the tyrosine protein kinase p561ck in T cells, and then recruiting other tyrosine protein kinases (such as ZAP-70) containing SH2 (Scr homology 2) domain. The phosphorylation of ITAM and the binding to ZAP-70 are one of the important biochemical reactions in the early stages of the signaling process of T cell activation. Therefore, the function of the CD3 molecule is to transduce the activation signal generated by the TCR to recognize the antigen. In this application, the exogenous receptor that can bind to the target antigen and trigger CD3 signal activation includes at least one CD3 binding site and at least one additional antigen binding site specific to bacterial substance, viral protein, autoimmune marker, or antigen present specific cells (e.g., cell surface proteins of B cells, T cells, natural killer (NK) cells, bone marrow cells, phagocytes, or tumor cells). Such exogenous receptors can cross-link two kinds of cells and can be used to direct T cells to specific targets and trigger the cytotoxic activity of T cells on the target cells. Examples of such targets may be tumor cells or infectious agents, such as viral pathogens or bacterial pathogens.

The term "stimulation" refers to a primary response induced by the binding of a stimulation domain or a stimulating molecule (eg, TCR/CD3 complex) to its cognate ligand, thereby mediating signaling events, such as, but not limited to, signaling via TCR/ CD3 complex. The stimulation can mediate changes in the expression of certain molecules and/or reorganization of the cytoskeleton structure.

The term "stimulatory molecule" or "stimulatory domain" refers to a molecule or a part thereof expressed by T cells, which provides a primary cytoplasmic signaling sequence that modulates the primary activation of the TCR complex in a stimulating manner for at least some aspects of the T cell signaling pathway. In one aspect, the primary signal is initiated by, for example, the binding of the TCR/CD3 complex to the peptide-loaded MHC molecule, and it leads to the mediation of T cell responses including but not limited to proliferation, activation, differentiation, etc. Primary cytoplasmic signaling sequences that act in a stimulating manner (also referred to as "primary signaling domains") may contain signaling motifs, which are called immunoreceptor tyrosine-based activation motifs or "ITAM". Examples of ITAM-containing primary cytoplasmic signaling sequences that are particularly useful in the present invention include but are not limited to those derived from TCRζ, FcRγ, FcRβ, CD3γ, CD3δ, CD3ε, CD5, CD22, CD79a, CD79b, CD278 (also known as "ICOS ") and CD66d.

The term "intracellular signaling domain" refers to the intracellular part of a molecule. Intracellular signaling domains generate signals that promote immune effector functions of TFP-containing cells, such as T cells expressing TFP. For example, examples of immune effector functions in T cells expressing TFP include cytolytic activity and T helper cell activity, including secretion of cytokines. In one embodiment, the intracellular signaling domain may comprise a primary intracellular signaling domain. Exemplary primary intracellular signaling domains include intracellular signaling domains derived from molecules responsible for primary stimulation or antigen-dependent stimulation. In one embodiment, the intracellular signaling domain may comprise a costimulatory intracellular domain. Exemplary costimulatory intracellular signaling domains include intracellular signaling domains derived from molecules responsible for costimulatory signals or antigen-independent stimulation.

The primary intracellular signaling domain may contain ITAM ("immunoreceptor tyrosine-based activation motif"). Examples of ITAM-containing primary cytoplasmic signaling sequences include, but are not limited to, those derived from CD3ζ, FcRγ, FcRβ, CD3γ, CD3δ, CD3ε, CD5, CD22, CD79a, CD79b, and CD66d DAP10 and DAP12.

The term "costimulatory molecule" refers to a homologous binding partner on T cells, which specifically binds to a costimulatory ligand, thereby mediating the costimulatory response of T cells, such as but not limited to proliferation. Co-stimulatory molecules are cell surface molecules other than antigen receptors or their ligands required for an effective immune response. Costimulatory molecules include but are not limited to MHC class 1 molecules, BTLA and Toll ligand receptors, as well as DAP10, DAP12, CD30, LIGHT, OX40, CD2, CD27, CD28, CDS, ICAM-1, LFA-1 (CD11a/CD18) and 4-1BB (CD137). The costimulatory intracellular signaling domain can be the intracellular part of a costimulatory molecule. Costimulatory molecules can be represented in the following protein families: TNF receptor proteins, immunoglobulin-like proteins, cytokine receptors, integrins, signaling lymphocyte activation molecules (SLAM proteins), and activating NK cell receptors. Examples of such molecules include CD27, CD28, 4-1BB (CD137), OX40, GITR, CD30, CD40, ICOS, BAFFR, HVEM, lymphocyte function associated antigen 1 (LFA-1), CD2, CD7, LIGHT, NKG2C , SLAMF7, NKp80, CD160, B7-H3 and ligands that specifically bind to CD83. The intracellular signaling domain may comprise the entire intracellular part of the molecule from which it is derived or the entire natural intracellular signaling domain, or a functional fragment thereof. The term "4-1BB" refers to a member of the TNFR superfamily, which has the amino acid sequence provided under GenBank accession number AAA62478.2. or equivalent residues from non-human species such as mice, rodents, monkeys, apes, etc.; and "4-1BB costimulatory domain" is defined as amino acid residues 214-255 of GenBank accession number AAA62478.2. or equivalent residues from non-human species such as mice, rodents, monkeys, apes, etc.

The term "encoding" refers to the inherent properties of polynucleotides such as genes, cDNAs, or mRNAs in which specific nucleotide sequences are used as templates for the synthesis of other polymers and macromolecules in biological processes. The polymers and macromolecules have a certain Nucleotide sequence (e.g., rRNA, tRNA, and mRNA) or defined amino acid sequence and the resulting biological properties. Therefore, if the transcription and translation of mRNA corresponding to a gene produces a protein in a cell or other biological system, the gene, cDNA, or RNA encodes the protein. The coding strand and its nucleotide sequence are identical to the mRNA sequence and are usually provided in the sequence listing, while the non-coding strand used as a template for the transcription of a gene or cDNA can be referred to as a coding protein or other products of the gene or cDNA. Unless otherwise specified, "nucleotide sequence encoding an amino acid sequence" includes all nucleotide sequences that are degenerate forms of each other and encode the same amino acid sequence. The phrase "nucleotide sequence" encoding a protein or RNA may also include introns to the extent that the nucleotide sequence encoding the protein may contain one or more introns in some forms.

The term "expression" refers to the transcription and/or translation of a specific nucleotide sequence driven by a promoter.

The term "transfer vector" refers to a composition containing an isolated nucleic acid and a substance that can be used to deliver the isolated nucleic acid to the inside of a cell. Many vectors are known in the art, including but not limited to linear polynucleotides, polynucleotides associated with ionic or amphiphilic compounds, plasmids, and viruses. Therefore, the term "transfer vector" includes autonomously replicating plasmids or viruses. The term should also be interpreted to further include non-plasmid and non-viral compounds that facilitate the transfer of nucleic acids into cells, such as polylysine compounds, liposomes, and the like. Examples of virus transfer vectors include, but are not limited to, adenoviral vectors, adeno-associated virus vectors, retroviral vectors, lentiviral vectors, and the like.

The term "expression vector" refers to a vector comprising a recombinant polynucleotide comprising an expression control sequence operably linked to the nucleotide sequence to be expressed. The expression vector contains sufficient cis-acting elements for expression; other elements for expression can be provided by the host cell or in an in vitro expression system. Expression vectors include all expression vectors known in the art, including cosmids, plasmids (for example, naked or contained in liposomes), and viruses incorporating recombinant polynucleotides (for example, lentivirus, retrovirus, adenovirus). Virus and adeno-associated virus).

The term "homology" or "identity" refers to the identity of subunit sequence between two polymer molecules, for example, between two nucleic acid molecules, such as two DNA molecules or two RNA molecules, or between two polypeptide molecules. When subunit positions in two molecules are occupied by the same monomer subunit; for example, if the position of each of two DNA molecules is occupied by adenine, they are homologous or identical at that position. The homology between two sequences is a direct function of the number of matching or homologous positions; for example, if half of the positions in the two sequences (for example, 5 positions in a polymer of 10 subunits in length) are homologous, the two sequences are 50% homologous; if 90% of the positions (for example, 9 out of 10) are matched or homologous, then the two sequences are 90% homologous .

In the context of two or more nucleic acid or polypeptide sequences, identity percent refers to two or more sequences that are the same. When comparing and aligning for maximum correspondence in a comparison window or a designated area, as measured by using one of the following sequence comparison algorithms or by manual alignment and visual inspection, if the two sequences have a specified percentage of identical amino acid residues or nucleotides (e.g., 60% identity, optionally 70%, 71%, 72%, 73%, 74 %, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity over a specified region, or if not specified, over the entire sequence), then the two sequences are "substantially the same". Optionally, the identity exists over a region of at least about 50 nucleotides (or 10 amino acids) in length, or more preferably over a region of 100 to 500 or 1000 or more nucleotides in length (Or 20, 50, 200 or more amino acids). For sequence comparison, usually a sequence serves as a reference sequence against which the test sequence is compared. When a sequence comparison algorithm is used, a test sequence and a reference sequence are input into a computer, and the sub-sequence coordinates and the sequence algorithm program parameters are specified, if necessary. Default program parameters can be used, or alternative parameters can be specified. Subsequently, the sequence comparison algorithm calculates the percent sequence identity of the test sequence relative to the reference sequence based on the program parameters. Methods of sequence alignment for comparison are well known in the art. In one aspect, the invention contemplates modification of the amino acid sequence of the starting antibody or fragment (e.g., scFv) that produces a functionally equivalent molecule. For example, the anti-GPC3 or Claudin 18.2 binding domain contained in TFP, such as the VH or VL of scFv, can be modified to retain at least about 70%, 71%, 72%, 73%, 74%, 75%, 76%, 77%, 78%, 79%, 80%, 81%, 82%, 83%, 84%, 85%, 86%, 87 %, 88%, 89%, 90%, 91%, 92%, 93%, 94%, 95%, 96%, 97%, 98%, 99% identity to the initial VH or VL framework region of the anti-GPC3 or Claudin 18.2 binding domain, such as scFv. The present invention considers the modification of the entire TFP construct, for example, the modification on one or more amino acid sequences of each domain of the TFP construct, for producing a functionally equivalent molecule. The TFP construct can be modified to retain at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98 % or 99% identity of the starting TFP construct.

In a specific embodiment, the amino acid sequences of the anti-GPC3 antibody that can be used in the present invention and its CDR sequence, heavy chain variable region and light chain variable region are shown in SEQ ID NO: 23, 26-33 and 137-208. In a preferred embodiment, the amino acid sequences of the anti-GPC3 antibody used in the present invention and it's CDR sequence, heavy chain variable region and light chain variable region are shown in SEQ ID NOs: 23 and 26-33.

In one embodiment, the amino acid sequences of the anti-Claudin 18.2 antibody that can be used in the present invention and its CDR sequence, heavy chain variable region and light chain variable region are shown in SEQ ID NO: 1, 15-22 and 56-136. In a preferred embodiment, the amino acid sequences of the anti-Claudin 18.2 antibody used in the present invention and its CDR sequence, heavy chain variable region and light chain variable region are shown in SEQ ID NO: 1 and 15-22.

The term "isolated" means changed or removed from the natural state. For example, a nucleic acid or peptide naturally present in a living animal is not "isolated", but the same nucleic acid or peptide that is partially or completely separated from a substance co-existing in its natural state is "isolated." The isolated nucleic acid or protein may exist in a substantially purified form or may exist in a non-natural environment such as a host cell.

The term "operably linked" or "transcription control" refers to a functional linkage between a regulatory sequence and a heterologous nucleic acid sequence, which results in the expression of the latter. For example, when the first nucleic acid sequence and the second nucleic acid sequence are arranged in a functional relationship, the first nucleic acid sequence and the second nucleic acid sequence are operably linked. For example, if a promoter affects the transcription or expression of a coding sequence, the promoter is operably linked to the coding sequence. The operably linked DNA sequences may be adjacent to each other, and for example, in the case where two protein coding regions need to be linked, the DNA sequences are in the same reading frame.

The term "nucleic acid" or "polynucleotide" refers to deoxyribonucleic acid (DNA) or ribonucleic acid (RNA) and polymers thereof in single-stranded or double-stranded form. Unless specifically defined, the term includes nucleic acids containing known analogs of natural nucleotides that have binding properties similar to the reference nucleic acid and are metabolized in a manner similar to naturally occurring nucleotides. Unless otherwise specified, a specific nucleic acid sequence also implicitly includes its conservatively modified variants (e.g., degenerate codon substitutions), alleles, orthologs, SNPs and complementary sequences, as well as explicitly indicated sequences. Specifically, degenerate codon replacement can be achieved by generating a sequence in which the third position of one or more selected (or all) codons is replaced by mixed bases and/or deoxyinosine residues.

The terms "peptide", "polypeptide" and "protein" are used interchangeably and refer to a compound composed of amino acid residues covalently linked by peptide bonds. A protein or peptide must contain at least two amino acids, and there is no limit to the maximum number of amino acids that can constitute a protein or peptide sequence. Polypeptides include any peptide or protein comprising two or more amino acids connected to each other by peptide bonds. As used herein, the term refers to both short and long chains. Short chains are also commonly referred to in the art as peptides, oligopeptides, and oligomers, and long chains are commonly referred to as proteins in the art, and there are many types. "Polypeptide" includes, for example, biologically active fragments, substantially homologous polypeptides, oligopeptides, homodimers, heterodimers, polypeptide variants, modified polypeptides, derivatives, analogs, fusion proteins, and the like. Polypeptides include natural peptides, recombinant peptides or a combination thereof.

The term "promoter/regulatory sequence" refers to a nucleic acid sequence required to express a gene product operably linked to a promoter/regulatory sequence. The term "constitutive" promoter refers to a nucleotide sequence that, when operably linked to a polynucleotide encoding or specifying a gene product, results in the production of a gene product in the cell under most or all physiological conditions of the cell. The term "inducible" promoter means that when operably linked to a polynucleotide encoding a specified gene product, it basically results in the production of a gene in the cell only when the inducer corresponding to the promoter is present in the cell The nucleotide sequence of the product.

*"In vitro* transcribed RNA" refers to RNA that has been synthesized *in vitro,* preferably mRNA. Generally, *in vitro* transcribed RNA is produced by an *in vitro* transcription vector. The *in vitro* transcription vector contains a template for producing *in vitro* transcribed RNA.

The term "antibody" refers to a protein or polypeptide sequence derived from an immunoglobulin molecule that specifically binds to an antigen. Antibodies can be polyclonal or monoclonal, multi-chain or single-chain, or whole immunoglobulins, and can be derived from natural sources or recombinant sources. The antibody may be a tetramer of immunoglobulin molecules.

The term "antibody fragment" refers to at least a portion of an antibody that retains the ability to specifically interact with an epitope of an antigen (e.g., through binding, steric hindrance, stabilization/destabilization, spatial distribution). Examples of antibody fragments include, but are not limited to, Fab, Fab', F(ab')₂, Fv fragments, scFv antibody fragments, disulfide-linked Fvs (sdFv), Fd fragments composed of VH and CH1 domains, linear antibodies, single domain antibodies such as sdAb (VL or VH), camelid VHH domains, multispecific antibodies formed by antibody fragments (e.g., bivalent fragments including two Fab fragments connected by disulfide bonds in the hinge region) and isolated CDR or other epitope binding fragments of antibodies. Antigen-binding fragments can also be incorporated into single domain antibodies, maximal antibodies, minibodies, nanobodies, intracellular antibodies, diabodies, tribodies, tetrabodies, v-NAR and double-scFv (see, for example, Hollinger and Hudson, " Nature Biotechnology" (23): 1126-1136, 2005).

The term "scFv" refers to a fusion protein comprising at least one antibody fragment including a light chain variable region and at least one antibody fragment including a heavy chain variable region, wherein the light chain and heavy chain variable regions are contiguous (For example, via a synthetic linker such as a short flexible polypeptide linker), and can be expressed as a single-chain polypeptide, and wherein the scFv retains the specificity of the intact antibody from which it is derived. Unless specified, as used herein, the scFv may have the VL and VH variable regions in any order (for example, relative to the N-terminus and C-terminus of the polypeptide), and the scFv may include VL-linker-VH or may include VH-Linker-VL.

The term "antibody heavy chain" refers to the larger of the two polypeptide chains present in the antibody molecule in its naturally occurring configuration and usually determines the type of antibody to which it belongs.

The term "antibody light chain" refers to the smaller of the two polypeptide chains present in an antibody molecule in its naturally occurring configuration. κ(k) and λ(1) light chains refer to two main isotypes of antibody light chains.

The term "recombinant antibody" refers to an antibody produced using recombinant DNA technology, such as, an antibody expressed by a phage or yeast expression system. The term should also be interpreted as referring to antibodies that have been produced by synthesizing a DNA molecule encoding the antibody (and wherein the DNA molecule expresses the antibody protein) or the amino acid sequence of the specified antibody, wherein the DNA or amino acid sequence has been obtained by using recombinant DNA or amino acid sequence technology is available and the well-known in the art.

A skilled person in the art will understand that the antibodies or antibody fragments of the present invention can be further modified so that there are changes in amino acid sequence (for example, relative to the wild type), but no change in desired activities. For example, additional nucleotide substitutions can be made to the protein, resulting in amino acid substitutions at "non-essential" amino acid residues. For example, a non-essential amino acid residue in the molecule can be replaced by another amino acid residue from the same side chain family. In another embodiment, the amino acid string may be replaced by a string that is similar in structure but different in sequence and/or composition from a member of the side chain family. For example, conservative substitutions may be made in which the amino acid residue is replaced by an amino acid residue having a similar side chain.

In the art, families of amino acid residues with similar side chains have been defined, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), non-polar side chains (e.g., alanine, valine, leucine, isoleucine, proline, phenylalanine, methionine, tryptophan), β-branched side chains (e.g., threonine, valine, Isoleucine) and aromatic side chains (e.g., tyrosine, phenylalanine, tryptophan, histidine).

The term "antigen" or "Ag" refers to a molecule that causes an immune response. The immune response may involve the production of antibodies or the activation of cells with specific immunity, or both. A skilled person in the art should understand that any macromolecule including virtually all proteins or peptides can serve as an antigen. In addition, the antigen can be derived from recombinant or genomic DNA. When the term is used herein, a skilled person in the art should understand that the term includes any DNA including a nucleotide sequence or part of a nucleotide sequence encoding a protein that causes an immune response, and therefore encoding an "antigen". In addition, a skilled person in the art should understand that the antigen need not be encoded only by the full-length nucleotide sequence of the gene. It is obvious that the present invention includes, but is not limited to, the use of partial nucleotide sequences of more than one gene, and these nucleotide sequences are arranged in different combinations to encode polypeptides eliciting a desired immune response. Moreover, those skilled in the art should understand that the antigen does not need to be encoded by a "gene" at all. It is obvious that the antigen can be produced synthetically, or it can be derived from a biological sample, or it can be a macromolecule other than a polypeptide. Such biological samples may include, but are not limited to, tissue samples, tumor samples, cells or fluids with other biological components.

The term "antigen recognition unit" as used herein refers to immunoglobulin molecules and immunologically active parts of immune molecules, that is, a molecule that contains an antigen binding site that specifically binds to an antigen ("immune response"). The term "antigen recognition unit" also includes immunoglobulin molecules derived from various species, including invertebrates and vertebrates. Structurally, the simplest naturally occurring antibody (e.g., IgG) contains four polypeptide chains, two heavy (H) chains and two light (L) chains interconnected by disulfide bonds. Immunoglobulins represent a large family of molecules including several types of molecules, such as IgD, IgG, IgA, IgM, and IgE. The term "immunoglobulin molecule" includes, for example, hybrid antibodies or modified antibodies and fragments thereof. It has been shown that the antigen-binding function of antibodies can be performed by fragments of naturally-occurring antibodies. These fragments are collectively referred to as "antigen recognition units". The term "antigen recognition unit" also includes any molecular structure containing a polypeptide chain that has a specific shape that matches the epitope and recognizes the epitope, in which one or more non-covalent binding interactions stabilize the complex between the molecular structure and the epitope. Examples of the antigen recognition unit include Fab fragments, monovalent fragments consisting of VL, VH, CL and CH1 domains, and bivalent fragments (F(ab)2 fragments); Fd fragments composed of VH and CH1 domains, Fv fragments composed of single-arm VL and VH domains of antibodies; dAb fragments composed of VH domains (Ward et al., Nature, 341:544- 546, 1989); and an isolated complementarity determining region (CDR) or any fusion protein containing such an antigen recognition unit.

If the antigen recognition unit binds to an antigen with greater affinity or avidity compared with binding with other reference antigens (including polypeptides or other substances), the antigen recognition unit "specifically binds" to the antigen or is "immunoreactive with the antigen".

"Tumor antigen" refers to an antigen common to specific hyperproliferative diseases. In certain aspects, the hyperproliferative disorder antigens of the invention are derived from cancer. The tumor antigens of the present invention include but are not limited to: Thyroid Stimulating Hormone Receptor (TSHR); CD171; CS-1; C-type lectin-like molecule-1; Ganglioside GD3; Tn antigen; CD19; CD20; CD 22; CD 30; CD 70; CD 123; CD 138; CD33; CD44; CD44v7/8; CD38; CD44v6; B7H3 (CD276), B7H6; KIT (CD117); Interleukin 13 receptor subunit α (IL-13Rα); Interleukin 11 receptor alpha (IL-11Rα); prostate stem cell antigen (PSCA); prostate specific membrane antigen (PSMA); carcinoembryonic antigen (CEA); NY-ESO-1; HIV-1 Gag; MART-1; gp100; Tyrosinase; Mesothelin; EpCAM; Protease Serine 21 (PRSS21); Vascular Endothelial Growth Factor Receptor, Vascular Endothelial Growth Factor Receptor 2 (VEGFR2); Lewis (Y) Antigen; CD24; Platelet Derived Growth Factor Receptor β (PDGFR-β); stage-specific embryonic antigen-4 (SSEA-4); cell surface-associated mucin 1 (MUC1), MUC6; epidermal growth factor receptor family and its mutants (EGFR, EGFR2, ERBB3, ERBB4, EGFRvIII); Neural cell adhesion molecule (NCAM); Carbonic anhydrase IX (CAIX); LMP2; Ephrin A receptor 2 (EphA2); Fucosyl GM1; Sialyl Lewis adhesion molecule (sLe); Ganglioside GM3(aNeu5Ac(2-3)bDGalp(1-4)bDGlcp(1-1)Cer; TGS5; High molecular weight melanoma-associated antigen (HMWMAA); o-acetyl GD2 ganglioside (OAcGD2); Folate receptor; Tumor vascular endothelial marker 1 (TEM1/CD248); Tumor vascular endothelial marker 7 related (TEM7R); Claudin 6, Claudin 18.2, Claudin 18.1; ASGPR1; CDH16; 5T4; 8H9; αvβ6 Integrin; B cell maturation antigen (BCMA); CA9; kappa light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; Fibronectin; Tenascin; Carcinoembryonic variant of tumor necrosis zone; G protein coupled receptor C class 5 group-member D (GPRC5D); X chromosome open reading frame 61 (CXORF61); CD97; CD17 9a; Anaplastic lymphoma kinase (ALK); polysialic acid; placenta specific 1 (PLAC1); hexose part of globoH glycoceramide (GloboH); breast differentiation antigen (NY-BR-1); uroplakin 2 (UPK2); Hepatitis A virus cell receptor 1 (HAVCR1); adrenergic receptor β3 (ADRB3); pannexin 3 (PANX3); G protein-coupled receptor 20 (GPR20); lymphocyte antigen 6 complex locus K9 (LY6K); Olfactory receptor 51E2 (OR51E2); TCRγ alternating reading frame protein (TARP); Wilms tumor protein (WT1); ETS translocation variant gene 6 (ETV6-AML); Sperm protein 17 (SPA17); X antigen family member 1A (XAGE1); Angiopoietin binds to cell surface receptor 2 (Tie2); Melanoma cancer testis antigen-1 (MAD-CT-1); Melanoma cancer testis antigen-2 (MAD-CT-2); Fos related antigen 1; p53 mutant; human telomerase reverse transcriptase (hTERT); sarcoma translocation breakpoint; melanoma inhibitor of apoptosis (ML-IAP); ERG (transmembrane protease serine 2 (TMPRSS2))ETS fusion gene); N-acetylglucosaminyltransferase V (NA17); Pairing box protein Pax-3 (PAX3); Androgen receptor; Cyclin B1; V-myc avian myeloidoma virus cancer Gene neuroblastoma-derived homolog (MYCN); Ras homolog family member C (RhoC); Cytochrome P450 1B1 (CYP1B1); CCCTC binding factor (zinc finger protein)-like (BORIS); recognized by T cells Squamous cell carcinoma antigen 3 (SART3); paired box protein Pax-5 (PAX5); proacrosin binding protein sp32 (OYTES1); lymphocyte-specific protein tyrosine kinase (LCK); A kinase anchor protein 4 (AKAP -4); Synovial sarcoma X breakpoint 2 (SSX2); CD79a; CD79b; CD72; Leukocyte-associated immunoglobulin-like receptor 1 (LAIR1); Fc fragment of IgA receptor (FCAR); Leukocyte immunoglobulin-like receptor Body subfamily member 2 (LILRA2); CD300 molecular-like family member f (CD300LF); C-type lectin domain family 12 member A (CLEC12A); bone marrow stromal cell antigen 2 (BST2); mucin-like hormone Receptor-like 2 containing EGF-like module (EMR2); Lymphocyte antigen 75 (LY75); Glypican-3 (GPC3); Fc receptor-like 5 (FCRL5); Immunoglobulin lambda-like polypeptide 1 (IGLL1).

The pathogen antigen is selected from: antigens from virus, bacteria, fungus, protozoa, or parasite; and the virus antigen is selected from: cytomegalovirus antigen, Epstein-Barr virus antigen, human immunodeficiency virus antigen, or influenza virus antigen.

The term "tumor heterogeneity" means that, after multiple divisions and proliferation during the growth of a tumor, daughter cells of the tumor its show molecular biological or genetic changes, so that there are differences in the growth rate, invasion ability, and drug sensitivity, prognosis and other aspects of the tumor. It is one of the characteristics of malignant tumors.

The term "cancer" refers to a broad category of disorders characterized by hyperproliferative cell growth *in vitro* (e.g., transformed cells) or *in vivo.* The conditions that can be treated or prevented by the method of the present invention include, for example, various neoplasms, including benign or malignant tumors, various hyperplasias, and the like. The method of the present invention can achieve the inhibition and/or reversal of the undesirable hyperproliferative cell growth involved in such conditions. Specific examples of cancer include, but are not limited to: blood cancer, colon cancer, rectal cancer, renal cell carcinoma, liver cancer, non-small cell carcinoma of the lung, small intestine cancer, esophageal cancer, melanoma, bone cancer, pancreatic cancer, skin cancer , Head and neck cancer, skin or intraocular melanoma, uterine cancer, ovarian cancer, rectal cancer, anal cancer, stomach cancer, testicular cancer, uterine cancer, fallopian tube cancer, endometrial cancer, cervical cancer, vagina cancer, vaginal cancer, Hodgkin's disease, non-Hodgkin's lymphoma, endocrine system cancer, thyroid cancer, parathyroid cancer, adrenal cancer, soft tissue sarcoma, urethral cancer, penile cancer, childhood solid tumors, bladder cancer, kidney or ureter cancer, Renal pelvis cancer, central nervous system (CNS) tumor, primary CNS lymphoma, tumor angiogenesis, spinal tumor, brain stem glioma, pituitary adenoma, Kaposi's sarcoma, epidermoid carcinoma, squamous cell carcinoma, T-cell lymphoma, environmentally induced cancer, a combination of the cancers and the metastatic foci of the cancers.

The term "transfected" or "transformed" or "transduced" refers to the process by which exogenous nucleic acid is transferred or introduced into a host cell. A "transfected" or "transformed" or "transduced" cell is a cell that has been transfected, transformed or transduced with exogenous nucleic acid. The cells include primary cell of a subject and progenies thereof.

The term "specifically binds" refers to an antibody or ligand that recognizes and binds a protein of a binding partner (such as a tumor antigen) present in a sample, but the antibody or ligand does not substantially recognize or bind to other molecules in the sample.

"Refractory" as used herein refers to a disease, such as cancer, which does not respond to treatment. In an embodiment, a refractory cancer may be resistant to treatment before or at the beginning of the treatment. In other embodiments, a refractory cancer may become resistant during treatment. Refractory cancers are also called resistant cancers. In the present invention, refractory cancers include, but are not limited to, cancers that are not sensitive to radiotherapy, relapse after radiotherapy, are not sensitive to chemotherapy, relapse after chemotherapy, are not sensitive to CAR-T treatment, or relapse after treatment. Refractory or recurrent malignant tumors can use the treatment regimens described herein.

"Relapsed" as used herein refers to the return of the signs and symptoms of a disease (e.g. cancer) or the return of a disease such as cancer during a period of improvement, for example, after a therapy, such as a previous treatment of cancer therapy.

The terms "individual" and "subject" have the same meaning herein, and can be a human and animal from other species. A "patient" is a subject who has a disease, disorder, or condition, or is at risk of suffering from a disease, disorder, or condition, or is otherwise in need of the compositions and methods provided herein.

The term "enhancement" refers to allowing a subject or tumor cell to improve its ability to respond to the treatment disclosed herein. For example, an enhanced response may include an increase of 5%, 10%, 15%, 20%, 25%, 30%, 35%, 40%, 45%, 50%, 55%, 60%, 65%, 70%, 75%, 80%, 85%, 90%, 95%, or 98% or highere in responsiveness. As used herein, "enhancing" can also refer to increasing the number of subjects responding to treatment, such as immune effector cell therapy. For example, an enhanced response may refer to the total percentage of subjects responding to treatment, where the percentages are 5%, 10%, 15%, 20%, 25%, 30%, 35%, 40%, 45%, 50%, 55 %, 60%, 65%, 70%, 75%, 80%, 85%, 90%, 95% or 98% more.

In one aspect, treatment is judged by clinical results, and can also be based on the increase, enhancement or extension of the anti-tumor activity of T cells, for example the increase in the number of anti-tumor T cells or activated T cells promotes IFN-γ secretion , both of them, as compared with the number before treatment. In another aspect, the clinical outcome is tumor regression; tumor shrinkage; tumor necrosis; anti-tumor response through the immune system; tumor enlargement, recurrence or spread, or a combination thereof. In an additional aspect, the therapeutic effect is predicted by the presence of T cells, the presence of genetic markers indicative of T cell inflammation, promotion of IFN-γ secretion, or a combination thereof.

The cells as disclosed herein can be administered to an individual by various routes, including, for example, oral or parenteral, such as intravenous, intramuscular, subcutaneous, intraorbital, intrasaccular, intraperitoneal, intrarectal, intracisternal, intratumoral, intravasal, intradermal administration, or passive or promoted absorption through the skin using, for example, skin patches or transdermal iontophoresis, respectively.

The total amount of agent to be administered in practicing the method of the present invention can be administered to the subject as a single dose as a bolus or by infusion over a relatively short period of time, or can be administered using a graded treatment regimen, wherein multiple doses are administered in segments. A skilled person will know that the amount of the composition to treat a pathological condition in a subject depends on many factors, including the age and general health of the subject, as well as the route of administration and the number of treatments to be administered. Based on these factors, a skilled person will adjust the specific dosage as needed. Generally, phase I and phase II clinical trials are initially used to determine the formulation of the composition as well as the route and frequency of administration.

Range: Throughout this disclosure, various aspects of the invention may be presented in a range. It should be understood that the description of a range is merely for convenience and brevity, and should not be regarded as an unchangeable limitation on the scope of the present invention. Therefore, the description of a range should be considered as specifically disclosing all possible subranges and individual values within that range. For example, the description of a range such as from 1 to 6 should be considered to specifically disclose subranges such as 1 to 3, 1 to 4, 1 to 5, 2 to 4, 2 to 6, 3 to 6, etc., and individual values within the range, such as 1, 2, 2.7, 3, 4, 5, 5.3, and 6. As another example, a range such as 95-99% identity includes a range with 95%, 96%, 97%, 98%, or 99% identity, and includes sub-ranges such as 96-99%, 96-98%, 96-97%, 97-99%, 97-98% and 98-99% identity. This applies regardless of the width of the range.

Based on the present disclosure, a skilled person should understand that many changes can be made in the disclosed specific embodiments and still obtain the same or similar results without departing from the spirit and scope of the present invention. The present invention is not limited in scope to the specific embodiments described herein (which are only intended to exemplify aspects of the present invention), and functionally equivalent methods and components shall fall within the scope of the present invention. In fact, the various modifications of the present invention as well as those shown and described herein will become apparent to a skilled person based on the foregoing description.

### GPC3 and GPC3-positive tumors

As used herein, "GPC3" or "Glypican 3" is a member of the Glypican family, with gene registration numbers: NM_016697.3, NP_057906.2, which play an important role in regulating cell growth and differentiation. Abnormal expression of GPC3 is closely related to the occurrence and development of a variety of tumors, such as liver cancer, lung cancer, breast cancer, ovarian cancer, kidney cancer, thyroid cancer, gastric cancer, colorectal cancer, and so on.

In the present invention, immune effector cells target tumors that positively express GPC3. In a specific embodiment, the tumor includes, but is not limited to, liver cancer, stomach cancer, lung cancer, esophageal cancer, head and neck cancer, bladder cancer, ovarian cancer, cervical cancer, kidney cancer, pancreatic cancer, cervical cancer, liposarcoma, melanoma, adrenal carcinoma, schwannoma, malignant fibrous histiocytoma, esophageal cancer. A skilled person will know that some tumor cells, such as liver cancer cells, are not sensitive to many drugs. Therefore, even drugs that are effective *in vitro* may have poor effects *in vivo,* or even no effect. Therefore, in a preferred embodiment, the GPC3-positive tumors or GPC-positive tumors described herein are liver cancer, gastric cancer, lung cancer, and esophageal cancer.

### TCR modified T cells

The present invention also provides TCR-modified T cells, which are transduced with a nucleic acid encoding the TCR or with the aforementioned recombinant plasmid containing the nucleic acid, or a virus containing the plasmid. Conventional nucleic acid transduction methods in the art, including non-viral and viral transduction methods, can be used in the present invention. Non-viral-based transduction methods include electroporation and transposon methods. Recently, the Nucleofector nuclear transfection instrument developed by Amaxa can directly introduce foreign genes into the nucleus to obtain efficient transduction of the target gene. In addition, the transduction efficiency of transposon systems based on Sleeping Beauty system or PiggyBac transposon is much higher than that of ordinary electroporation. The combined application of nucleofector transfection instrument and Sleeping Beauty transposon system has been reported [Davies JK., et al. Combining CD19 redirection and alloanergization to generate tumor-specific human T cells for allogeneic cell therapy of B-cell malignancies. Cancer Res, 2010, 70(10): OF1-10.], and this method not only has high transduction efficiency but also can realize the targeted integration of the target gene. In one embodiment of the present invention, the method for transduction of immune effector cells that achieve chimeric antigen receptor gene modification is a transduction method based on viruses, such as retroviruses or lentiviruses. The method has the advantages of high transduction efficiency, stable expression of foreign genes, and shortening the time for culturing immune effector cells to reach clinical level *in vitro.* On the surface of the transgenic immune effector cell, the transduced nucleic acid is expressed on its surface through transcription and translation. Through *in vitro* cytotoxicity experiments on various cultured tumor cells, it is proved that the immune effector cells modified by the chimeric antigen of the present invention have highly specific tumor cell killing effects (also known as cytotoxicity), and can be found in tumor tissues. Effectively survive. Therefore, the nucleic acid encoding the chimeric antigen receptor of the present invention, the plasmid containing the nucleic acid, the virus containing the plasmid, and the transgenic immune effector cells transduced with the nucleic acid, plasmid or virus of the present invention can be effectively used for tumor immunotherapy.

The TCR-modified T cells of the present invention can be applied to the preparation of pharmaceutical compositions or diagnostic reagents. In addition to including an effective amount of the immune cells, the composition may also include a pharmaceutically acceptable carrier. The term "pharmaceutically acceptable" means that when the molecular entities and compositions are properly administered to animals or humans, they will not produce adverse, allergic or other adverse reactions.

Specific examples of some substances that can be used as pharmaceutically acceptable carriers or components thereof are sugars, such as lactose, glucose, and sucrose; starches, such as corn starch and potato starch; cellulose and its derivatives, such as carboxymethyl fiber Sodium, ethyl cellulose and methyl cellulose; tragacanth powder; malt; gelatin; talc; solid lubricants, such as stearic acid and magnesium stearate; calcium sulfate; vegetable oils, such as peanut oil, cottonseed oil, Sesame oil, olive oil, corn oil, and cocoa butter; polyols, such as propylene glycol, glycerin, sorbitol, mannitol, and polyethylene glycol; alginic acid; emulsifiers, such as Tween^{®}; wetting agents, such as sodium lauryl sulfate; coloring agent; flavoring agent; tablet pressing agent, stabilizer; antioxidant; preservative; pyrogen-free water; isotonic salt solution and phosphate buffer, etc.

The composition of the present invention can be made into various dosage forms according to needs, and the doctor can determine the beneficial dosage for the patient according to factors such as the patient's type, age, weight, general disease condition, and administration method. The mode of administration can be injection or other treatment methods.

### Advantages of the present invention:

Compositions for using T cell receptor (TCR) fusion proteins to treat diseases, such as cancer and methods using the sam are provided herein. As used herein, "T cell receptor (TCR) fusion protein" or "TFP" includes recombinant polypeptides derived from various polypeptides containing TCRs, which are generally capable of i) binding to a surface antigen on a target cell, and ii) interacting with other polypeptide components of the intact TCR complex, usually when co-located in or on the surface of the T cell. As provided herein, compared with a chimeric antigen receptor, TFP can not only inhibit the growth of tumor cells, but also release fewer cytokines, thereby effectively reducing the possibility of cytokine storms.

The present invention will be further explained below in conjunction with specific embodiments. It should be understood that these embodiments are only used to illustrate the present invention and not to limit the scope of the present invention. The experimental methods without specific conditions in the following examples usually follow the conventional conditions as described in J. Sambrook et al., Molecular Cloning Experiment Guide, Third Edition, Science Press, 2002, or according to the conditions described in the manufacturer The suggested conditions. All publications, patents, and patent applications mentioned in this specification are incorporated herein by reference to the extent that it is specifically and individually indicated that each individual publication, patent or patent application is incorporated by reference.

Exemplary antigen receptors of the present invention, including CAR, and methods for engineering and introducing receptors into cells, can refer to those disclosed in, for example, CN107058354A, CN107460201A, CN105194661A, CN105315375A, CN105713881A, CN106146666A, CN106519037A, CN106554414A , CN105331585A, CN106397593A, CN106467573A, CN104140974A, WO2017186121A1, WO2018006882A1, WO2015172339A8, and WO2018/018958A1.

### Example 1. Construction of T cells expressing TCR fusion protein

Using conventional molecular biology methods in the art, the scFv used in this example was an antibody targeting claudin 18.2, the amino acid sequence of which is shown in SEQ ID NO:1.

### 1. Construction of Plasmid

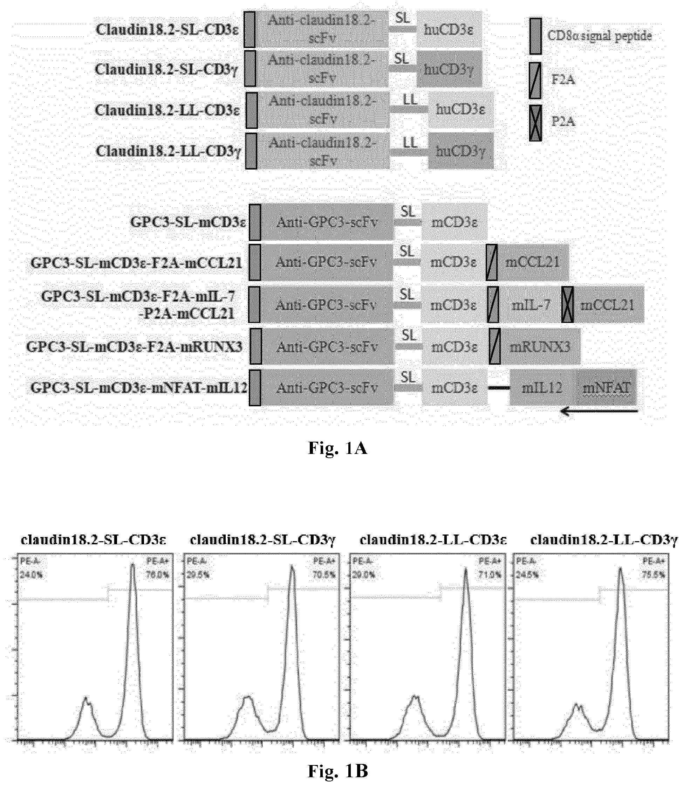

PRRLSIN-cPPT.EF-1α (purchased from addgene) was used as a vector, 4 different anti-Claudin18.2 lentiviral plasmids were prepared by inserting anti-Caludin18.2 single-chain antibody with CD3ε or CD3γ linked by different linkers in lengths (Figure 1A).

pRRLSIN-cPPT.EF-1α-claudin18.2-LL-CD3ε, sequentially connected anti-cluadin18.2 single-chain antibody (SEQ ID NO: 1), long linker (SEQ ID NO: 2), CD3ε (SEQ ID NO : 3). Gene sequences of the three fragments, single-chain antibody claudin18.2 (SEQ ID NO: 4), long linker (SEQ ID NO: 5) and CD3ε (SEQ ID NO: 6) were joined together by bridge PCR, and double-digested by restriction enzymes MluI&SalI, thereby forming the fragment claudin18.2-LL-CD3ε (SEQ ID NO: 7). The vector was double-digested with restriction endonucleases MluI&SalI to obtain the linearized vector pRRL-MluI&SalI, and the homologous recombinase was used to circularize the vector and fragments to form the plasmid pRRL-claudin 18.2-LL-CD3ε.

pRRLSIN-cPPT.EF-1α-claudin18.2-LL-CD3γ, sequentially connected anti-cluadin18.2 single-chain antibody cluadin18.2 (SEQ ID NO: 1), long linker (SEQ ID NO: 2), CD3γ (SEQ ID NO: 8). Gene sequences of the three fragments, single-chain antibody (SEQ ID NO: 4), long linker (SEQ ID NO: 5) and CD3γ (SEQ ID NO: 9) were joined together by bridging PCR, and double-digested by restriction enzymes MluI&SalI, thereby forming the fragment claudin18.2-LL-CD3γ (SEQ ID NO: 10). The vector was double-digested with restriction endonucleases MluI&SalI to obtain the linearized vector pRRL-MluI&SalI, and the homologous recombinase was used to circularize the vector and fragments to form the plasmid pRRL-claudin18.2-LL-CD3γ.

pRRLSIN-cPPT.EF-1α-claudin18.2-SL-CD3ε, sequentially connected anti-cluadin18.2 single-chain antibody cluadin18.2 (SEQ ID NO: 1), short linker (SEQ ID NO: 11), CD3ε (SEQ ID NO: 3). Gene sequences of the three fragments, single-chain antibody (SEQ ID NO: 4), short linker (SEQ ID NO: 12) and CD3ε (SEQ ID NO: 6) were joined together by bridging PCR, and double-digested by restriction enzymes MluI&SalI, thereby forming the fragment claudin18.2-SL-CD3ε (SEQ ID NO: 13). The vector was double-digested with restriction endonucleases MluI&SalI to obtain the linearized vector pRRL-MluI&SalI, and the homologous recombinase was used to circularize the vector and fragments to form the plasmid pRRL-claudin18.2-SL-CD3ε.

pRRLSIN-cPPT.EF-1α-claudin18.2-SL-CD3γ, sequentially connected anti-cluadin18.2 single-chain antibody cluadin18.2 (SEQ ID NO: 1), short linker (SEQ ID NO: 11), CD3γ (SEQ ID NO: 8). Gene sequences of the three fragments, single-chain antibody (SEQ ID NO: 4), short linker (SEQ ID NO: 12) and CD3γ (SEQ ID NO: 9) were joined together by bridging PCR, and double-digested by restriction enzymes MluI&SalI, thereby forming the fragment claudin18.2-SL-CD3γ (SEQ ID NO: 14). The vector was double-digested with restriction endonucleases MluI&SalI to obtain the linearized vector pRRL-MluI&SalI, and the homologous recombinase was used to circularize the vector and fragments to form the plasmid pRRL-claudin18.2-SL-CD3γ.

### 2. Preparation of T cells expressing TCR fusion protein

1) 293T cells were inoculated in a culture dish. Using conventional techniques in the field, plasmids pRRL-claudin18.2-LL-CD3ε, pRRL-claudin18.2-LL-CD3γ, pRRL-claudin18.2-SL-CD3ε, pRRL-claudin18.2-SL-CD3γ were transfected into 293T cells, respectively. After transfection for 72 hrs, the virus supernatant was collected to obtain lentivirus claudin18.2-LL-CD3ε, claudin18.2-LL-CD3γ, claudin18.2-SL-CD3ε, and claudin18.2-SL-CD3γ, respectively.
2) Peripheral blood mononuclear cells (PBMC) were separated from the blood of healthy donors using Ficoll (from GE) density gradient centrifugation method according to standard procedures. AIM-V medium (containing 2% human AB serum) was added at a density of about 1×10⁶/mL and CD3/CD28 activated magnetic beads (Invitrogen) at a cell: magnetic bead ratio of 1: 1 and recombinant human IL-2 at a final concentration of 300U/ mL were added for stimulation for 48 hrs. Then T cells were infected with the recombinant lentivirus constructed above at MOI = 10 to obtain T cells expressing TCR fusion protein: claudin18.2-SL-CD3ε cells, claudin18.2-SL-CD3γ cells, claudin18.2-LL-CD3ε cells, claudin18.2-LL-CD3γ cells.

The results of flow cytometry are shown in Figure 1B, and all of the positive rates are over 70%.

The detection method for positive rate is: primary antibody: claudin18.2 antibody-Biotin-F(ab)2 (CARSGEN THERAPEUTICS CO., LTD.) at a concentration of 20 ug/ml, incubated on ice for 45 min, secondary antibody: Streptavidin PE(eBioscience) (1:200), incubated on ice for 45 min. Note: The primary antibody and secondary antibody were washed with PBS+1%FBS for 1 time.

### Example 2. In vitro killing toxicity test and in vitro cytokine secretion test

CytoTox 96 non-radioactive cytotoxicity detection kit (Promega) was used. The specific method refers to the instructions of CytoTox 96 non-radioactive cytotoxicity detection kit.

The number of target cells was (BxPC-3:15000/well; HGC-27:10000/well), , and co-cultured with effector cells at an effector target ratio of 3: 1, 1: 1 or 1: 3 for 18 hrs and detected (1640+5% FBS, 200ul system), the effector cells were Untransduced (UTD) T cells, claudin18.2-28Z (the construction of which may refer to CN105315375A), claudin18.2-BBZ (the construction of which may refer to CN105315375A), claudin18.2-SL-CD3ε cells, claudin18.2-SL-CD3γ cells, claudin18.2-LL-CD3ε cells, claudin18.2-LL-CD3γ cells. The human claudin 18.2 fragment (SEQ ID NO: 54) was transferred into pancreatic cancer cell Bxpc-3 (purchased from ATCC) and gastric cancer cell HGC-27 (purchased from ATCC) to construct Bxpc-3-claudin 18.2 and HGC-27-claudin 18.2 cells expressing human claudin 18.2.

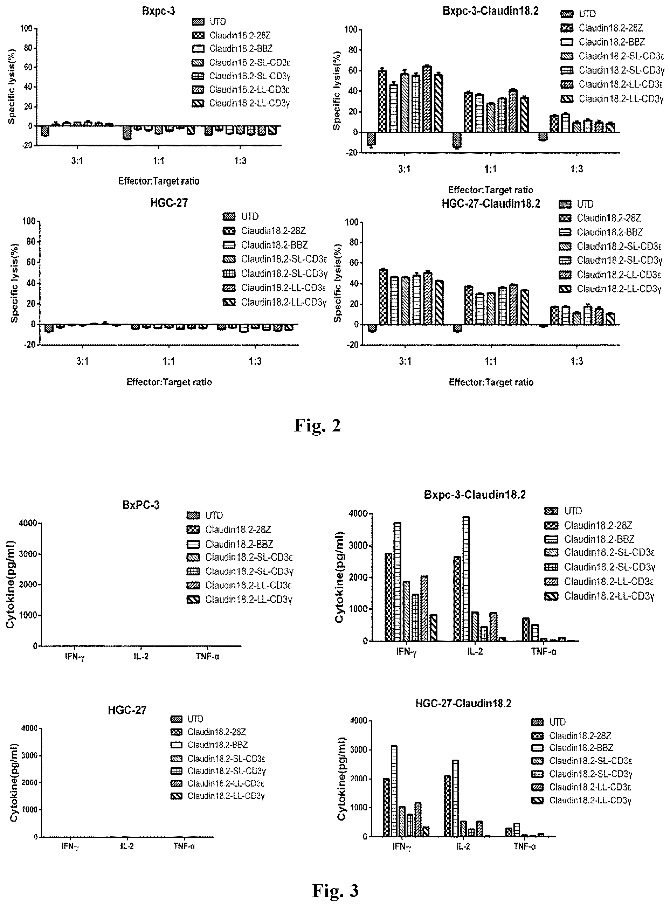

The experimental results are shown in Figure 2. For Bxpc-3-claudin18.2 and HGC-27-claudin18.2 cells positive for the target antigen, all of claudin18.2-28Z, claudin18.2-BBZ and claudin18.2-SL-CD3ε cells , Claudin18.2-SL-CD3γ cells, claudin18.2-LL-CD3ε cells, claudin18.2-LL-CD3γ cells showed very significant specific cytotoxicities, and showed a gradient-dependent effect target ratio; that is, the higher the ratio, the stronger the cytotoxicity. While there is no specific cytotoxicity for Bxpc-3 and HGC-27 cells that do not express Claudin 18.2. Among them, when the effector target ratio is 3: 1, the cytotoxicities of claudin18.2-28Z, claudin18.2-BBZ, claudin18.2-SL-CD3ε cells, claudin18.2-SL-CD3γ cells, claudin18.2-LL-CD3ε cells, claudin18 2-LL-CD3γ cells to Bxpc-3-claudin 18.2 were 59.66%, 45.77%, 59.51%, 57.34%, 63.91%, and 58.10%, respectively, and the cytotoxicities to HGC-27-claudin 18.2 were 46.18%, 47.93%, 50.56%, 42.71%, respectively. Compared with the second-generation of CAR T cells claudin18.2-28Z and claudin18.2-BBZ, the T cells expressing the TCR fusion protein have almost no difference in toxicities and killing effects on target cells.

CBA kit (BD Biosciences) was used to detect the secretion of cytokines from T cells expressing TCR fusion protein after co-incubated with pancreatic cancer BxPC-3, BxPC-3-Claudin 18.2, and gastric cancer HGC-27, HGC-27-Claudin 18.2 for 24 hours at an effector target ratio of 1: 1. The cytokine expression in the cell culture supernatant was detected. The results are shown in Figure 3.

When co-incubated with target cells BxPC-3-Claudin18.2 and HGC-27-Claudin18.2 overexpressing Claudin 18.2, T cells expressing the TCR fusion protein can secrete large amounts of IFN-γ, IL-2 and TNF- α, in which, claudin18.2-SL-CD3γ cells and claudin18.2-LL-CD3γ secrete the three cytokines in lower amounts than CD3ε claudin18.2-SL-CD3ε cells and claudin18.2-LL-CD3ε cells (See Table 1). However, compared with the second-generation of CAR T cells claudin18.2-28Z and claudin18.2-BBZ, the amount of cytokine secreted by T cells expressing the TCR fusion protein was significantly reduced. When co-incubated with BxPC-3 and HGC-27 cells not expressing Claudin 18.2, the secretion of the above-mentioned cytokines was almost undetectable.

**Table 1. Cytokine secretion of T cells expressing TCR fusion protein after co-incubation with target cells**

| BxPC-3-Claudin18.2 | | | | | | |
|---|---|---|---|---|---|---|
| | Claudin 1 8.2-28Z | Claudin 18. 2-BBZ | Claudin 18.2-SL-CD3ε | Claudin 18. 2-SL-CD3 γ | Claudin 18. 2-LL-CD3 ε | Claudin 18. 2-LL-CD3 γ |
| IFN-γ | 2751.61 | 3711.9 | 1873.35 | 1458.71 | 2030.58 | 812.55 |
| IL-2 | 2644.31 | 3895.33 | 900.06 | 440.31 | 885.75 | 117.06 |
| TNF-α | 714.4 | 505.06 | 83.11 | 34.18 | 118.48 | 13.22 |

| HGC27-Claudin18. | | | | | | |
|---|---|---|---|---|---|---|
| | Claudin 1 8.2-28Z | Claudin 18. 2-BBZ | Claudin 18.2-SL-CD3ε | Claudin 18. 2-SL-CD3 γ | Claudin 18. 2-LL-CD3 ε | Claudin 18. 2-LL-CD3 γ |
| IFN-γ | 2007.27 | 3136.15 | 1033.04 | 761.53 | 1179.5 | 348.8 |
| IL-2 | 2113.24 | 2644.31 | 540.75 | 278.89 | 532.25 | 27.72 |
| TNF-α | 300.05 | 466.75 | 62.91 | 45.11 | 107.4 | 13.85 |

### Example 3. Anti-tumor treatment experiment of subcutaneous xenograft tumor

NPG mouse of subcutaneous xenograft tumor with HGC27-Claudin18.2 gastric cancer cell 3×10⁶ of gastric cancer cells HGC27-Claudin18.2 were subcutaneously inoculated into the right axillary of female NPG mice (Beijing Weitongda Biotechnology Co., Ltd.), and the inoculation day was recorded as D0.

On D17 after subcutaneous inoculation of tumor tissue, the average tumor volume was about 270 mm³. The mice with xenograft tumor were divided into 5 groups: UTD group, claudin18.2-SL-CD3ε cell group, claudin18.2-SL-CD3γ cell group, claudin18.2-LL-CD3ε cell group and claudin18.2-LL-CD3γ cell group, and injected with the corresponding T cells expressing the TCR fusion protein respectively. The injection dosage was 5×10⁵ cells/animal. The UTD group was injected with 5×10⁵ cells/animal as untransduced T cell control.

After administration of T cells expressing TCR fusion protein, the volume of HGC27-Claudin18.2 xenograft tumor was measured every 3-4 days, and the changes in tumor volume of each group of mice were recorded. The tumor volume calculation formula is: (length × width²)/2. The results show that,

The mice were euthanized on D21 after the T cell expressing the TCR fusion protein were injected. Compared with the UTD group, the tumor inhibition rate of claudin18.2-SL-CD3ε cells was 40.42%, and the inhibition rates of claudin18.2-SL-CD3 γ, claudin18.2-LL-CD3ε cells and claudin18.2-LL-CD3 γ cells were lower than that of claudin18.2-SL-CD3ε cells. The changes in the body weight of the mice were recorded, and it was found that there was no significant difference in the body weight of the mice in each group. While in the liver cancer model of C57BL/6 mice with normal immune, compared with UTD group, the tumor inhibition rates of claudin18.2-SL-CD3 γ, claudin18.2-LL-CD3ε, claudin18.2-LL-CD3γ, and claudin18.2-SL-CD3ε cells were about 25-45%.

### Example 4. Construction of T cells expressing TCR fusion protein and targeting GPC3

Using conventional molecular biology methods in the art, the scFv used in this example is an antibody targeting GPC3, the amino acid sequence of which is shown in SEQ ID NO: 23, and the nucleotide sequence of which is shown in SEQ ID NO: 24.

### 1. Construction of Plasmid

pMSCV (purchased from addgene) was used as a vector, and an anti-GPC3 retroviral plasmid was formed by inserting an anti-GPC3 single-chain antibody GPC3 and CD3ε connected by a short linker.

The anti-GPC3 single-chain antibody (SEQ ID NO: 23), the short linker (SEQ ID NO: 11), and mCD3ε (SEQ ID NO: 34) were sequentially connected. Gene sequences of the three fragments, single-chain antibody (SEQ ID NO: 24), short linker (SEQ ID NO: 12) and mCD3γ (SEQ ID NO: 35) were linked together by bridge PCR to form the fragment GPC3-SL-mCD3ε (SEQ ID NO: 25) (Figure 1A). The vector was double-digested with restriction enzymes EcoRI&HindIII to obtain a linearized vector pMSCV-EcoRI&HindIII, and the vector and fragments were circularized by the homologous recombinase to form a plasmid pMSCV-GPC3-SL-mCD3ε.

For GPC3-SL-mCD3ε, F2A (SEQ ID NO: 36) and mCCL21b (SEQ ID NO: 38) were sequentially connected. Gene sequences of F2A (SEQ ID NO: 37) and mCCL21b (SEQ ID NO: 39) were linked together by bridge PCR to form F2A-mCCL21b. The vector was double-digested with restriction enzymes EcoRI&HindIII to obtain a linearized vector pMSCV-EcoRI&HindIII, and the vector and fragments GPC3-SL-mCD3ε and F2A-mCCL21b were circularized by the homologous recombinase to form a plasmid pMSCV-GPC3- SL-mCD3ε-F2A-mCCL21b (Figure 1A).

For GPC3-SL-mCD3ε, F2A (SEQ ID NO: 36) and mIL7 (SEQ ID NO: 40) were sequentially connected. Gene sequences of F2A (SEQ ID NO: 37) and mIL7 (SEQ ID NO: 41) were linked together by bridge PCR to form F2A-mIL7. Gene sequences of P2A (SEQ ID NO: 43) and mCCL21b (SEQ ID NO: 39) were linked together by bridge PCR to form P2A-mCCL21b. The vector was double-digested with restriction enzymes EcoRI&HindIII to obtain a linearized vector pMSCV-EcoRI&HindIII, and the vector and fragments GPC3-SL-mCD3ε, F2A-mIL7, P2A-mCCL21b were circularized by the homologous recombinase to form a plasmid pMSCV-GPC3-SL-mCD3ε-F2A-mIL7-P2A-mCCL21b (Figure 1A).

For GPC3-SL-mCD3ε, NFAT (SEQ ID NO: 44), mIL2 Minimal Promoter (SEQ ID NO: 46), mIL12 (SEQ ID NO: 48) and PA2 (SEQ ID NO: 52) were connected, and gene sequences of the 4 fragenmts, NFAT (SEQ ID NO: 45), mIL2 Minimal Promoter (SEQ ID NO: 47), mIL12 (SEQ ID NO: 49) and PA2 (SEQ ID NO: 53) were linked together by bridge PCR to form NFAT-mIL12-PA2. The vector was double-digested with restriction enzymes EcoRI&HindIII to obtain a linearized vector pMSCV-EcoRI&HindIII. The vector and fragments GPC3-SL-mCD3ε and NFAT-mIL12-PA2 were circularized by a homologous recombinase to form a plasmid pMSCV- GPC3-SL-mCD3ε-NFAT-mIL12 (Figure 1A).

For GPC3-SL-mCD3ε, F2A (SEQ ID NO: 36) and mRunx3 (SEQ ID NO: 50) were sequentially connected. Gene sequences of the two fragment, F2A (SEQ ID NO: 37) and mRunx3 (SEQ ID NO: 51) were joined by bridge PCR to form F2A-mRunx3. The vector was double-digested with restriction enzymes EcoRI&HindIII to obtain a linearized vector pMSCV-EcoRI&HindIII, and the digested vector and the fragments GPC3-SL-mCD3ε and F2A-mRunx3 were circularized by a homologous recombinase to to form a plasmid pMSCV-GPC3- SL-mCD3ε-F2A-mRunx3 (Figure 1A).

### 2. Construction of T cells expressing TCR fusion protein

1) 293T cells were inoculated in a culture dish. Using conventional techniques in the field, plasmids pMSCV-GPC3-SL-mCD3ε; pMSCV-GPC3-SL-mCD3ε-F2A-mCCL21b; pMSCV-GPC3-SL-mCD3ε-F2A- mIL7-P2A-mCCL21b; pMSCV-GPC3-SL-mCD3ε-NFAT-mIL12; or pMSCV-GPC3-SL-mCD3ε-F2A-mRunx3 were transfected into 293T cells. After 48 hours of transfection, the virus supernatant was collected to obtain the retrovirus GPC3-SL-mCD3ε, GPC3-SL-mCD3ε-F2A-mCCL21b, GPC3-SL-mCD3ε-F2A-mIL7-P2A-mCCL21b, GPC3-SL-mCD3ε-NFAT-mIL12, GPC3 -SL-mCD3ε-F2A-mRunx3.
2) Mouse T cells were infected with the above retroviruses to obtain GPC3-SL-mCD3ε cells, GPC3-SL-mCD3ε-F2A-mCCL21b cells, GPC3-SL-mCD3ε-F2A-mIL7-P2A-mCCL21b cells, GPC3-SL -mCD3ε-NFAT-mIL12 cells, GPC3-SL-mCD3ε-F2A-mRunx3 cells.

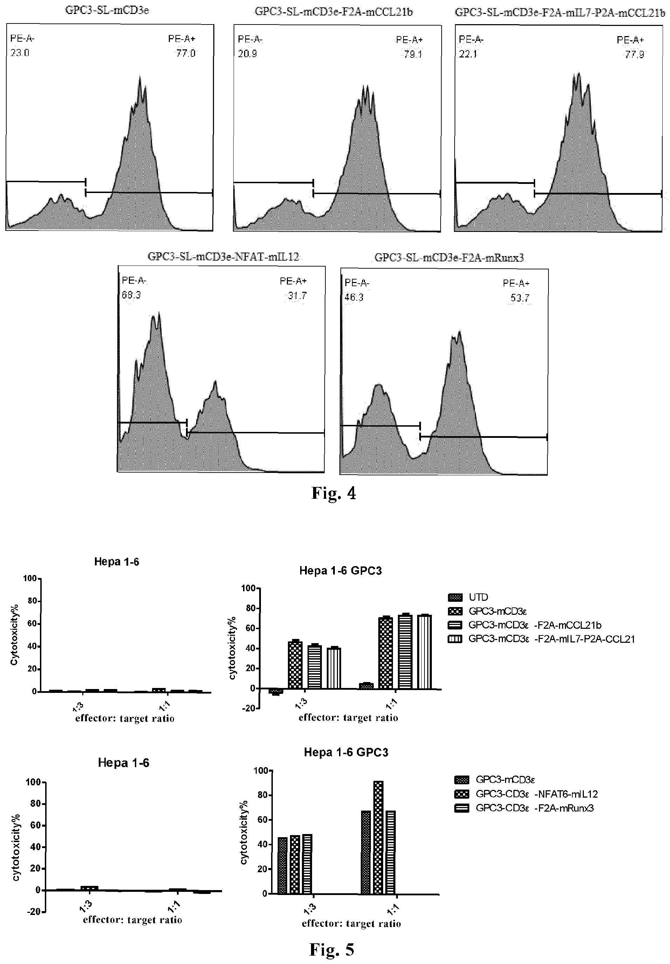

Results of the positive rate test are shown in Figure 4. The positive rates of GPC3-SL-mCD3ε, GPC3-SL-mCD3ε-F2A-mCCL21b, GPC3-SL-mCD3ε-F2A-mIL7-P2A-mCCL21b is over 70%. The positive rate of GPC3-SL-mCD3ε-NFAT-mIL12 is over 30%. The positive rate of GPC3-SL-mCD3ε-F2A-mRunx3 cells is over 50%.

The detection method for positive rate is: Primary antibody: Anti-GPC3 antibody-Biotin-F(ab)2 (CARSGEN THERAPEUTICS CO., LTD.) at a concentration of 20 ug/ml, incubated on ice for 45 mins, secondary antibody: Streptavidin PE (1: 200), incubated on ice for 45 mins.

### Example 5. In vitro killing toxicity test and in vitro cytokine secretion test

CytoTox 96 non-radioactive cytotoxicity detection kit (Promega) was used. The specific method can be found in the instructions of CytoTox 96 non-radioactive cytotoxicity detection kit. Using conventional molecular biology techniques, the mouse GPC3 fragment (SEQ ID NO: 55) was transferred into the hepatocarcinoma cell Hepa 1-6 (Cell Collection Center of the Chinese Academy of Sciences (Shanghai)) to construct Hepa 1-6 GPC3 cells expressing the mouse GPC3 protein.

The number of target cells is (Hepa 1-6:10000/well; Hepa 1-6 GPC3:10000/well), at an effector target ratio of 1: 1, 1: 3, co-cultured with effector cells for 18h for detection (1640+10% FBS , 200 ul of system), the effector cells are T cells expressing TCR fusion protein, GPC3-SL-mCD3ε cells, GPC3-SL-CD3ε-F2A-mCCL21b cells, GPC3-SL-mCD3ε-F2A-mIL7-P2A-mCCL21b cells, GPC3-SL-mCD3ε-NFAT-mIL12 cells, GPC3-SL-mCD3ε-F2A-mRunx3 cells, and Untransduced (UTD) T cells.

The experimental results are shown in Fig. 5. For Hepa 1-6 cells not expressing GPC3, the above-mentioned T cells expressing the TCR fusion protein have no cytotoxic killing effects under different effector target ratios. For Hepa 1-6 GPC3 cells expressing the target antigen GPC3, all of GPC3-SL-mCD3ε cells, GPC3-SL-CD3ε-F2A-mCCL21b cells, GPC3-SL-mCD3ε-F2A-mIL7-P2A-mCCL21b cells, GPC3-SL-mCD3ε-NFAT-mIL12 cells and GPC3-SL-mCD3ε-F2A-mRunx3 cells can achieve better killing effects, showing very significant specific cytotoxicities, and a gradient-dependency on effector target ratio, that is, the higher effector target ratio, the stronger the cytotoxicity; in which, when the effector target ratio is 1: 1, the cytotoxicities to Hepa 1-6 GPC3 were 70.7%, 73.2%, 73.4%, 91.5%, 82.2%, respectively.

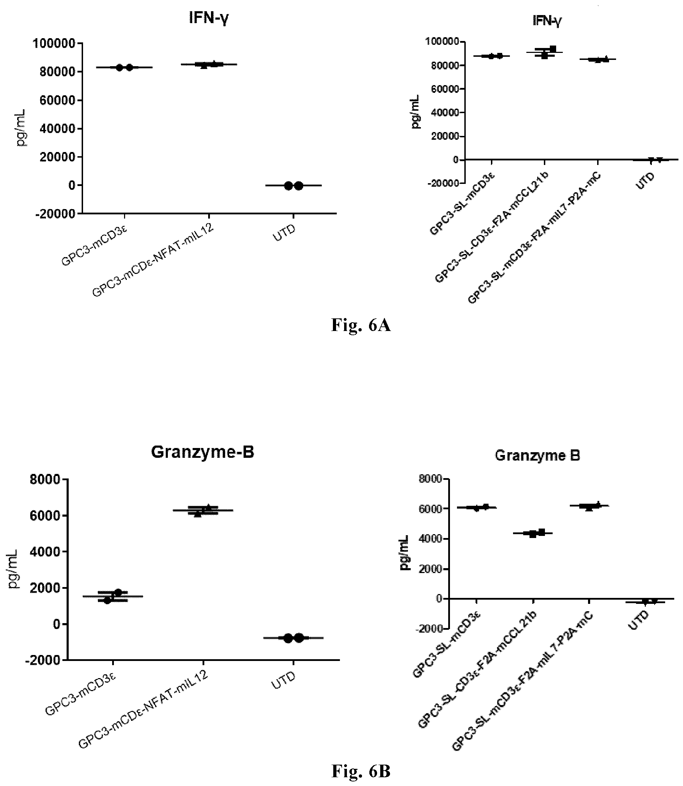

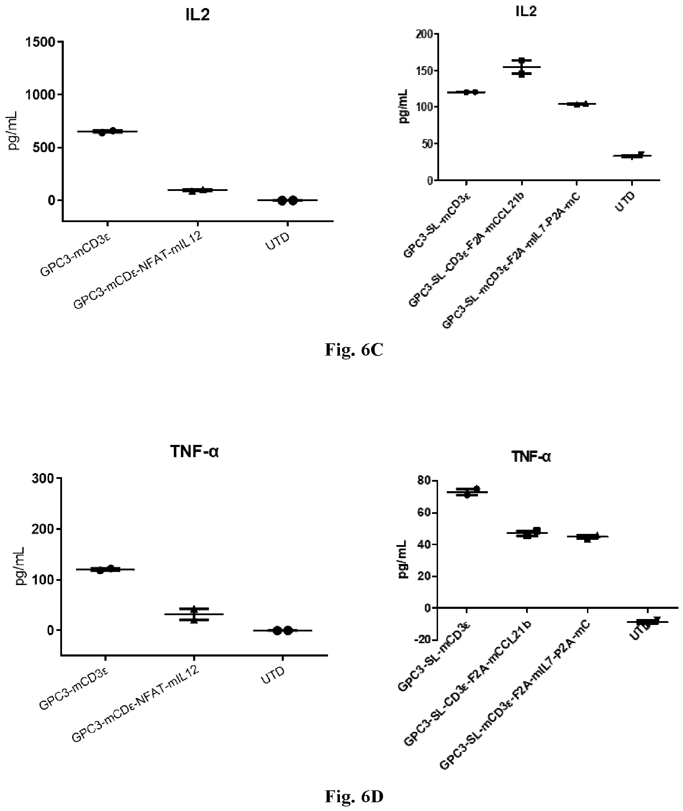

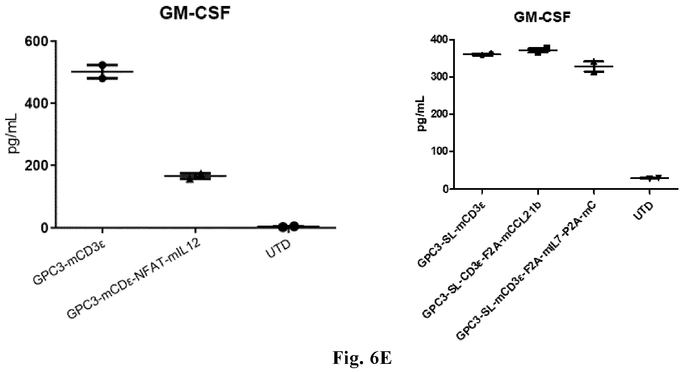

ELISA was used to detect the cytokine secretion of T cells expressing TCR fusion protein after co-incubated with hepatocarcinoma Hepa 1-6 GPC3 at an effector target ratio of 1: 1 for 24 hours. The expression of cytokine in the cell culture supernatant was detected. The secretion of IFN-γ, Granzyme-B, IL2, TNF-α and GM-CSF are shown in Figure 6A, 6B, 6C, 6D, and 6E, respectively, in which Granzyme-B represents T cell degranulation, and GM-CSF is a cytokine released after T cell activation.

When co-incubated with target cells Hepa 1-6 GPC3 overexpressing GPC3, T cells expressing TCR fusion protein secreted a large amount of IFN-γ and Granzyme-B, among which GPC3-SL-mCD3ε-NFAT-mIL12 secreted a higher amount than that in other groups.

### Example 7. Anti-tumor treatment experiment of subcutaneous xenograft tumor

### 1. subcutaneous xenograft tumor of Hepa 1-6 GPC3 liver cancer cells in C57BL/6 mice

1×10⁷ of hepatocarcinoma cells Hepa 1-6 GPC3 were subcutaneously inoculated into the right axilla of female C57BL/6 mice (Shanghai Xipuer-Bikai Experimental Animal Co., Ltd.), and the inoculation day was recorded as D0.

At D7 after subcutaneous inoculation of tumor tissue, the average tumor volume was about 355-373mm³. T cells expressing TCR fusion protein were injected into the tail vein at a dosage of 1.5×10⁶ cells/animal. In the blank control group, 1.5×10⁶ cells per animal was injected.

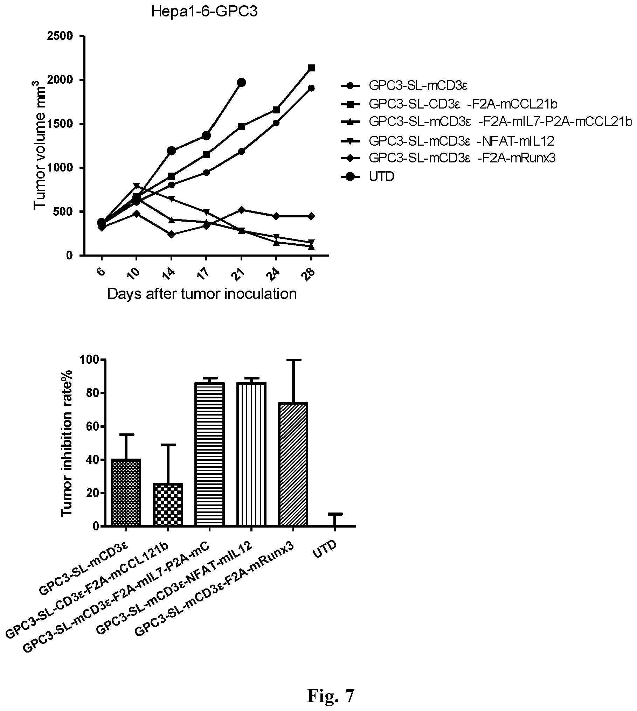

After administration of T cells expressing the TCR fusion protein, the volume of Hepa 1-6 GPC3 xenograft tumor was measured every 3-4 days, and the changes in tumor volume of each group of mice were recorded. The calculation formula for tumor volume is: (length×width²)/2. The results are shown in Figure 7. 21 days after tumor inoculation, compared with UTD group, the tumor inhibition rates of GPC3-SL-mCD3ε, GPC3-SL-CD3ε-F2A-mCCL21b, GPC3-SL-mCD3ε-F2A-mIL7-P2A-mCCL21b, GPC3 -SL-mCD3ε-NFAT-mIL12 and GPC3-SL-mCD3ε-F2A-mRunx3 treatment groups were 39.9%, 25.3%, 85.75%, 85.8% and 73.7%, respectively.

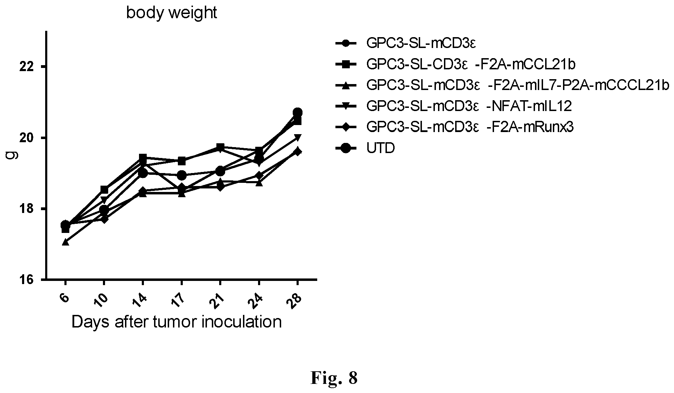

There is no significant changes in the body weight of the mice, as compared with the control group, and the results are shown in Figure 8.

The following table lists the sequences involved herein:

| SEQ ID NO: | Name | Sequence |
|---|---|---|
| 1 | claudin18.2-scFv | Qvqlqesgpglikpsqtlsltctvsggsissgynwhwirqppgkglewigyihytgstnynpalrsrvtisvdts knqfslklssvtaadtaiyycariyngnsfpywgqgttvtvssggggsggggsggggsdivMtqspdslavslge ratinckssqslfnsgnqknyltwyqqkpgqppklliywastresgvpdrfsgsgsgtdftltisslqaedvavy ycqnaysfpytfgggtkleikr |
| 2 | long linker | aaievMypppylggggsggggsggggsle |
| 3 | CD3ε | dgneeMggitqtpykvsisgttviltcpqypgseilwqhndkniggdeddknigsdedhlslkefseleqsgyyv cyprgskpedanfylylrarvcencMeMdvMsvativivdicitggllllvyywsknrkakakpvtrgagaggrqr gqnkerpppvpnpdyepirkgqrdlysglnqrri |
| 4 | claudin18.2-scFv | Caggtgcagctgcaggagagcggccccggcctgatcaagcccagccagaccctgagcctgacctgcaccgtgagc Ggcggcagcatcagcagcggctacaactggcactggatccggcagcccccggcaagggcctggagtggatcggc Tacatccactacaccggcagcaccaactacaaccccgccctgcggagccgggtgaccatcagcgtggacaccagc Aagaaccagttcagcctgaagctgagcagcgtgaccgccgccgacaccgccatctactactgcgcccggatctac Aacggcaacagcttcccctactggggccagggcaccaccgtgaccgtgagcagcggtggaggcggttcaggcgga Ggtggttctggcggtggcggatcggacatcgtgatgacccagagccccgacagcctggccgtgagcctgggcgag Cgggccaccatcaactgcaagagcagccagagcctgttcaacagcggcaaccagaagaactacctgacctggtac Cagcagaagcccggccagcccccaagctgctgatctactgggccagcacccgggagagcggcgtgcccgaccgg Ttcagcggcagcggcagcggcaccgacttcaccctgaccatcagcagcctgcaggccgaggacgtggccgtgtac tactgccagaacgcctacagcttcccctacaccttcggcggcggcaccaagctggagatcaagcgg |
| 5 | long linker | Gcggccgcaattgaagttatgtatcctcctccttacctaggtggcggcggttctggtggcggcggttctggtggc ggcggttctctcgag |
| 6 | CD3ε | Gatggtaatgaagaaatgggtggtattacacagacaccatataaagtctccatctctggaaccacagtaatattg Acatgccctcagtatcctggatctgaaatactatggcaacacaatgataaaaacataggcggtgatgaggatgat Aaaaacataggcagtgatgaggatcacctgtcactgaaggaattttcagaattggagcaaagtggttattatgtc Tgctaccccagaggaagcaaaccagaagatgcgaacttttatctctacctgagggcaagagtgtgtgagaactgc Atggagatggatgtgatgtcggtggccacaattgtcatagtggacatctgcatcactgggggcttgctgctgctg Gtttactactggagcaagaatagaaaggccaaggccaagcctgtgacacgaggagcgggtgctggcggcaggcaa Aggggacaaaacaaggagaggccaccacctgttcccaacccagactatgagcccatccggaaaggccagcgggac ctgtattctggcctgaatcagagacgcatc |
| 7 | claudin18.2-LL-CD3 ε | Caggtgcagctgcaggagagcggccccggcctgatcaagcccagccagaccctgagcctgacctgcaccgtgagc Ggcggcagcatcagcagcggctacaactggcactggatccggcagcccccggcaagggcctggagtggatcggc Tacatccactacaccggcagcaccaactacaaccccgccctgcggagccgggtgaccatcagcgtggacaccagc Aagaaccagttcagcctgaagctgagcagcgtgaccgccgccgacaccgccatctactactgcgcccggatctac Aacggcaacagcttcccctactggggccagggcaccaccgtgaccgtgagcagcggtggaggcggttcaggcgga Ggtggttctggcggtggcggatcggacatcgtgatgacccagagccccgacagcctggccgtgagcctgggcgag Cgggccaccatcaactgcaagagcagccagagcctgttcaacagcggcaaccagaagaactacctgacctggtac Cagcagaagcccggccagccccccaagctgctgatctactgggccagcacccgggagagcggcgtgcccgaccgg Ttcagcggcagcggcagcggcaccgacttcaccctgaccatcagcagcctgcaggccgaggacgtggccgtgtac Tactgccagaacgcctacagcttcccctacaccttcggcggcggcaccaagctggagatcaagcgggcggccgca Attgaagttatgtatcctcctccttacctaggtggcggcggttctggtggcggcggttctggtggcggcggttct Ctcgaggatggtaatgaagaaatgggtggtattacacagacaccatataaagtctccatctctggaaccacagta Atattgacatgccctcagtatcctggatctgaaatactatggcaacacaatgataaaaacataggcggtgatgag Gatgataaaaacataggcagtgatgaggatcacctgtcactgaaggaattttcagaattggagcaaagtggttat Tatgtctgctaccccagaggaagcaaaccagaagatgcgaacttttatctctacctgagggcaagagtgtgtgag Aactgcatggagatggatgtgatgtcggtggccacaattgtcatagtggacatctgcatcactgggggcttgctg Ctgctggtttactactggagcaagaatagaaaggccaaggccaagcctgtgacacgaggagcgggtgctggcggc Aggcaaaggggacaaaacaaggagaggccaccacctgttcccaacccagactatgagcccatccggaaaggccag cgggacctgtattctggcctgaatcagagacgcatc |
| 8 | CD3 γ | qsikgnhlvkvydyqedgsvlltcdaeaknitwfkdgkMigfltedkkkwnlgsnakdprgMyqckgsqnkskpl qvyyrMcqncielnaatisgflfaeivsifvlavgvyfiagqdgvrqsrasdkqtllpndqlyqplkdreddqys hlqgnqlrrn |
| 9 | CD3 γ | Cagtcaatcaaaggaaaccacttggttaaggtgtatgactatcaagaagatggttcggtacttctgacttgtga Tgcagaagccaaaaatatcacatggtttaaagatgggaagatgatcggcttcctaactgaagataaaaaaaaat Ggaatctgggaagtaatgccaaggacccacgagggatgtatcagtgtaaaggatcacagaacaagtcaaaacca Ctccaagtgtattacagaatgtgtcagaactgcattgaactaaatgcagccaccatatctggctttctctttgc Tgaaatcgtcagcattttcgtccttgctgttggggtctacttcattgctggacaggatggagttcgccagtcga Gagcttcagacaagcagactctgttgcccaatgaccagctctaccagcccctcaaggatcgagaagatgaccag tacagccaccttcaaggaaaccagttgaggaggaat |
| 10 | claudin18.2-LL-CD3 γ | Caggtgcagctgcaggagagcggccccggcctgatcaagcccagccagaccctgagcctgacctgcaccgt Gagcggcggcagcatcagcagcggctacaactggcactggatccggcagcccccggcaagggcctggagt Ggatcggctacatccactacaccggcagcaccaactacaaccccgccctgcggagccgggtgaccatcagc Gtggacaccagcaagaaccagttcagcctgaagctgagcagcgtgaccgccgccgacaccgccatctacta Ctgcgcccggatctacaacggcaacagcttcccctactggggccagggcaccaccgtgaccgtgagcagcg Gtggaggcggttcaggcggaggtggttctggcggtggcggatcggacatcgtgatgacccagagccccgac Agcctggccgtgagcctgggcgagcgggccaccatcaactgcaagagcagccagagcctgttcaacagcgg Caaccagaagaactacctgacctggtaccagcagaagcccggccagccccccaagctgctgatctactggg Ccagcacccgggagagcggcgtgcccgaccggttcagcggcagcggcagcggcaccgacttcaccctgacc Atcagcagcctgcaggccgaggacgtggccgtgtactactgccagaacgcctacagcttcccctacacctt Cggcggcggcaccaagctggagatcaagcgggcggccgcaattgaagttatgtatcctcctccttacctag Gtggcggcggttctggtggcggcggttctggtggcggcggttctctcgagcagtcaatcaaaggaaaccac Ttggttaaggtgtatgactatcaagaagatggttcggtacttctgacttgtgatgcagaagccaaaaatat Cacatggtttaaagatgggaagatgatcggcttcctaactgaagataaaaaaaaatggaatctgggaagta Atgccaaggacccacgagggatgtatcagtgtaaaggatcacagaacaagtcaaaaccactccaagtgtat Tacagaatgtgtcagaactgcattgaactaaatgcagccaccatatctggctttctctttgctgaaatcgt Cagcattttcgtccttgctgttggggtctacttcattgctggacaggatggagttcgccagtcgagagctt Cagacaagcagactctgttgcccaatgaccagctctaccagcccctcaaggatcgagaagatgaccagtac agccaccttcaaggaaaccagttgaggaggaat |
| 11 | short linker 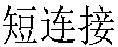 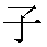 | aaaggggsggggsggggsle |
| 12 | short linker | gcggccgcaggtggcggcggttctggtggcggcggttctggtggcggcggttctctcgag |
| 13 | claudin18.2-SL-CD3 ε | Caggtgcagctgcaggagagcggccccggcctgatcaagcccagccagaccctgagcctgacctgcaccgt Gagcggcggcagcatcagcagcggctacaactggcactggatccggcagcccccggcaagggcctggagt Ggatcggctacatccactacaccggcagcaccaactacaaccccgccctgcggagccgggtgaccatcagc Gtggacaccagcaagaaccagttcagcctgaagctgagcagcgtgaccgccgccgacaccgccatctacta Ctgcgcccggatctacaacggcaacagcttcccctactggggccagggcaccaccgtgaccgtgagcagcg Gtggaggcggttcaggcggaggtggttctggcggtggcggatcggacatcgtgatgacccagagccccgac Agcctggccgtgagcctgggcgagcgggccaccatcaactgcaagagcagccagagcctgttcaacagcgg Caaccagaagaactacctgacctggtaccagcagaagcccggccagccccccaagctgctgatctactggg Ccagcacccgggagagcggcgtgcccgaccggttcagcggcagcggcagcggcaccgacttcaccctgacc Atcagcagcctgcaggccgaggacgtggccgtgtactactgccagaacgcctacagcttcccctacacctt Cggcggcggcaccaagctggagatcaagcgggcggccgcaggtggcggcggttctggtggcggcggttctg Gtggcggcggttctctcgaggatggtaatgaagaaatgggtggtattacacagacaccatataaagtctcc Atctctggaaccacagtaatattgacatgccctcagtatcctggatctgaaatactatggcaacacaatga Taaaaacataggcggtgatgaggatgataaaaacataggcagtgatgaggatcacctgtcactgaaggaat Tttcagaattggagcaaagtggttattatgtctgctaccccagaggaagcaaaccagaagatgcgaacttt Tatctctacctgagggcaagagtgtgtgagaactgcatggagatggatgtgatgtcggtggccacaattgt Catagtggacatctgcatcactgggggcttgctgctgctggtttactactggagcaagaatagaaaggcca Aggccaagcctgtgacacgaggagcgggtgctggcggcaggcaaaggggacaaaacaaggagaggccacca Cctgttcccaacccagactatgagcccatccggaaaggccagcgggacctgtattctggcctgaatcagag acgcatc |
| 14 | claudin18.2-SL-CD3 γ | Caggtgcagctgcaggagagcggccccggcctgatcaagcccagccagaccctgagcctgacctgcaccgtg Agcggcggcagcatcagcagcggctacaactggcactggatccggcagcccccggcaagggcctggagtgg Atcggctacatccactacaccggcagcaccaactacaaccccgccctgcggagccgggtgaccatcagcgtg Gacaccagcaagaaccagttcagcctgaagctgagcagcgtgaccgccgccgacaccgccatctactactgc Gcccggatctacaacggcaacagcttcccctactggggccagggcaccaccgtgaccgtgagcagcggtgga Ggcggttcaggcggaggtggttctggcggtggcggatcggacatcgtgatgacccagagccccgacagcctg Gccgtgagcctgggcgagcgggccaccatcaactgcaagagcagccagagcctgttcaacagcggcaaccag Aagaactacctgacctggtaccagcagaagcccggccagcccccaagctgctgatctactgggccagcacc Cgggagagcggcgtgcccgaccggttcagcggcagcggcagcggcaccgacttcaccctgaccatcagcagc Ctgcaggccgaggacgtggccgtgtactactgccagaacgcctacagcttcccctacaccttcggcggcggc Accaagctggagatcaagcgggcggccgcaggtggcggcggttctggtggcggcggttctggtggcggcggt Tctctcgagcagtcaatcaaaggaaaccacttggttaaggtgtatgactatcaagaagatggttcggtactt Ctgacttgtgatgcagaagccaaaaatatcacatggtttaaagatggaagatgatcggcttcctaactgaa Gataaaaaaaaatggaatctgggaagtaatgccaaggacccacgagggatgtatcagtgtaaaggatcacag Aacaagtcaaaaccactccaagtgtattacagaatgtgtcagaactgcattgaactaaatgcagccaccata Tctggctttctctttgctgaaatcgtcagcattttcgtccttgctgttggggtctacttcattgctggacag Gatggagttcgccagtcgagagcttcagacaagcagactctgttgcccaatgaccagctctaccagcccctc aaggatcgagaagatgaccagtacagccaccttcaaggaaaccagttgaggaggaat |
| 15 | claudin18.2 antibody HCDR1 | SGYNWH |
| 16 | claudin18.2 antibody HCDR2 | yihytgstnynpalrs |
| 17 | claudin18.2 antibody HCDR3 | IYNGNSFPY |
| 18 | claudin18.2 antibody LCDR1 | KSSQSLFNSGNQKNYLT |
| 19 | claudin18.2 antibody LCDR2 | WASTRES |
| 20 | claudin18.2 antibody LCDR3 | QNAYSFPYT |
| 21 | claudin18.2 antibody VH | QVQLQESGPGLIKPSQTLSLTCTVSGGSISSGYNWHWIRQPPGKGLEWIGYIHYTG STNYNPALRSRVTISV DTSKNQFSLKLSSVTAADTAIYYCARIYNGNSFPYWGQGTTVTVSS |
| 22 | claudin18.2 antibody VL | divMtqspdslavslgeratinckssqslfnsgnqknyltwyqqkpgqppklliywastresgvpdrfsgsg sgtdftltisslqaedvavyycqnaysfpytfgggtkleikr |
| 23 | amino acid sequence of GPC3 antibody | EVQLVQSGAEVKKPGASVKVSCKASGYTFSDYEMHWVRQAPGQGLEWMGAIHP GSGDTAYNQRFKGRVTITAD KSTSTAYMELSSLRSEDTAVYYCARFYSYAYWGQGTLVTVSAGGGGSGGGGSG GGGSDIVMTQTPLSLPVTPG EPASISCRSSQSLVHSNGNTYLQWYLQKPGQSPQLLIYKVSNRFSGVPDRFSGSGS GTDFTLKISRVEAEDVG VYYCSQSIYVPYTFGQGTKLEIKR |
| 24 | nucleic acid sequence of GPC3 antibody | Gaggtgcagctggtgcagagcggcgccgaggtgaagaagcccggcgccagcgtgaaggtgagctgcaaggcca Gcggctacaccttcagcgactacgagatgcactgggtgcggcaggcccccggccagggcctggagtggatggg Cgccatccaccccggcagcggcgacaccgcctacaaccagcggttcaagggccgggtgaccatcaccgccgac Aagagcaccagcaccgcctacatggagctgagcagcctgcggagcgaggacaccgccgtgtactactgcgccc Ggttctacagctacgcctactggggccagggcaccctggtgaccgtgagcgccggtggaggcggttcaggcgg Aggtggttctggcggtggcggatcggacatcgtgatgacccagacccccctgagcctgcccgtgacccccggc Gagcccgccagcatcagctgccggagcagccagagcctggtgcacagcaacggcaacacctacctgcagtggt Acctgcagaagcccggccagagcccccagctgctgatctacaaggtgagcaaccggttcagcggcgtgcccga Ccggttcagcggcagcggcagcggcaccgacttcaccctgaagatcagccgggtggaggccgaggacgtgggc gtgtactactgcagccagagcatctacgtgccctacaccttcggccagggcaccaagctggagatcaaacgt |
| 25 | nucleic acid sequence of GPC3-MCD3 ε | Gaggtgcagctggtgcagagcggcgccgaggtgaagaagcccggcgccagcgtgaaggtgagctgcaaggcca Gcggctacaccttcagcgactacgagatgcactgggtgcggcaggcccccggccagggcctggagtggatggg Cgccatccaccccggcagcggcgacaccgcctacaaccagcggttcaagggccgggtgaccatcaccgccgac Aagagcaccagcaccgcctacatggagctgagcagcctgcggagcgaggacaccgccgtgtactactgcgccc Ggttctacagctacgcctactggggccagggcaccctggtgaccgtgagcgccggtggaggcggttcaggcgg Aggtggttctggcggtggcggatcggacatcgtgatgacccagacccccctgagcctgcccgtgacccccggc Gagcccgccagcatcagctgccggagcagccagagcctggtgcacagcaacggcaacacctacctgcagtggt Acctgcagaagcccggccagagcccccagctgctgatctacaaggtgagcaaccggttcagcggcgtgcccga Ccggttcagcggcagcggcagcggcaccgacttcaccctgaagatcagccgggtggaggccgaggacgtgggc Gtgtactactgcagccagagcatctacgtgccctacaccttcggccagggcaccaagctggagatcaaacgtg Cggccgcaggtggcggcggttctggtggcggcggttctggtggcggcggttctctcgagatgcggtggaacac Tttctggggcatcctgtgcctcagcctcctagctgttggcacttgccaggacgatgccgagaacattgaatac Aaagtctccatctcaggaaccagtgtagagttgacgtgccctctagacagtgacgagaacttaaaatggaaa Aaaatggccaagagctgcctcagaagcatgataagcacctggtgctccaggatttctcggaagtcgaggacag Tggctactacgtctgctacacaccagcctcaaataaaaacacgtacttgtacctgaaagctcgagtgtgtgag Tactgtgtggaggtggacctgacagcagtagccataatcatcattgttgacatctgtatcactctgggcttgc Tgatggtcatttattactggagcaagaataggaaggccaaggccaagcctgtgacccgaggaaccggtgctgg Tagcaggcccagagggcaaaacaaggagcggccaccacctgttcccaacccagactatgagcccatccgcaaa ggccagcgggacctgtattctggcctgaatcagagagcagtc |
| 26 | GPC3 antibody HCDR1 | DYEMH |
| 27 | GPC3 antibody HCDR2 | AIHPGSGDTAYNQRFKG |
| 28 | GPC3 antibody HCDR3 | FYSYAY |
| 29 | GPC3 antibody LCDR1 | RSSQSLVHSNGNTYLQ |
| 30 | GPC3 antibody LCDR2 | KVSNRFS |
| 31 | GPC3 antibody LCDR3 | SQSIYVPYT |
| 32 | GPC3 antibody VH | EVQLVQSGAEVKKPGASVKVSCKASGYTFSDYEMHWVRQAPGQGLEWMGAIHP GSGDTAYNQRFKGRVTITA DKSTSTAYMELSSLRSEDTAVYYCARFYSYAYWGQGTLVTVSA |
| 33 | GPC3 antibody VL | DIVMTQTPLSLPVTPGEPASISCRSSQSLVHSNGNTYLQWYLQKPGQSPQLLIYKV SNRFSGVPDRFSGSGS GTDFTLKISRVEAEDVGVYYCSQSIYVPYTFGQGTKLEIKR |
| 34 | mCD3 ε | MRWNTFWGILCLSLLAVGTCQDDAENIEYKVSISGTSVELTCPLDSDENLKWEKN GQELPQKHDKHLVLQDF SEVEDSGYYVCYTPASNKNTYLYLKARVCEYCVEVDLTAVAIIIIVDICITLGLLM VIYYWSKNRKAKAKPV TRGTGAGSRPRGQNKERPPPVPNPDYEPIRKGQRDLYSGLNQRAV |
| 35 | mCD3 ε | Atgcggtggaacactttctggggcatcctgtgcctcagcctcctagctgttggcacttgccaggacgatgcc Gagaacattgaatacaaagtctccatctcaggaaccagtgtagagttgacgtgccctctagacagtgacgag Aacttaaaatgggaaaaaaatggccaagagctgcctcagaagcatgataagcacctggtgctccaggatttc Tcggaagtcgaggacagtggctactacgtctgctacacaccagcctcaaataaaaacacgtacttgtacctg Aaagctcgagtgtgtgagtactgtgtggaggtggacctgacagcagtagccataatcatcattgttgacatc Tgtatcactctgggcttgctgatggtcatttattactggagcaagaataggaaggccaaggccaagcctgtg Acccgaggaaccggtgctggtagcaggcccagagggcaaaacaaggagcggccaccacctgttcccaaccca gactatgagcccatccgcaaaggccagcgggacctgtattctggcctgaatcagagagcagtc |
| 36 | F2A | VKQTLNFDLLKLAGDVESNPGP |
| 37 | F2A | gtgaaacagactttgaattttgaccttctgaagttggcaggagacgttgagtccaaccctgggccc |
| 38 | mCCL21b | MAQMMTLSLLSLVLALCIPWTQGSDGGGQDCCLKYSQKKIPYSIVRGYRKQEPSL GCPIPAILFLPRKHSK PELCANPEEGWVQNLMRRLDQPPAPGKQSPGCRKNRGTSKSGKKGKGSKGCKRT EQTQPSRG |
| 39 | mCCL21b | Atggctcagatgatgactctgagcctccttagcctggtcctggctctctgcatcccctggacccaaggcag Tgatggagggggacaggactgctgccttaagtacagccagaagaaaattccctacagtattgtccgaggct Ataggaagcaagaaccaagtttaggctgtcccatcccggcaatcctgttcttaccccggaagcactctaag Cctgagctatgtgcaaaccctgaggaaggctgggtgcagaacctgatgcgccgcctggaccagcctccagc Cccagggaaacaaagccccggctgcaggaagaaccggggaacctctaagtctggaaagaaaggaaagggct ccaagggctgcaagagaactgaacagacacagccctcaagagga |
| 40 | MIL7 | MFHVSFRYIFGIPPLILVLLPVTSSECHIKDKEGKAYESVLMISIDELDKMTGTDSN CPNNEPNFFRKHVC DDTKEAAFLNRAARKLKQFLKMNISEEFNVHLLTVSQGTQTLVNCTSKEEKNVK EQKKNDACFLKRLLREI KTCWNKILKGSI |
| 41 | MIL7 | Atgttccatgtttcttttagatatatctttggaattcctccactgatccttgttctgctgcctgtcacat Catctgagtgccacattaaagacaaagaaggtaaagcatatgagagtgtactgatgatcagcatcgatga Attggacaaaatgacaggaactgatagtaattgcccgaataatgaaccaaactttttttagaaaacatgta Tgtgatgatacaaaggaagctgcttttctaaatcgtgctgctcgcaagttgaagcaatttcttaaaatga Atatcagtgaagaattcaatgtccacttactaacagtatcacaaggcacacaaacactggtgaactgcac Aagtaaggaagaaaaaaacgtaaaggaacagaaaaagaatgatgcatgtttcctaaagagactactgaga gaaataaaaacttgttggaataaaattttgaagggcagtata |
| 42 | P2A | ATNFSLLKQAGDVEENPGP |
| 43 | P2A | gctactaacttcagcctgctgaagcaggctggagac gtggaggagaaccctggacct |
| 44 | NFAT | KRKICFIQKALRGKFVSYRRR-EENLFHTEGVKRKICFIQKALRGKFVSYRRR-EEN LFHTEGV |
| 45 | NFAT | AAGAGGAAAATTTGTTTCATACAGAAGGCGTTAAGAGGAAAATTTGTTTCATA CAGAAGGCGTTAAGAG GAAAATTTGTTTCATACAGAAGGCGTTAAGAGGAAAATTTGTTTCATACAGAA GGCGTTAAGAGGAAAA TTTGTTTCATACAGAAGGCGTTAAGAGGAAAATTTGTTTCATACAGAAGGCGT T |
| 46 | mIL2 Minimal promoter | NIVTPPYYFSSINSINCLPC-RAAYHPC-SLLTVTSSP |
| 47 | mIL2 Minimal promoter | AACATCGTGACACCCCCATATTATTTTTCCAGCATTAACAGTATAAATTGCCTC CCATGCTGAAGAGCTGCCT ATCACCCTTGCTAATCACTCCTCACAGTGACCTCAAGTCCT |
| 48 | mIL12 | MWELEKDVYVVEVDWTPDAPGETVNLTCDTPEEDDITWTSDQRHGVIGSGKTLT ITVKEFLDAGQYTCHKGGET LSHSHLLLHKKENGIWSTEILKNFKNKTFLKCEAPNYSGRFTCSWLVQRNMDLKF NIKSSSSSPDSRAVTCGMA SLSAEKVTLDQRDYEKYSVSCQEDVTCPTAEETLPIELALEARQQNKYENYSTSFF IRDIIKPDPPKNLQMKPL KNSQVEVSWEYPDSWSTPHSYFSLKFFVRIQRKKEKMKETEEGCNQKGAFLVEK TSTEVQCKGGNVCVQAQDRY YNSSCSKWACVPCRVRSGGGGSGGGGSGGGGSCQSRYLLFLATLALLNHLSLAR VIPVSGPARCLSQSRNLLKT TDDMVKTAREKLKHYSCTAEDIDHEDITRDQTSTLKTCLPLELHKNESCLATRETS STTRGSCLPPQKTSLMMT LCLGSIYEDLKMYQTEFQAINAALQNHNHQQIILDKGMLVAIDELMQSLNHNGET LRQKPPVGEADPYRVKMKL CILLHAFSTRVVTINRVMGYLSSA |
| 49 | mIL12 | Atgtgggagctggagaaagacgtttatgttgtagaggtggactggactcccgatgcccctggagaaacagtgaacc Tcacctgtgacacgcctgaagaagatgacatcacctggacctcagaccagagacatggagtcataggctctggaaa Gaccctgaccatcactgtcaaagagtttctagatgctggccagtacacctgccacaaaggaggcgagactctgagc Cactcacatctgctgctccacaagaaggaaaatggaatttggtccactgaaattttaaaaaatttcaaaaacaaga Ctttcctgaagtgtgaagcaccaaattactccggacggttcacgtgctcatggctggtgcaaagaaacatggactt Gaagttcaacatcaagagcagtagcagttcccctgactctcgggcagtgacatgtggaatggcgtctctgtctgca Gagaaggtcacactggaccaaagggactatgagaagtattcagtgtcctgccaggaggatgtcacctgcccaactg Ccgaggagaccctgcccattgaactggcgttggaagcacggcagcagaataaatatgagaactacagcaccagctt Cttcatcagggacatcatcaaaccagacccgcccaagaacttgcagatgaagcctttgaagaactcacaggtggag Gtcagctgggagtaccctgactcctggagcactccccattcctacttctccctcaagttctttgttcgaatccagc Gcaagaaagaaaagatgaaggagacagaggaggggtgtaaccagaaaggtgcgttcctcgtagagaagacatctac Cgaagtccaatgcaaaggcgggaatgtctgcgtgcaagctcaggatcgctattacaattcctcatgcagcaagtgg Gcatgtgttccctgcagggtccgatccggtggcggtggctcgggcggtggtgggtcgggtggcggcggatcttgtc Aatcacgctacctcctctttttggccacccttgccctcctaaaccacctcagtttggccagggtcattccagtctc Tggacctgccaggtgtcttagccagtcccgaaacctgctgaagaccacagatgacatggtgaagacggccagagaa Aaactgaaacattattcctgcactgctgaagacatcgatcatgaagacatcacacgggaccaaaccagcacattga Agacctgtttaccactggaactacacaagaacgagagttgcctggctactagagagacttcttccacaacaagagg Gagctgcctgcccccacagaagacgtctttgatgatgaccctgtgccttggtagcatctatgaggacttgaagatg |
| | | Taccagacagagttccaggccatcaacgcagcacttcagaatcacaaccatcagcagatcattctagacaagggca Tgctggtggccatcgatgagctgatgcagtctctgaatcataatggcgagactctgcgccagaaacctcctgtggg Agaagcagacccttacagagtgaaaatgaagctctgcatcctgcttcacgccttcagcacccgcgtcgtgaccatc aacagggtgatgggctatctgagctccgcc |
| 50 | mRunx3 | MRIPVDPSTSRRFTPPSTAFPCGGGGGGKMGENSGALSAQATAGPGGRTRPEVRS MVDVLADHAGELVRTDSPNF LCSVLPSHWRCNKTLPVAFKVVALGDVPDGTVVTVMAGNDENYSAELRNASAV MKNQVARFNDLRFVGRSGRGKS FTLTITVFTNPTQVATYHRAIKVTVDGPREPRRHRQKIEDQTKAFPDRFGDLRMRV TPSTPSPRGSLSTTSHFSS QAQTPIQGSSDLNPFSDPRQFDRSFPTLQSLTESRFPDPRMHYPGAMSAAFPYSATP SGTSLGSLSVAGMPASSR FHHTYLPPPYPGAPQSQSGPFQANPAPYHLFYGASSGSYQFSMAAAGGGERSPTR MLTSCPSGASVSAGNLMNPS LGQADGVEADGSHSNSPTALSTPGRMDEAVWRPY |
| 51 | mRunx3 | Atgcgtattcccgtagacccgagcaccagccgccgcttcactccccctccacggccttcccctgcggcggcggc Ggcggcggcaagatgggcgagaacagcggcgcgctaagcgcgcaggcaaccgcgggccccggcggccgcacccgg Cccgaagtgcgctcgatggtggacgtgctggccgaccacgcgggagagctcgtgcgcaccgacagccccaacttc Ctctgctccgtgctgccctcgcactggcgctgcaacaagacgctgccggtcgccttcaaggtggtggccctgggg Gatgtgccggatggaacggtggtgaccgtgatggccggcaatgatgagaactactccgccgagctgcgcaacgct Tccgctgtcatgaagaaccaagtggccaggttcaacgaccttcgattcgtgggccgcagtgggcgagggaagagt Ttcacgctcacaatcaccgtgttcaccaaccctacccaagtggctacctaccaccgagccatcaaggtcactgtg Gatggaccccgggaaccccgacggcaccggcagaagatagaagaccagaccaaggccttccccgaccgctttgga Gacctgcgcatgcgtgtaacaccaagcacacccagcccccgtggctctctcagcaccacgagccacttcagcagc Caggcccagaccccaatccaaggctcctcagacctgaaccccttctccgacccccgccagtttgaccgctccttc Cctacgctgcagagcctcacagagagccgcttcccggaccccaggatgcactacccgggagccatgtctgccgcc Ttcccctacagcgccacaccatcgggcaccagcctgggcagcctgagcgtggcgggcatgccggccagcagccgc Ttccaccacacctacctccctccgccctaccccggggccccacagagccagagcgggccctttcaggccaacccc Gcgccctaccacctcttttacggcgcctcctccggctcctaccagttctccatggcagccgcgggaggtggtgag Cgctcgcccacccgcatgctgacctcctgccccagcggcgcttcggtgtcagcaggcaacctcatgaaccccagc Ctgggccaggctgatggcgtggaagccgacggcagccacagcaactcgcccacggccctgagcacgccgggccgc atggacgaggccgtgtggcggccctac |
| 52 | PA2 | NKISLFSLHLCVGFLCE |
| 53 | PA2 | aataaaatatctttattttcattacatctgtgtgttggttttttgtgtgag |
| 54 | Claudin18.2 | MAVTACQGLGFVVSLIGIAGIIAATCMDQWSTQDLYNNPVTAVFNYQGLWRSCV RESSGFTECRGYFTLLGLPAMLQAVRALMIVGIVLGAIGLLVSIFALKCIRIGSMED SAKANMTLTSGIMFIVSGLCAIAGVSVFANMLVTNFWMSTANMYTGMGGMVQT VQTRYTFGAALFVGWVAGGLTLIGGVMMCIACRGLAPEETNYKAVSYHASGHS VAYKPGGFKASTGFGSNTKNKKIYDGGARTEDEVQSYPSKHDYV |
| 55 | GPC3 | MAVTACQGLGFVVSLIGIAGIIAATCMDQWSTQDLYNNPVTAVFNYQGLWRSCV RESSGFTECRGYFTLLGLPAMLQAVRALMIVGIVLGAIGLLVSIFALKCIRIGSMED SAKANMTLTSGIMFIVSGLCAIAGVSVFANMLVTNFWMSTANMYTGMGGMVQT VQTRYTFGAALFVGWVAGGLTLIGGVMMCIACRGLAPEETNYKAVSYHASGHS VAYKPGGFKASTGFGSNTKNKKIYDGGARTEDEVQSYPSKHDYV MAGTVRTACLLVAMLLGLGCLGQAQPPPPPDATCHQVRSFFQRLQPGLKWVPET PVPGSDLQVCLPKGPTCCSRKMEEKYQLTARLNMEQLLQSASMELKFLIIQNAAV FQEAFEIVVRHAKNYTNAMFKNNYPSLTPQAFEFVGEFFTDVSLYILGSDINVDD MVNELFDSLFPVIYTQMMNPGLPESVLDINECLRGARRDLKVFGSFPKLIMTQVSK SLQVTRIFLQALNLGIEVINTTDHLKFSKDCGRMLTRMWYCSYCQGLMMVKPCG GYCNVVMQGCMAGVVEIDKYWREYILSLEELVNGMYRIYDMENVLLGLFSTIHD SIQYVQKNGGKLTTTIGKLCAHSQQRQYRSAYYPEDLFIDKKILKVAHVEHEETLS SRRRELIQKLKSFINFYSALPGYICSHSPVAENDTLCWNGQELVERYSQKAARNG MKNQFNLHELKMKGPEPVVSQIIDKLKHINQLLRTMSVPKGKVLDKSLDEEGLES GDCGDDEDECIGSSGDGMVKVKNQLRFLAELAYDLDVDDAPGNKQHGNQKDNE ITTSHSVGNMPSPLKILISVAIYVACFFFLVH |
| 56 | claudin 18.2 antibody 2 | QVQLQQSGAELARPGASVKMSCKASGYTFTSYTMHWVKQRPGQGLEWIGYINPS SGYTNYNQKFKDKATLTADKSSSTAYMQLSSLTSEDSAVYYCARIYYGNSFAYW GQGTTVTVSSGGGGSGGGGSGGGGSDIVMTQSPSSLTVTAGEKVTMSCKSSQSLL NSGNQKNYLTWYQQKPGQPPKLLIYWASTRESGVPDRFTGSGSGTDFTLTISSVQ AEDLAVYYCQNDYSYPLTFGAGTKLELKR |
| 57 | claudin 18.2 antibody 2-HCDR1 | SYTMH |
| 58 | claudin 18.2 antibody 2-HCDR2 | YINPSSGYTNYNQKFKD |
| 59 | claudin 18.2 antibody 2-HCDR3 | IYYGNSF AY |
| 60 | claudin 18.2 antibody 2-LCDR1 | KSSQSLLNSGNQKNYLT |
| 61 | claudin 18.2 antibody 2-LCDR2 | WASTRES |
| 62 | claudin 18.2 antibody 2-LCDR3 | QNDYSYPLT |
| 63 | claudin 18.2 antibody 2-VH | QVQLQQSGAELARPGASVKMSCKASGYTFTSYTMHWVKQRPGQGLEWIGYINPS SGYTNYNQKFKDKATLTADKSSSTAYMQLSSLTSEDSAVYYCARIYYGNSFAYW GQGTTVTVSS |
| 64 | claudin 18.2 antibody 2-VL | DIVMTQSPSSLTVTAGEKVTMSCKSSQSLLNSGNQKNYLTWYQQKPGQPPKLLIY WASTRESGVPDRFTGSGSGTDFTLTISSVQAEDLAVYYCQNDYSYPLTFGAGTKL ELKR |
| 65 | claudin 18.2 antibody 3 | QVQLQQSGPELVKPGALVKISCKASGYTFTSYDINWVKQRPGQGLEWIGWIYPGD GSTKYNEKFKGKATLTADKSSSTAYMQLSSLTSENSAVYFCARGGYRYDEAMDY WGQGTTVTVSSGGGGSGGGGSGGGGSQIVLTQSPAIMSASPGEKVTMTCSASSSIS YMHWYQQKPGTSPKRWIYDTSKLASGVPARFSGSGSGTSYSLTISSMEAEDAATY YCHQRSSYPYTFGGGTKLEIKR |
| 66 | claudin 18.2 antibody 3-HCDR1 | SYDIN |
| 67 | claudin 18.2 antibody 3-HCDR2 | WIYPGDGSTKYNEKFKG |
| 68 | claudin 18.2 antibody 3-HCDR3 | GGYRYDEAMDY |
| 69 | claudin 18.2 antibody 3-LCDR1 | SASSSISYMH |
| 70 | claudin 18.2 antibody 3-LCDR2 | DTSKLAS |
| 71 | claudin 18.2 antibody 3-LCDR3 | HQRSSYPYT |
| 72 | claudin 18.2 antibody 3-VH | QVQLQQSGPELVKPGALVKISCKASGYTFTSYDINWVKQRPGQGLEWIGWIYPGD GSTKYNEKFKGKATLTADKSSSTAYMQLSSLTSENSAVYFCARGGYRYDEAMDY WGQGTTVTVSS |
| 73 | claudin 18.2 antibody 3-VL | QIVLTQSPAIMSASPGEKVTMTCSASSSISYMHWYQQKPGTSPKRWIYDTSKLASG VPARFSGSGSGTSYSLTISSMEAEDAATYYCHQRSSYPYTFGGGTKLEIKR |
| 74 | claudin 18.2 antibody 4 | QIQLVQSGPELKKPGETVKISCKASGYTFTNYGMNWVKQAPGKGLKWMGWINT NTGEPTYAEEFKGRFAFSLETSASTAYLQINNLKNEDTATYFCARFSYGNSFAYW GQGTTVTVSSGGGGSGGGGSGGGGSDIVMTQSPSSLSVSAGEKVTMSCKSSQSLL NSGNQKNYLAWYQQKPGQPPKLLIYGASTRESGVPDRFTGSGSGTDFTLTISSVQ AEDLAVYYCQNDHSYPLTFGAGTKLELKR |
| 75 | claudin 18.2 antibody 4-HCDR1 | NYGMN |
| 76 | claudin 18.2 antibody 4-HCDR2 | WINTNTGEPTYAEEFKG |
| 77 | claudin 18.2 antibody 4-HCDR3 | FSYGNSFAY |
| 78 | claudin 18.2 antibody 4-LCDR1 | KSSQSLLNSGNQKNYLA |
| 79 | claudin 18.2 antibody 4-LCDR2 | GASTRES |
| 80 | claudin 18.2 antibody 4-LCDR3 | QNDHSYPLT |
| 81 | claudin 18.2 antibody 4-VH | QIQLVQSGPELKKPGETVKISCKASGYTFTNYGMNWVKQAPGKGLKWMGWINT NTGEPTYAEEFKGRFAFSLETSASTAYLQINNLKNEDTATYFCARFSYGNSFAYW GQGTTVTVSS |
| 82 | claudin 18.2 antibody 4-VL | DIVMTQSPSSLSVSAGEKVTMSCKSSQSLLNSGNQKNYLAWYQQKPGQPPKLLIY GASTRESGVPDRFTGSGSGTDFTLTISSVQAEDLAVYYCQNDHSYPLTFGAGTKLE LKR |
| 83 | claudin 18.2 antibody 5 | DVQLQESGPDLVKPSQSLSLTCTVTGYSITSGYNWHWIRQFPGNKMEWMGYIHY TGSTNYNPSLRSRISITRDTSKNQFFLQLNSVTTDDTATYYCTRIYNGNSFPYWGQ GTSVTVSSGGGGSGGGGSGGGGSDIVMTQSPSSLTVTPGEKVTMTCKSSQSLFNS GNQKNYLTWYQQRPGQPPKMLIYWASTRESGVPDRFTGSGSGTDFTLTISSVQAE DLAVFYCQNAYSFPYTFGGGTKLEIKR |
| 84 | claudin 18.2 antibody 5-HCDR1 | SGYNWH |
| 85 | claudin 18.2 antibody 5-HCDR2 | YIHYTGSTNYNPSLRS |
| 86 | claudin 18.2 antibody 5-HCDR3 | IYNGNSFPY |
| 87 | claudin 18.2 antibody 5-LCDR1 | KSSQSLFNSGNQKNYLT |
| 88 | claudin 18.2 antibody 5-LCDR2 | WASTRES |
| 89 | claudin 18.2 antibody 5-LCDR3 | QNAYSFPYT |
| 90 | claudin 18.2 antibody 5-VH | DVQLQESGPDLVKPSQSLSLTCTVTGYSITSGYNWHWIRQFPGNKMEWMGYIHY TGSTNYNPSLRSRISITRDTSKNQFFLQLNSVTTDDTATYYCTRIYNGNSFPYWGQ GTSVTVSS |
| 91 | claudin 18.2 antibody 5-VL | DIVMTQSPSSLTVTPGEKVTMTCKSSQSLFNSGNQKNYLTWYQQRPGQPPKMLIY WASTRESGVPDRFTGSGSGTDFTLTISSVQAEDLAVFYCQNAYSFPYTFGGGTKLE IKR |
| 92 | claudin 18.2 antibody 6 | QVQLQQSGAELARPGASVKMSCKASGYTFTSYTMHWVKQRPGQGLEWIGYIDPS SGYTNYNQKFKDKATLTADKSSSTAYMQLSSLTSEDSAVYYCARIYYGNSFAYW GQGTTVTVSSGGGGSGGGGSGGGGSDIVMTQSPSSLTVTAGEKVTMSCKSSQSLL NSGNQKNYLTWYQQKPGQPPKLLIYWASTRESGVPDRFTGSGSGTDFTLTISSVQ AEDLAVYYCQNDYSYPLTFGAGTKLELKR |
| 93 | claudin 18.2 antibody 6-HCDR1 | SYTMH |
| 94 | claudin 18.2 antibody 6-HCDR2 | YIDPSSGYTNYNQKFKD |
| 95 | claudin 18.2 antibody 6-HCDR3 | IYYGNSF AY |
| 96 | claudin 18.2 antibody 6-LCDR1 | KSSQSLLNSGNQKNYLT |
| 97 | claudin 18.2 antibody 6-LCDR2 | WASTRES |
| 98 | claudin 18.2 antibody 6-LCDR3 | QNDYSYPL T |
| 99 | claudin 18.2 antibody 6-VH | QVQLQQSGAELARPGASVKMSCKASGYTFTSYTMHWVKQRPGQGLEWIGYIDPS SGYTNYNQKFKDKATLTADKSSSTAYMQLSSLTSEDSAVYYCARIYYGNSFAYW GQGTTVTVSS |
| 100 | claudin 18.2 antibody 6-VL | DIVMTQSPSSLTVTAGEKVTMSCKSSQSLLNSGNQKNYLTWYQQKPGQPPKLLIY WASTRESGVPDRFTGSGSGTDFTLTISSVQAEDLAVYYCQNDYSYPLTFGAGTKL ELKR |
| 101 | claudin 18.2 antibody 7 | QVQLQQSGAELARPGASVKMSCKASGYTFTSYTMHWVKQRPGQGLEWIGYINP ASGYTNYNQKFKDKATLTADKSSSTAYMQLSSLTSEDSAVYYCARIYYGNSFAY WGQGTTVTVSSGGGGSGGGGSGGGGSDIVMTQSPSSLTVTAGEKVTMSCKSSQS LLNSGNQKNYLTWYQQKPGQPPKLLIYWASTRESGVPDRFTGSGSGTDFTLTISSV QAEDLAVYYCQNDYSYPLTFGAGTKLELKR |
| 102 | claudin 18.2 antibody 7-HCDR1 | SYTMH |
| 103 | claudin 18.2 antibody 7-HCDR2 | YINP AS GYTNYNQKFKD |
| 104 | claudin 18.2 antibody 7-HCDR3 | IYYGNSF AY |
| 105 | claudin 18.2 antibody 7-LCDR1 | KSSQSLLNSGNQKNYLT |
| 106 | claudin 18.2 antibody 7-LCDR2 | WASTRES |
| 107 | claudin 18.2 antibody 7-LCDR3 | QNDYSYPL T |
| 108 | claudin 18.2 antibody 7-VH | QVQLQQSGAELARPGASVKMSCKASGYTFTSYTMHWVKQRPGQGLEWIGYINP ASGYTNYNQKFKDKATLTADKSSSTAYMQLSSLTSEDSAVYYCARIYYGNSFAY WGQGTTVTVSS |
| 109 | claudin 18.2 antibody 7-VL | DIVMTQSPSSLTVTAGEKVTMSCKSSQSLLNSGNQKNYLTWYQQKPGQPPKLLIY WASTRESGVPDRFTGSGSGTDFTLTISSVQAEDLAVYYCQNDYSYPLTFGAGTKL ELKR |
| 110 | claudin 18.2 antibody 8 | QVQLQQSGAELARPGASVKMSCKASGYTFTSYTMHWVKQRPGQGLEWIGYINP ASGYTNYNQKFKDKATLTADKSSSTAYMQLSSLTSEDSAVYYCARIYYGNSFAY WGQGTTVTVSSGGGGSGGGGSGGGGSDIVMTQSPSSLTVTAGEKVTMSCKSSQS LLNSGNQKNYLTWYQQKPGQPPKLLIYWASTRESGVPDRFTGSGSGTDFTLTISSV |
| | | QAEDLAVYYCQNDYSYPLTFGAGTKLELKR |
| 111 | claudin 18.2 antibody 8-HCDR1 | SYTMH |
| 112 | claudin 18.2 antibody 8-HCDR2 | YINP AS GYTNYNQKFKD |
| 113 | claudin 18.2 antibody 8-HCDR3 | IYYGNSF AY |
| 114 | claudin 18.2 antibody 8-LCDR1 | KSSQSLLNSGNQKNYLT |
| 115 | claudin 18.2 antibody 8-LCDR2 | WASTRES |
| 116 | claudin 18.2 antibody 8-LCDR3 | QNDYSYPL T |
| 117 | claudin 18.2 antibody 8-VH | QVQLQQSGAELARPGASVKMSCKASGYTFTSYTMHWVKQRPGQGLEWIGYINP ASGYTNYNQKFKDKATLTADKSSSTAYMQLSSLTSEDSAVYYCARIYYGNSFAY WGQGTTVTVSS |
| 118 | claudin 18.2 antibody 8-VL | DIVMTQSPSSLTVTAGEKVTMSCKSSQSLLNSGNQKNYLTWYQQKPGQPPKLLIY WASTRESGVPDRFTGSGSGTDFTLTISSVQAEDLAVYYCQNDYSYPLTFGAGTKL ELKR |
| 119 | claudin 18.2 antibody 9 | QVQLVQSGAEVKKPGASVKVSCKASGYTFTSYTMHWVRQAPGQGLEWMGYINP ASGYTNYNQKFKDRVTMTRDTSTSTAYMELSSLRSEDTAVYYCARIYYGNSFAY WGQGTLVTVSSGGGGSGGGGSGGGGSDIVMTQSPDSLAVSLGERATINCKSSQSL LNSGNQKNYLTWYQQKPGQPPKLLIYWASTRESGVPDRFSGSGSGTDFTLTISSLQ AEDVAVYYCQNDYSYPLTFGGGTKVEIKR |
| 120 | claudin 18.2 antibody 9-HCDR1 | SYTMH |
| 121 | claudin 18.2 antibody 9-HCDR2 | YINP AS GYTNYNQKFKD |
| 122 | claudin 18.2 antibody 9-HCDR3 | IYYGNSF AY |
| 123 | claudin 18.2 antibody 9-LCDR1 | KSSQSLLNSGNQKNYLT |
| 124 | claudin 18.2 antibody 9-LCDR2 | WASTRES |
| 125 | claudin 18.2 antibody 9-LCDR3 | QNDYSYPL T |
| 126 | claudin 18.2 antibody 9-VH | QVQLVQSGAEVKKPGASVKVSCKASGYTFTSYTMHWVRQAPGQGLEWMGYINP ASGYTNYNQKFKDRVTMTRDTSTSTAYMELSSLRSEDTAVYYCARIYYGNSFAY WGQGTLVTVSS |
| 127 | claudin 18.2 antibody 9-VL | DIVMTQSPDSLAVSLGERATINCKSSQSLLNSGNQKNYLTWYQQKPGQPPKLLIY WASTRESGVPDRFSGSGSGTDFTLTISSLQAEDVAVYYCQNDYSYPLTFGGGTKV EIKR |
| 128 | claudin 18.2 antibody 10 | QVQLQESGPGLVKPSQTLSLTCTVSGGSISSGYNWHWIRQPPGKGLEWIGYIHYTG STNYNPALRSRVTISVDTSKNQFSLKLSSVTAADTAVYYCARIYNGNSFPYWGQG TTVTVSSGGGGSGGGGSGGGGSDIVMTQSPDSLAVSLGERATINCKSSQSLFNSGN QKNYLTWYQQKPGQPPKLLIYWASTRESGVPDRFSGSGSGTDFTLTISSLQAEDV AVYYCQNAYSFPYTFGGGTKLEIKR |
| 129 | claudin 18.2 antibody 10-HCDR1 | SGYNWH |
| 130 | claudin 18.2 antibody 10-HCDR2 | YIHYTGSTNYNP ALRS |
| 131 | claudin 18.2 antibody 10-HCDR3 | IYNGNSFPY |
| 132 | claudin 18.2 antibody 10-LCDR1 | KSSQSLFNSGNQKNYLT |
| 133 | claudin 18.2 antibody 10-LCDR2 | WASTRES |
| 134 | claudin 18.2 antibody 10-LCDR3 | QNAYSFPYT |
| 135 | claudin 18.2 antibody 10-VH | QVQLQESGPGLVKPSQTLSLTCTVSGGSISSGYNWHWIRQPPGKGLEWIGYIHYTG STNYNPALRSRVTISVDTSKNQFSLKLSSVTAADTAVYYCARIYNGNSFPYWGQG TTVTVSS |
| 136 | claudin 18.2 antibody 10-VL | DIVMTQSPDSLAVSLGERATINCKSSQSLFNSGNQKNYLTWYQQKPGQPPKLLIY WASTRESGVPDRFSGSGSGTDFTLTISSLQAEDVAVYYCQNAYSFPYTFGGGTKL EIKR |
| 137 | GPC3 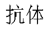 2 | QVQLQESGGGLVQPGRSLRLSCAASGFTFSSYAMHWVRQAPGKGLEWVSAISGS GGSTYYADSVKGRFTISRDNSKNTLYLQMNSLRAEDTAVYYCAKDRRGSHADAF |
| | | DVWGQGTLVTVSSGGGGSGGGGSGGGGSQSALTQPPSASGSPGQSVTISCTGTSS DVGGYNYVSWYQQYPGKAPKLLIYGNSNRPSGVPDRFSGSKSGTSASLAITGLQA EDGADYYCQSYDSSLRVVFGGGTKVTVLG |
| 138 | GPC3 antibody 2-HCDR1 | GFTFSSYAMH |
| 139 | GPC3 antibody 2-HCDR2 | AISGSGGSTYYADSVKG |
| 140 | GPC3 antibody 2-HCDR3 | DRRGSHADAFDV |
| 141 | GPC3 antibody 2-LCDR1 | TGTSSDVGGYNYVS |
| 142 | GPC3 antibody 2-LCDR2 | GNSNRPS |
| 143 | GPC3 antibody 2-LCDR3 | QSYDSSLRVV |
| 144 | GPC3 antibody 2-VH | QVQLQESGGGLVQPGRSLRLSCAASGFTFSSYAMHWVRQAPGKGLEWVSAISGS GGSTYYADSVKGRFTISRDNSKNTLYLQMNSLRAEDTAVYYCAKDRRGSHADAF DVWGQGTLVTVSS |
| 145 | GPC3 antibody 2-VL | QVQLQESGGGLVQPGRSLRLSCAASGFTFSTYAMTWVRQAPGKGLEWVSSISSSG ESTYYADSVKGRFTISRDNSKNTLYLQMNSLRAEDTAVYYCAKDRRGSHADAFD VWGQGTLVTVSSGGGGSGGGGSGGGGSQSALTQPPSASGSPGQSVTISCTGTSSD VGGYNYVSWYQQYPGKAPKLLIYGNSNRPSGVPDRFSGSKSGTSASLAITGLQAE DGADYYCQSYDSSLRVVFGGGTKVTVLG |
| 146 | GPC3 antibody 3 | QVQLQESGGGLVQPGRSLRLSCAASGFTFSTYAMTWVRQAPGKGLEWVSSISSSG ESTYYADSVKGRFTISRDNSKNTLYLQMNSLRAEDTAVYYCAKDRRGSHADAFD VWGQGTLVTVSSGGGGSGGGGSGGGGSQSALTQPPSASGSPGQSVTISCTGTSSD VGGYNYVSWYQQYPGKAPKLLIYGNSNRPSGVPDRFSGSKSGTSASLAITGLQAE DGADYYCQSYDSSLRVVFGGGTKVTVLG |
| 147 | GPC3 antibody 3-HCDR1 | GFTFSTYAMT |
| 148 | GPC3 antibody 3-HCDR2 | SISSSGESTYY ADSVKG |
| 149 | GPC3 antibody 3-HCDR3 | DRRGSHADAFDV |
| 150 | GPC3 antibody 3-LCDR1 | TGTSSDVGGYNYVS |
| 151 | GPC3 antibody 3-LCDR2 | GNSNRPS |
| 152 | GPC3 antibody 3-LCDR3 | QSYDSSLRVV |
| 153 | GPC3 antibody 3-VH | QVQLQESGGGLVQPGRSLRLSCAASGFTFSTYAMTWVRQAPGKGLEWVSSISSSG ESTYYADSVKGRFTISRDNSKNTLYLQMNSLRAEDTAVYYCAKDRRGSHADAFD VWGQGTLVTVSS |
| 154 | GPC3 antibody 3-VL | QSALTQPPSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGGTKVTVL G |
| 155 | GPC3 antibody 4 | QVQLQESGGGLVQPGRSLRLSCAASGFTFSTYAMAWVRQAPGKGLEWVSEISSS GSRTYYADSVKGRFTISRDNSKNTLYLQMNSLRAEDTAVYYCAKDRRGSHADAF DVWGQGTLVTVSSGGGGSGGGGSGGGGSQSALTQPPSASGSPGQSVTISCTGTSS DVGGYNYVSWYQQYPGKAPKLLIYGNSNRPSGVPDRFSGSKSGTSASLAITGLQA EDGADYYCQSYDSSLRVVFGGGTKVTVLG |
| 156 | GPC3 antibody 4-HCDR1 | GFTFSTYAMA |
| 157 | GPC3 antibody 4-HCDR2 | EISSSGSRTYY ADSVKG |
| 158 | GPC3 antibody 4-HCDR3 | DRRGSHADAFDV |
| 159 | GPC3 antibody 4-LCDR1 | TGTSSDVGGYNYVS |
| 160 | GPC3 antibody 4-LCDR2 | GNSNRPS |
| 161 | GPC3 antibody 4-LCDR3 | QSYDSSLRVV |
| 162 | GPC3 antibody 4-VH | QVQLQESGGGLVQPGRSLRLSCAASGFTFSTYAMAWVRQAPGKGLEWVSEISSS GSRTYYADSVKGRFTISRDNSKNTLYLQMNSLRAEDTAVYYCAKDRRGSHADAF DVWGQGTLVTVSS QSALTQPPSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGGTKVTVL G |
| 163 | GPC3 antibody 4-VL | QSALTQPPSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGGTKVTVL G |
| 164 | GPC3 antibody 5 | QVQLQESGGGLVQPGRSLRLSCAASGFTFSTYAMNWVRQAPGKGLEWVSAISMS GESTYYADSVKGRFTISRDNSKNTLYLQMNSLRAEDTAVYYCAKDRRGSHADAF DVWGQGTLVTVSSGGGGSGGGGSGGGGSQSALTQPPSASGSPGQSVTISCTGTSS DVGGYNYVSWYQQYPGKAPKLLIYGNSNRPSGVPDRFSGSKSGTSASLAITGLQA EDGADYYCQSYDSSLRVVFGGGTKVTVLG |
| 165 | GPC3 antibody 5-HCDR1 | GFTFSTYAMA |
| 166 | GPC3 antibody 5-HCDR2 | AISMSGESTYYADSVKG |
| 167 | GPC3 antibody 5-HCDR3 | DRRGSHADAFDV |
| 168 | GPC3 antibody 5-LCDR1 | TGTSSDVGGYNYVS |
| 169 | GPC3 antibody 5-LCDR2 | GNSNRPS |
| 170 | GPC3 antibody 5-LCDR3 | QSYDSSLRVV |
| 171 | GPC3 antibody 5-VH | QVQLQESGGGLVQPGRSLRLSCAASGFTFSTYAMNWVRQAPGKGLEWVSAISMS GESTYYADSVKGRFTISRDNSKNTLYLQMNSLRAEDTAVYYCAKDRRGSHADAF DVWGQGTLVTVSS |
| 172 | GPC3 antibody 5-VL | QSALTQPPSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGGTKVTVL G [?] |
| 173 | GPC3 antibody 6 | QVQLQESGGGLVQPGRSLRLSCAASGFTFSSYAMHWVRQAPGKGLEWVSAISSS GGSTYYADSVKGRFTISRDNSKNTLYLQMNSLRAEDTAVYYCAKDRRGSHADAF DVWGQGTLVTVSSGGGGSGGGGSGGGGSQSALTQPPSASGSPGQSVTISCTGTSS DVGHKFPVSWYQQYPGKAPKLLIYKNLLRPSGVPDRFSGSKSGTSASLAITGLQA EDGADYYCQSYDSSLRVVFGGGTKVTVLG |
| 174 | GPC3 antibody 6-HCDR1 | GFTFSSYAMH |
| 175 | GPC3 antibody 6-HCDR2 | AISSSGGSTYY ADSVKG |
| 176 | GPC3 antibody 6-HCDR3 | DRRGSHADAFDV |
| 177 | GPC3 antibody 6-LCDR1 | TGTSSDVGHKFPVS |
| 178 | GPC3 antibody 6-LCDR2 | KNLLRPS |
| 179 | GPC3 antibody 6-LCDR3 | QSYDSSLRVV |
| 180 | GPC3 antibody 6-VH | QVQLQESGGGLVQPGRSLRLSCAASGFTFSSYAMHWVRQAPGKGLEWVSAISSS GGSTYYADSVKGRFTISRDNSKNTLYLQMNSLRAEDTAVYYCAKDRRGSHADAF DVWGQGTLVTVSS |
| 181 | GPC3 antibody 6-VL | QSALTQPPSASGSPGQSVTISCTGTSSDVGHKFPVSWYQQYPGKAPKLLIYKNLLR PSGVPDRFSGSKSGTSASLAITGLOAEDGADYYCOSYDSSLRVVFGGGTKVTVLG |
| 182 | GPC3 antibody 7 | QVQLQESGGGLVQPGRSLRLSCAASGFTFSSYAMHWVRQAPGKGLEWVSAISSS GGSTYYADSVKGRFTISRDNSKNTLYLQMNSLRAEDTAVYYCAKDRRGSHADAF DVWGQGTLVTVSSGGGGSGGGGSGGGGSQSALTQPPSASGSPGQSVTISCTGTSS DVGLMHNVSWYQQYPGKAPKLLIYKSSSRPSGVPDRFSGSKSGTSASLAITGLQA EDGADYYCQSYDSSLRVVFGGGTKVTVLG |
| 183 | GPC3 antibody 7-HCDR1 | GFTFSSYAMH |
| 184 | GPC3 antibody 7-HCDR2 | AISSSGGSTYY ADSVKG |
| 185 | GPC3 antibody 7-HCDR3 | DRRGSHADAFDV |
| 186 | GPC3 antibody 7-LCDR1 | TGTSSDVGLMHNVS |
| 187 | GPC3 antibody 7-LCDR2 | KSSSRPS |
| 188 | GPC3 antibody 7-LCDR3 | QSYDSSLRVV |
| 189 | GPC3 antibody 7-VH | QVQLQESGGGLVQPGRSLRLSCAASGFTFSSYAMHWVRQAPGKGLEWVSAISSS GGSTYYADSVKGRFTISRDNSKNTLYLQMNSLRAEDTAVYYCAKDRRGSHADAF DVWGQGTLVTVSS QSALTQPPSASGSPGQSVTISCTGTSSDVGLMHNVSWYQQYPGKAPKLLIYKSSSR PSGVPDRFSGSKSGTSASLAITGLQAEDGADYYCQSYDSSLRVVFGGGTKVTVLG |
| 190 | GPC3 antibody 7-VL | QSALTQPPSASGSPGQSVTISCTGTSSDVGLMHNVSWYQQYPGKAPKLLIYKSSSR PSGVPDRFSGSKSGTSASLAITGLQAEDGADYYCQSYDSSLRVVFGGGTKVTVLG |
| 191 | GPC3 antibody 8 | QVQLQESGGGLVQPGRSLRLSCAASGFTFSSYAMHWVRQAPGKGLEWVSAISSS GRSTYYADSVEGRFTISRDNSKNTLYLQMNSLRAEDTAVYYCAKDRRGSHADAL NVWGQGTLVTVSSGGGGSGGGGSGGGGSQSALTQPPSASGSPGQSVTISCTGTSS DVGGYNYVSWYQQYPGKAPKLLIYGNSNRPSGVPDRFSGSKSGTSASLAITGLQA EDGADYYCQSYDSSLRVVFGGGTKVTVLG |
| 192 | GPC3 antibody 8-HCDR1 | GFTFSSYAMH |
| 193 | GPC3 antibody 8-HCDR2 | AISSSGRSTYY ADSVEG |
| 194 | GPC3 antibody 8-HCDR3 | DRRGSHADALNV |
| 195 | GPC3 antibody 8-LCDR1 | TGTSSDVGGYNYVS |
| 196 | GPC3 antibody 8-LCDR2 | KSSSRPS |
| 197 | GPC3 antibody 8-LCDR3 | QSYDSSLRVV |
| 198 | GPC3 antibody 8-VH | QVQLQESGGGLVQPGRSLRLSCAASGFTFSSYAMHWVRQAPGKGLEWVSAISSS GRSTYYADSVEGRFTISRDNSKNTLYLQMNSLRAEDTAVYYCAKDRRGSHADAL NVWGQGTLVTVSS |
| 199 | GPC3 antibody 8-VL | QSALTQPPSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGGTKVTVL G |
| 200 | GPC3 antibody 9 | QVQLQQSGTELVRPGASVKLSCKALGYTFTDYEMHWVKQTPVHGLEWIGAIHPG SGDTAYNQRFKGKATLTADKSSSTAYMEYSSLTSEDSAVYYCTRFYSYAYWGQG TLVTVSAGGGGSGGGGSGGGGSDVVMTQTPLSLPVSLGDQASISCRSSQSLVHSN GNTYLQWYLQKPGQSPKLLIYKVSNRFSGVPDRFSGRGSGTDFTLKISRVEAEDL GVYFCSQSIYVPYTFGGGTKLEIKR |
| 201 | GPC3 antibody 9-HCDR1 | DYEMH |
| 202 | GPC3 antibody 9-HCDR2 | AIHPGSGDTAYNQRFKG |
| 203 | GPC3 antibody 9-HCDR3 | FYSYAY |
| 204 | GPC3 antibody 9-LCDR1 | RSSQSLVHSNGNTYLQ |
| 205 | GPC3 antibody 9-LCDR2 | KVSNRFS |
| 206 | GPC3 antibody 9-LCDR3 | SQSIYVPYTF |
| 207 | GPC3 antibody 9-VH | QVQLQQSGTELVRPGASVKLSCKALGYTFTDYEMHWVKQTPVHGLEWIGAIHPG SGDTAYNQRFKGKATLTADKSSSTAYMEYSSLTSEDSAVYYCTRFYSYAYWGQG TLVTVSA |
| 208 | GPC3 antibody 9-VL | DVVMTQTPLSLPVSLGDQASISCRSSQSLVHSNGNTYLQWYLQKPGQSPKLLIYK VSNRFSGVPDRFSGRGSGTDFTLKISRVEAEDLGVYFCSQSIYVPYTFGGGTKLEIK R |

All documents mentioned in the present invention are cited as references in this application, as if each document was individually cited as a reference. In addition, it should be understood that after reading the above teaching content of the present invention, those skilled in the art can make various changes or modifications to the present invention, and these equivalent forms also fall within the scope defined by the appended claims of the present application.

## Claims

1. A T cell receptor (TCR) fusion protein (TFP), the fusion protein comprising:
(a) a TCR subunit (or a TCR unit); and
(b) an antigen recognition unit that recognizes the antigen;
the antigen is GPC3 or claudin 18.2;
wherein the TCR subunit and the antigen recognition unit are operably connected.

2. The fusion protein of claim 1, wherein the TCR subunit comprises:
(i) at least a part of the extracellular domain of TCR, and
(ii) a TCR intracellular domain comprising a stimulatory domain derived from an intracellular signaling domain of CD3ε, CD3γ, CD3δ, TCRα, or TCRβ.

3. The fusion protein of claim 1, wherein the light chain LCDR1, LCDR2, and LCDR3 of the amino acid sequence of the antigen recognition unit that recognizes GPC3 are independently selected from or have 70-100% sequence identity with the light chain LCDR1, LCDR2, and LCDR3 shown in the following table, and/or the heavy chain HCDR1, HCDR2 and HCDR3 of the amino acid sequence of the antigen recognition unit that recognizes GPC3 are independently selected from or have 70-100% sequence identity with the heavy chain HCDR1, HCDR2 and HCDR3 shown in the following table;
| LCDR1 | LCDR2 | LCDR3 | HCDR1 | HCDR2 | HCDR3 |
|---|---|---|---|---|---|
| TGTSSDVGGYNY VS | GNSNR PS | QSYDSSL RVV | GFTFSSYA MH | AISGSGGSTYYADS VKG | DRRGSHADAF DV |
| TGTSSDVGGYNY VS | GNSNR PS | QSYDSSL RVV | GFTFSTYA MT | SISSSGESTYYADSV KG | DRRGSHADAF DV |
| TGTSSDVGGYNY VS | GNSNR PS | QSYDSSL RVV | GFTFSTYA MA | EISSSGSRTYYADS VKG | DRRGSHADAF DV |
| TGTSSDVGGYNY VS | GNSNR PS | QSYDSSL RVV | GFTFSTYA MA | AISMSGESTYYADS VKG | DRRGSHADAF DV |
| TGTSSDVGHKFP VS | KNLLR PS | QSYDSSL RVV | GFTFSSYA MH | AISSSGGSTYYADS VKG | DRRGSHADAF DV |
| TGTSSDVGLMHN VS | KSSSRP S | QSYDSSL RVV | GFTFSSYA MH | AISSSGGSTYYADS VKG | DRRGSHADAF DV |
| TGTSSDVGGYNY VS | KSSSRP S | QSYDSSL RVV | GFTFSSYA MH | AISSSGRSTYYADS VEG | DRRGSHADAL NV |
| RSSQSLVHSNGN TYLQ | KVSNR FS | SQSIYVPY T | DYEMH | AIHPGSGDTAYNQR FKG | FYSYAY |
| RSSQSLVHSNGN TYLQ | KVSNR FS | SQSIYVPY TF | DYEMH | AIHPGSGDTAYNQR FKG | FYSYAY |
or
the light chain LCDR1, LCDR2, and LCDR3 of the amino acid sequence of the antigen recognition unit that recognizes claudin 18.2 are independently selected from or have 70-100% sequence identity with the light chain LCDR1, LCDR2, and LCDR3 shown in the following table, and/or the heavy chain HCDR1, HCDR2 and HCDR3 of the amino acid sequence of the antigen recognition unit that recognizes claudin 18.2 are independently selected from or have 70-100% sequence identity with the heavy chain HCDR1, HCDR2 and HCDR3 shown in the following table;
| LCDR1 | LCDR2 | LCDR3 | HCDR1 | HCDR2 | HCDR3 |
|---|---|---|---|---|---|
| KSSQSLLNSGNQKNY LT | WASTR ES | QNDYSYP LT | SYTMH | YINPSSGYTNYNQKF KD | IYYGNSFAY |
| SASSSISYMH | DTSKL AS | HQRSSYP YT | SYDIN | WIYPGDGSTKYNEKF KG | GGYRYDEAM DY |
| KSSQSLLNSGNQKNY LA | GASTR ES | QNDHSYP LT | NYGM N | WINTNTGEPTYAEEF KG | FSYGNSF A Y |
| KSSQSLFNSGNQKNY LT | WASTR ES | QNAYSFP YT | SGYNW H | YIHYTGSTNYNPSLRS | IYNGNSFPY |
| KSSQSLLNSGNQKNY LT | WASTR ES | QNDYSYP LT | SYTMH | YIDPSSGYTNYNQKF KD | IYYGNSFAY |
| KSSQSLLNSGNQKNY LT | WASTR ES | QNDYSYP LT | SYTMH | YINPASGYTNYNQKF KD | IYYGNSFAY |
| KSSQSLLNSGNQKNY LT | WASTR ES | QNDYSYP LT | SYTMH | YINPASGYTNYNQKF KD | IYYGNSFAY |
| KSSQSLLNSGNQKNY LT | WASTR ES | QNDYSYP LT | SYTMH | YINPASGYTNYNQKF KD | IYYGNSFAY |
| KSSQSLFNSGNQKNY LT | WASTR ES | QNAYSFP YT | SGYNW H | YIHYTGSTNYNPALR S | IYNGNSFPY |
| KSSQSLFNSGNQKNY LT | WASTR ES | QNAYSFP YT | SGYNW H | YIHYTGSTNYNPALR S | IYNGNSFPY. |

4. The fusion protein of claim 1, wherein the light chain variable region of the amino acid sequence of the antigen recognition unit that recognizes GPC3 is independently selected from or has 70-100% sequence identity with the light chain variable regions shown in the following table, and/or the heavy chain variable region of the amino acid sequence of the antigen recognition unit that recognizes GPC3 is independently selected from or has 70-100% sequence identity with the heavy chain variable region shown in the following table;
| VH | VL |
|---|---|
| QVQLQESGGGLVQPGRSLRLSCAASGFTFSSYAMHW VRQAPGKGLEWVSAISGSGGSTYYADSVKGRFTISRD NSKNTLYLQMNSLRAEDTAVYYCAKDRRGSHADAF DVWGQGTLVTVSS | QSALTQPPSASGSPGQSVTISCTGTSSDVGGYNY VSWYQQYPGKAPKLLIYGNSNRPSGVPDRFSGS KSGTSASLAITGLQAEDGADYYCQSYDSSLRVV FGGGTKVTVLG |
| QVQLQESGGGLVQPGRSLRLSCAASGFTFSTYAMTW VRQAPGKGLEWVSSISSSGESTYYADSVKGRFTISRD NSKNTLYLQMNSLRAEDTAVYYCAKDRRGSHADAF DVWGQGTLVTVSS | QSALTQPPSASGSPGQSVTISCTGTSSDVGGYNY VSWYQQYPGKAPKLLIYGNSNRPSGVPDRFSGS KSGTSASLAITGLQAEDGADYYCQSYDSSLRVV FGGGTKVTVLG |
| QVQLQESGGGLVQPGRSLRLSCAASGFTFSTYAMAW VRQAPGKGLEWVSEISSSGSRTYYADSVKGRFTISRD NSKNTLYLQMNSLRAEDTAVYYCAKDRRGSHADAF DVWGQGTLVTVSS | QSALTQPPSASGSPGQSVTISCTGTSSDVGGYNY VSWYQQYPGKAPKLLIYGNSNRPSGVPDRFSGS KSGTSASLAITGLQAEDGADYYCQSYDSSLRVV FGGGTKVTVLG |
| QVQLQESGGGLVQPGRSLRLSCAASGFTFSTYAMNW VRQAPGKGLEWVSAISMSGESTYYADSVKGRFTISRD NSKNTLYLQMNSLRAEDTAVYYCAKDRRGSHADAF DVWGQGTLVTVSS | QSALTQPPSASGSPGQSVTISCTGTSSDVGGYNY VSWYQQYPGKAPKLLIYGNSNRPSGVPDRFSGS KSGTSASLAITGLQAEDGADYYCQSYDSSLRVV FGGGTKVTVLG |
| QVQLQESGGGLVQPGRSLRLSCAASGFTFSSYAMHW VRQAPGKGLEWVSAISSSGGSTYYADSVKGRFTISRD NSKNTLYLQMNSLRAEDTAVYYCAKDRRGSHADAF DVWGQGTLVTVSS | QSALTQPPSASGSPGQSVTISCTGTSSDVGHKFP VSWYQQYPGKAPKLLIYKNLLRPSGVPDRFSGS KSGTSASLAITGLQAEDGADYYCQSYDSSLRVV FGGGTKVTVLG |
| QVQLQESGGGLVQPGRSLRLSCAASGFTFSSYAMHW VRQAPGKGLEWVSAISSSGGSTYYADSVKGRFTISRD NSKNTLYLQMNSLRAEDTAVYYCAKDRRGSHADAF DVWGQGTLVTVSS | QSALTQPPSASGSPGQSVTISCTGTSSDVGLMHN VSWYQQYPGKAPKLLIYKSSSRPSGVPDRFSGS KSGTSASLAITGLQAEDGADYYCQSYDSSLRVV FGGGTKVTVLG |
| QVQLQESGGGLVQPGRSLRLSCAASGFTFSSYAMHW VRQAPGKGLEWVSAISSSGRSTYYADSVEGRFTISRD NSKNTLYLQMNSLRAEDTAVYYCAKDRRGSHADAL NVWGQGTLVTVSS | QSALTQPPSASGSPGQSVTISCTGTSSDVGGYNY VSWYQQYPGKAPKLLIYGNSNRPSGVPDRFSGS KSGTSASLAITGLQAEDGADYYCQSYDSSLRVV FGGGTKVTVLG |
| EVQLVQSGAEVKKPGASVKVSCKASGYTFSDYEMH WVRQAPGQGLEWMGAIHPGSGDTAYNQRFKGRVTIT ADKSTSTAYMELSSLRSEDTAVYYCARFYSYAYWGQ GTLVTVSA | DIVMTQTPLSLPVTPGEPASISCRSSQSLVHSNG NTYLQWYLQKPGQSPQLLIYKVSNRFSGVPDRF SGSGSGTDFTLKISRVEAEDVGVYYCSQSIYVPY TFGQGTKLEIKR |
| QVQLQQSGTELVRPGASVKLSCKALGYTFTDYEMHW VKQTPVHGLEWIGAIHPGSGDTAYNQRFKGKATLTA DKSSSTAYMEYSSLTSEDSAVYYCTRFYSYAYWGQG | DVVMTQTPLSLPVSLGDQASISCRSSQSLVHSN GNTYLQWYLQKPGQSPKLLIYKVSNRFSGVPDR FSGRGSGTDFTLKISRVEAEDLGVYFCSQSIYVP |
| TLVTVSA | YTFGGGTKLEIKR |
or,
the light chain variable region of the amino acid sequence of the antigen recognition unit that recognizes claudin18.2 is independently selected from or has 70-100% sequence identity with the light chain variable regions shown in the following table, and/or the heavy chain variable region of the amino acid sequence of the antigen recognition unit that recognizes claudin18.2 is independently selected from or has 70-100% sequence identity with the heavy chain variable region shown in the following table;
| VH | VL |
|---|---|
| QVQLQQSGAELARPGASVKMSCKASGYTFTSYT MHWVKQRPGQGLEWIGYINPSSGYTNYNQKFKD KATLTADKSSSTAYMQLSSLTSEDSAVYYCARIY YGNSFAYWGQGTTVTVSS | DIVMTQSPSSLTVTAGEKVTMSCKSSQSLLNS GNQKNYLTWYQQKPGQPPKLLIYWASTRESG VPDRFTGSGSGTDFTLTISSVQAEDLAVYYCQ NDYSYPLTFGAGTKLELKR |
| QVQLQQSGPELVKPGALVKISCKASGYTFTSYDIN WVKQRPGQGLEWIGWIYPGDGSTKYNEKFKGKA TLTADKSSSTAYMQLSSLTSENSAVYFCARGGYR YDEAMDYWGQGTTVTVSS | QIVLTQSPAIMSASPGEKVTMTCSASSSISYMH WYQQKPGTSPKRWIYDTSKLASGVPARFSGS GSGTSYSLTISSMEAEDAATYYCHQRSSYPYT FGGGTKLEIKR |
| QIQLVQSGPELKKPGETVKISCKASGYTFTNYGM NWVKQAPGKGLKWMGWINTNTGEPTYAEEFKG RFAFSLETSASTAYLQINNLKNEDTATYFCARFSY GNSFAYWGQGTTVTVSS | DIVMTQSPSSLSVSAGEKVTMSCKSSQSLLNS GNQKNYLAWYQQKPGQPPKLLIYGASTRESG VPDRFTGSGSGTDFTLTISSVQAEDLAVYYCQ NDHSYPLTFGAGTKLELKR |
| DVQLQESGPDLVKPSQSLSLTCTVTGYSITSGYNW HWIRQFPGNKMEWMGYIHYTGSTNYNPSLRSRISI TRDTSKNQFFLQLNSVTTDDTATYYCTRIYNGNSF PYWGQGTSVTVSS | DIVMTQSPSSLTVTPGEKVTMTCKSSQSLFNS GNQKNYLTWYQQRPGQPPKMLIYWASTRES GVPDRFTGSGSGTDFTLTISSVQAEDLAVFYC QNAYSFPYTFGGGTKLEIKR |
| QVQLQQSGAELARPGASVKMSCKASGYTFTSYT MHWVKQRPGQGLEWIGYIDPSSGYTNYNQKFKD KATLTADKSSSTAYMQLSSLTSEDSAVYYCARIY YGNSFAYWGQGTTVTVSS | DIVMTQSPSSLTVTAGEKVTMSCKSSQSLLNS GNQKNYLTWYQQKPGQPPKLLIYWASTRESG VPDRFTGSGSGTDFTLTISSVQAEDLAVYYCQ NDYSYPLTFGAGTKLELKR |
| QVQLQQSGAELARPGASVKMSCKASGYTFTSYT MHWVKQRPGQGLEWIGYINPASGYTNYNQKFKD KATLTADKSSSTAYMQLSSLTSEDSAVYYCARIY YGNSFAYWGQGTTVTVSS | DIVMTQSPSSLTVTAGEKVTMSCKSSQSLLNS GNQKNYLTWYQQKPGQPPKLLIYWASTRESG VPDRFTGSGSGTDFTLTISSVQAEDLAVYYCQ NDYSYPLTFGAGTKLELKR |
| QVQLQQSGAELARPGASVKMSCKASGYTFTSYT MHWVKQRPGQGLEWIGYINPASGYTNYNQKFKD KATLTADKSSSTAYMQLSSLTSEDSAVYYCARIY YGNSFAYWGQGTTVTVSS | DIVMTQSPSSLTVTAGEKVTMSCKSSQSLLNS GNQKNYLTWYQQKPGQPPKLLIYWASTRESG VPDRFTGSGSGTDFTLTISSVQAEDLAVYYCQ NDYSYPLTFGAGTKLELKR |
| QVQLVQSGAEVKKPGASVKVSCKASGYTFTSYT MHWVRQAPGQGLEWMGYINPASGYTNYNQKFK DRVTMTRDTSTSTAYMELSSLRSEDTAVYYCARI YYGNSFAYWGQGTLVTVSS | DIVMTQSPDSLAVSLGERATINCKSSQSLLNSG NQKNYLTWYQQKPGQPPKLLIYWASTRESGV PDRFSGSGSGTDFTLTISSLQAEDVAVYYCQN DYSYPLTFGGGTKVEIKR |
| QVQLQESGPGLVKPSQTLSLTCTVSGGSISSGYNW HWIRQPPGKGLEWIGYIHYTGSTNYNPALRSRVTI SVDTSKNQFSLKLSSVTAADTAVYYCARIYNGNS FPYWGQGTTVTVSS | DIVMTQSPDSLAVSLGERATINCKSSQSLFNSG NQKNYLTWYQQKPGQPPKLLIYWASTRESGV PDRFSGSGSGTDFTLTISSLQAEDVAVYYCQN AYSFPYTFGGGTKLEIKR |
| QVQLQESGPGLIKPSQTLSLTCTVSGGSISSGYNW HWIRQPPGKGLEWIGYIHYTGSTNYNPALRSRVTI SVDTSKNQFSLKLSSVTAADTAIYYCARIYNGNSF PYWGQGTTVTVSS | DIVMTQSPDSLAVSLGERATINCKSSQSLFNSG NQKNYLTWYQQKPGQPPKLLIYWASTRESGV PDRFSGSGSGTDFTLTISSLQAEDVAVYYCQN AYSFPYTFGGGTKLEIKR. |

5. The fusion protein of claim 1, wherein the light chain LCDR1, LCDR2, and LCDR3 of the amino acid sequence of the antigen recognition unit that recognizes GPC3 are or have 70-100% sequence identity with a combination of the light chain LCDR1, LCDR2, and LCDR3 shown in any row of the following table, and/or the heavy chain HCDR1, HCDR2, and HCDR3 of the amino acid sequence of the antigen recognition unit that recognizes GPC3 are or have 70-100% sequence identity with a combination of the heavy chain HCDR1, HCDR2, and HCDR3 shown in any row of the following table;
| LCDR1 | LCDR 2 | LCDR3 | HCDR1 | HCDR2 | HCDR3 |
|---|---|---|---|---|---|
| TGTSSDVGGYNY VS | GNSN RPS | QSYDSSLR VV | GFTFSSYA MH | AISGSGGSTYYADS VKG | DRRGSHAD AFDV |
| TGTSSDVGGYNY VS | GNSN RPS | QSYDSSLR VV | GFTFSTYA MT | SISSSGESTYYADS VKG | DRRGSHAD AFDV |
| TGTSSDVGGYNY VS | GNSN RPS | QSYDSSLR VV | GFTFSTYA MA | EISSSGSRTYYADS VKG | DRRGSHAD AFDV |
| TGTSSDVGGYNY VS | GNSN RPS | QSYDSSLR VV | GFTFSTYA MA | AISMSGESTYYADS VKG | DRRGSHAD AFDV |
| TGTSSDVGHKFP VS | KNLL RPS | QSYDSSLR VV | GFTFSSYA MH | AISSSGGSTYYADS VKG | DRRGSHAD AFDV |
| TGTSSDVGLMHN VS | KSSSR PS | QSYDSSLR VV | GFTFSSYA MH | AISSSGGSTYYADS VKG | DRRGSHAD AFDV |
| TGTSSDVGGYNY VS | KSSSR PS | QSYDSSLR VV | GFTFSSYA MH | AISSSGRSTYYADS VEG | DRRGSHAD ALNV |
| RSSQSLVHSNGN TYLQ | KVSN RFS | SQSIYVPY T | DYEMH | AIHPGSGDTAYNQ RFKG | FYSYAY |
| RSSQSLVHSNGN TYLQ | KVSN RFS | SQSIYVPY TF | DYEMH | AIHPGSGDTAYNQ RFKG | FYSYAY; |
or
the light chain LCDR1, LCDR2, and LCDR3 of the amino acid sequence of the antigen recognition unit that recognizes claudin18.2 are or have 70-100% sequence identity with a combination of the light chain LCDR1, LCDR2, and LCDR3 shown in any row of the following table, and/or the heavy chain HCDR1, HCDR2, and HCDR3 of the amino acid sequence of the antigen recognition unit that recognizes claudin18.2 are or have 70-100% sequence identity with a combination of the heavy chain HCDR1, HCDR2, and HCDR3 shown in any row of the following table;
| LCDR1 | LCDR2 | LCDR3 | HCDR1 | HCDR2 | HCDR3 |
|---|---|---|---|---|---|
| KSSQSLLNSGNQK NYLT | WASTR ES | QNDYSYP LT | SYTMH | YINPSSGYTNYNQK FKD | IYYGNSFAY |
| SASSSISYMH | DTSKL AS | HQRSSYP YT | SYDIN | WIYPGDGSTKYNEK FKG | GGYRYDEA MDY |
| KSSQSLLNSGNQK NYLA | GASTR ES | QNDHSYP LT | NYGM N | WINTNTGEPTYAEE FKG | FSYGNSF A Y |
| KSSQSLFNSGNQK NYLT | WASTR ES | QNAYSFP YT | SGYNW H | YIHYTGSTNYNPSL RS | IYNGNSFPY |
| KSSQSLLNSGNQK NYLT | WASTR ES | QNDYSYP LT | SYTMH | YIDPSSGYTNYNQK FKD | IYYGNSFAY |
| KSSQSLLNSGNQK NYLT | WASTR ES | QNDYSYP LT | SYTMH | YINPASGYTNYNQK FKD | IYYGNSFAY |
| KSSQSLLNSGNQK NYLT | WASTR ES | QNDYSYP LT | SYTMH | YINPASGYTNYNQK FKD | IYYGNSFAY |
| KSSQSLLNSGNQK NYLT | WASTR ES | QNDYSYP LT | SYTMH | YINPASGYTNYNQK FKD | IYYGNSFAY |
| KSSQSLFNSGNQK NYLT | WASTR ES | QNAYSFP YT | SGYNW H | YIHYTGSTNYNPAL RS | IYNGNSFPY |
| KSSQSLFNSGNQK NYLT | WASTR ES | QNAYSFP YT | SGYNW H | YIHYTGSTNYNPAL RS | IYNGNSFPY_{∘} |

6. The fusion protein of claim 1, wherein the light chain variable region and the heavy chain variable region of the amino acid sequence of the antigen recognition unit that recognizes GPC3 are or have 70-100% sequence identity with a combination of the light chain variable region and the heavy chain variable region shown in any row of the following table;
| VH | VL |
|---|---|
| QVQLQESGGGLVQPGRSLRLSCAASGFTFSSYAMHW | QSALTQPPSASGSPGQSVTISCTGTSSDVGGYNY |
| VRQAPGKGLEWVSAISGSGGSTYYADSVKGRFTISR DNSKNTLYLQMNSLRAEDTAVYYCAKDRRGSHADA FDVWGQGTLVTVSS | VSWYQQYPGKAPKLLIYGNSNRPSGVPDRFSGS KSGTSASLAITGLQAEDGADYYCQSYDSSLRVV FGGGTKVTVLG |
| QVQLQESGGGLVQPGRSLRLSCAASGFTFSTYAMTW VRQAPGKGLEWVSSISSSGESTYYADSVKGRFTISRD NSKNTLYLQMNSLRAEDTAVYYCAKDRRGSHADAF DVWGQGTLVTVSS | QSALTQPPSASGSPGQSVTISCTGTSSDVGGYNY VSWYQQYPGKAPKLLIYGNSNRPSGVPDRFSGS KSGTSASLAITGLQAEDGADYYCQSYDSSLRVV FGGGTKVTVLG |
| QVQLQESGGGLVQPGRSLRLSCAASGFTFSTYAMAW VRQAPGKGLEWVSEISSSGSRTYYADSVKGRFTISRD NSKNTLYLQMNSLRAEDTAVYYCAKDRRGSHADAF DVWGQGTLVTVSS | QSALTQPPSASGSPGQSVTISCTGTSSDVGGYNY VSWYQQYPGKAPKLLIYGNSNRPSGVPDRFSGS KSGTSASLAITGLQAEDGADYYCQSYDSSLRVV FGGGTKVTVLG |
| QVQLQESGGGLVQPGRSLRLSCAASGFTFSTYAMNW VRQAPGKGLEWVSAISMSGESTYYADSVKGRFTISR DNSKNTLYLQMNSLRAEDTAVYYCAKDRRGSHADA FDVWGQGTLVTVSS | QSALTQPPSASGSPGQSVTISCTGTSSDVGGYNY VSWYQQYPGKAPKLLIYGNSNRPSGVPDRFSGS KSGTSASLAITGLQAEDGADYYCQSYDSSLRVV FGGGTKVTVLG |
| QVQLQESGGGLVQPGRSLRLSCAASGFTFSSYAMHW VRQAPGKGLEWVSAISSSGGSTYYADSVKGRFTISRD NSKNTLYLQMNSLRAEDTAVYYCAKDRRGSHADAF DVWGQGTLVTVSS | QSALTQPPSASGSPGQSVTISCTGTSSDVGHKFP VSWYQQYPGKAPKLLIYKNLLRPSGVPDRFSGS KSGTSASLAITGLQAEDGADYYCQSYDSSLRVV FGGGTKVTVLG |
| QVQLQESGGGLVQPGRSLRLSCAASGFTFSSYAMHW VRQAPGKGLEWVSAISSSGGSTYYADSVKGRFTISRD NSKNTLYLQMNSLRAEDTAVYYCAKDRRGSHADAF DVWGQGTLVTVSS | QSALTQPPSASGSPGQSVTISCTGTSSDVGLMHN VSWYQQYPGKAPKLLIYKSSSRPSGVPDRFSGSK SGTSASLAITGLQAEDGADYYCQSYDSSLRVVF GGGTKVTVLG |
| QVQLQESGGGLVQPGRSLRLSCAASGFTFSSYAMHW VRQAPGKGLEWVSAISSSGRSTYYADSVEGRFTISRD NSKNTLYLQMNSLRAEDTAVYYCAKDRRGSHADAL NVWGQGTLVTVSS | QSALTQPPSASGSPGQSVTISCTGTSSDVGGYNY VSWYQQYPGKAPKLLIYGNSNRPSGVPDRFSGS KSGTSASLAITGLQAEDGADYYCQSYDSSLRVV FGGGTKVTVLG |
| EVQLVQSGAEVKKPGASVKVSCKASGYTFSDYEMH WVRQAPGQGLEWMGAIHPGSGDTAYNQRFKGRVTI TADKSTSTAYMELSSLRSEDTAVYYCARFYSYAYWG QGTLVTVSA | DIVMTQTPLSLPVTPGEPASISCRSSQSLVHSNGN TYLQWYLQKPGQSPQLLIYKVSNRFSGVPDRFS GSGSGTDFTLKISRVEAEDVGVYYCSQSIYVPYT FGQGTKLEIKR |
| QVQLQQSGTELVRPGASVKLSCKALGYTFTDYEMH WVKQTPVHGLEWIGAIHPGSGDTAYNQRFKGKATLT ADKSSSTAYMEYSSLTSEDSAVYYCTRFYSYAYWGQ GTLVTVSA | DVVMTQTPLSLPVSLGDQASISCRSSQSLVHSNG NTYLQWYLQKPGQSPKLLIYKVSNRFSGVPDRF SGRGSGTDFTLKISRVEAEDLGVYFCSQSIYVPY TFGGGTKLEIKR; |
or,
the light chain variable region and the heavy chain variable region of the amino acid sequence of the antigen recognition unit that recognizes claudin 18.2 are or have 70-100% sequence identity with a combination of the light chain variable region and the heavy chain variable region shown in any row of the following table;
| VH | VL |
|---|---|
| QVQLQQSGAELARPGASVKMSCKASGYTFTSYTMH WVKQRPGQGLEWIGYINPSSGYTNYNQKFKDKATLT ADKSSSTAYMQLSSLTSEDSAVYYCARIYYGNSFAY WGQGTTVTVSS | DIVMTQSPSSLTVTAGEKVTMSCKSSQSLLNSGNQ KNYLTWYQQKPGQPPKLLIYWASTRESGVPDRFT GSGSGTDFTLTISSVQAEDLAVYYCQNDYSYPLTF GAGTKLELKR |
| QVQLQQSGPELVKPGALVKISCKASGYTFTSYDINW VKQRPGQGLEWIGWIYPGDGSTKYNEKFKGKATLTA DKSSSTAYMQLSSLTSENSAVYFCARGGYRYDEAMD YWGQGTTVTVSS | QIVLTQSPAIMSASPGEKVTMTCSASSSISYMHWY QQKPGTSPKRWIYDTSKLASGVPARFSGSGSGTSY SLTISSMEAEDAATYYCHQRSSYPYTFGGGTKLEI KR |
| QIQLVQSGPELKKPGETVKISCKASGYTFTNYGMNW VKQAPGKGLKWMGWINTNTGEPTYAEEFKGRFAFS LETSASTAYLQINNLKNEDTATYFCARFSYGNSFAY WGQGTTVTVSS | DIVMTQSPSSLSVSAGEKVTMSCKSSQSLLNSGNQ KNYLAWYQQKPGQPPKLLIYGASTRESGVPDRFT GSGSGTDFTLTISSVQAEDLAVYYCQNDHSYPLTF GAGTKLELKR |
| DVQLQESGPDLVKPSQSLSLTCTVTGYSITSGYNWH WIRQFPGNKMEWMGYIHYTGSTNYNPSLRSRISITRD TSKNQFFLQLNSVTTDDTATYYCTRIYNGNSFPYWG QGTSVTVSS | DIVMTQSPSSLTVTPGEKVTMTCKSSQSLFNSGNQ KNYLTWYQQRPGQPPKMLIYWASTRESGVPDRFT GSGSGTDFTLTISSVQAEDLAVFYCQNAYSFPYTF GGGTKLEIKR |
| QVQLQQSGAELARPGASVKMSCKASGYTFTSYTMH WVKQRPGQGLEWIGYIDPSSGYTNYNQKFKDKATLT ADKSSSTAYMQLSSLTSEDSAVYYCARIYYGNSFAY WGQGTTVTVSS | DIVMTQSPSSLTVTAGEKVTMSCKSSQSLLNSGNQ KNYLTWYQQKPGQPPKLLIYWASTRESGVPDRFT GSGSGTDFTLTISSVQAEDLAVYYCQNDYSYPLTF GAGTKLELKR |
| QVQLQQSGAELARPGASVKMSCKASGYTFTSYTMH WVKQRPGQGLEWIGYINPASGYTNYNQKFKDKATL | DIVMTQSPSSLTVTAGEKVTMSCKSSQSLLNSGNQ KNYLTWYQQKPGQPPKLLIYWASTRESGVPDRFT |
| TADKSSSTAYMQLSSLTSEDSAVYYCARIYYGNSFA YWGQGTTVTVSS | GSGSGTDFTLTISSVQAEDLAVYYCQNDYSYPLTF GAGTKLELKR |
| QVQLQQSGAELARPGASVKMSCKASGYTFTSYTMH WVKQRPGQGLEWIGYINPASGYTNYNQKFKDKATL TADKSSSTAYMQLSSLTSEDSAVYYCARIYYGNSFA YWGQGTTVTVSS | DIVMTQSPSSLTVTAGEKVTMSCKSSQSLLNSGNQ KNYLTWYQQKPGQPPKLLIYWASTRESGVPDRFT GSGSGTDFTLTISSVQAEDLAVYYCQNDYSYPLTF GAGTKLELKR |
| QVQLVQSGAEVKKPGASVKVSCKASGYTFTSYTMH WVRQAPGQGLEWMGYINPASGYTNYNQKFKDRVT MTRDTSTSTAYMELSSLRSEDTAVYYCARIYYGNSF AYWGQGTLVTVSS | DIVMTQSPDSLAVSLGERATINCKSSQSLLNSGNQ KNYLTWYQQKPGQPPKLLIYWASTRESGVPDRFS GSGSGTDFTLTISSLQAEDVAVYYCQNDYSYPLTF GGGTKVEIKR |
| QVQLQESGPGLVKPSQTLSLTCTVSGGSISSGYNWH WIRQPPGKGLEWIGYIHYTGSTNYNPALRSRVTISVD TSKNQFSLKLSSVTAADTAVYYCARIYNGNSFPYWG QGTTVTVSS | DIVMTQSPDSLAVSLGERATINCKSSQSLFNSGNQ KNYLTWYQQKPGQPPKLLIYWASTRESGVPDRFS GSGSGTDFTLTISSLQAEDVAVYYCQNAYSFPYTF GGGTKLEIKR |
| QVQLQESGPGLIKPSQTLSLTCTVSGGSISSGYNWHW IRQPPGKGLEWIGYIHYTGSTNYNPALRSRVTISVDTS KNQFSLKLSSVTAADTAIYYCARIYNGNSFPYWGQG TTVTVSS | DIVMTQSPDSLAVSLGERATINCKSSQSLFNSGNQ KNYLTWYQQKPGQPPKLLIYWASTRESGVPDRFS GSGSGTDFTLTISSLQAEDVAVYYCQNAYSFPYTF GGGTKLEIKR. |

7. The fusion protein of claim 1, wherein the amino acid sequence of the antigen recognition unit that recognizes GPC3 is selected from or has 70-100% sequence identity with the sequence shown in the following table; 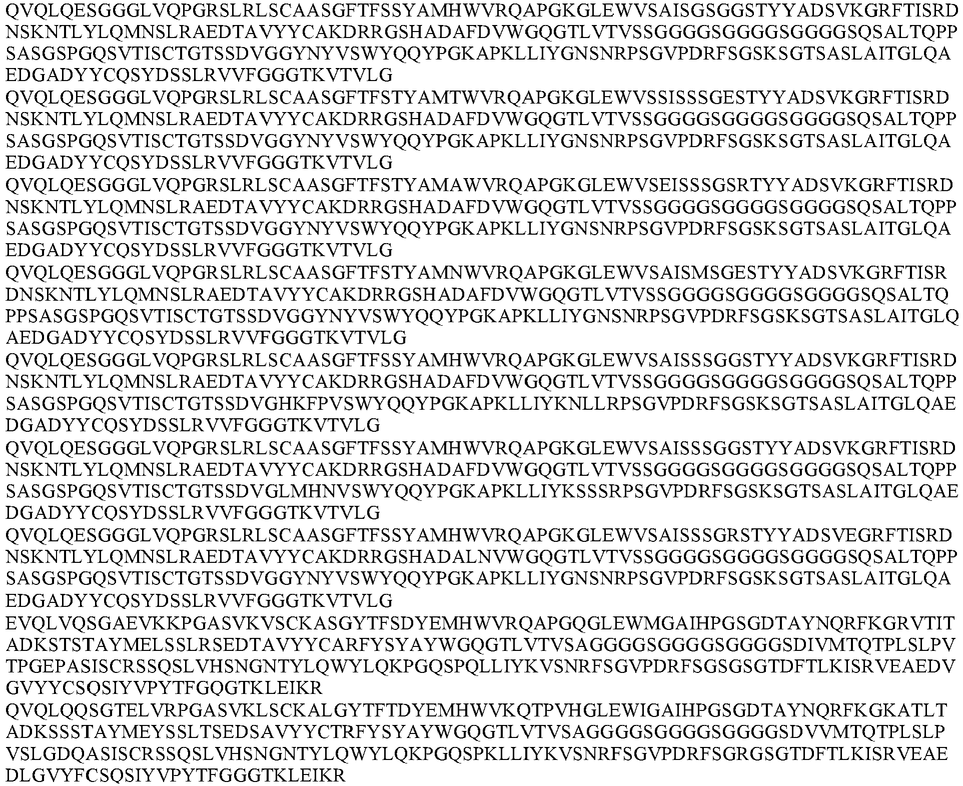 or,
the amino acid sequence of the antigen recognition unit that recognizes claudin18.2 is selected from or has 70-100% sequence identity with the sequence shown in the following table;

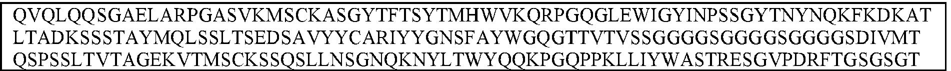

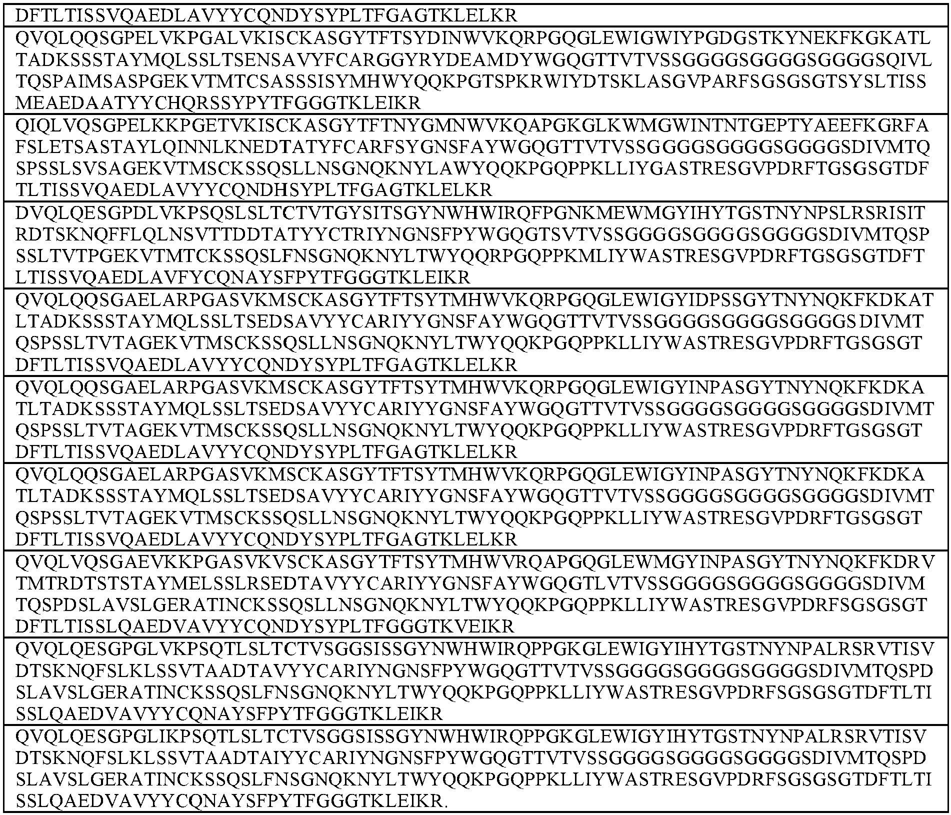

8. A combination of the fusion protein of any one of claims 1-7 and other factors, wherein the other factors includes cytokines, transcription factors, chemokines, and/or combinations thereof, and the expression cassette is constitutively or inducibly expressed; preferably, the promoter of the expression cassette is an immune cell inducible promoter; preferably, the immune cell inducible promoter is NFAT6 promoter; and preferably, the NFAT6 promoter is reversely regulated.

9. A nucleic acid molecule expressing the fusion protein of any one of claims 1-8 or the combination of claim 2.

10. A vector comprising the nucleic acid molecule of claim 9.

11. A cell comprising the vector of claim 10 or having the nucleic acid molecule of claim 9 integrated into its genome.

12. A protein complex comprising:
i) the fusion protein TFP molecule of any one of claims 1-7; and
ii) at least one endogenous TCR subunit or endogenous TCR complex.

13. A method for preparing cells, comprising transducing T cells with the vector of claim 10 or the nucleic acid molecule of claim 9.

14. A method for producing an RNA-engineered cell population, comprising introducing *in vitro* transcribed RNA or synthetic RNA into the cell,
(i) wherein the RNA includes the nucleic acid of claim 9.

15. A method for providing anti-tumor immunities in a mammal, comprising administering to the mammal an effective amount of the fusion protein of any one of claims 1-7 and the combination of claim 8, the nucleic acid molecule of claim 9, the vector of claim 10, or the cell of claim 11.

16. A method for treating a mammal suffering from a disease related to the expression of GPC3 or claudin 18.2, comprising administering to the mammal an effective amount of the fusion protein of any one of claims 1-7, the combination of claim 8, the nucleic acid molecule of claim 9, the vector of claim 10, or the cell of claim 11.

17. The fusion protein of any one of claims 1-7, the combination of claim 8, the nucleic acid molecule of claim 9, the vector of claim 10, or the cell of claim 11 as a medicament.


Sequence listing

<110>  CARSGEN THERAPEUTICS CO., LTD.
       SHANGHAI CANCER INSTITUTE

<120>  TCR FUSION PROTEIN AND CELL EXPRESSING TCR FUSION PROTEIN

<130>  P2020-0124

<150>  CN201910105233O
<151>  2019-02-01

<150>  CN201910838439 4
<151>  2019-09-05

<160>  208

<170>  PatentIn version 3.5

<210>  1
<211>  247
<212>  PRT
<213>  Artificial sequence

<220>
<223>  Synthesized polypeptide

<400>  1

Gln Val Gln Leu Gln Glu Ser Gly Pro Gly Leu Ile Lys Pro Ser Gln
1               5                   10                  15

Thr Leu Ser Leu Thr Cys Thr Val Ser Gly Gly Ser Ile Ser Ser Gly
            20                  25                  30

Tyr Asn Trp His Trp Ile Arg Gln Pro Pro Gly Lys Gly Leu Glu Trp
        35                  40                  45

Ile Gly Tyr Ile His Tyr Thr Gly Ser Thr Asn Tyr Asn Pro Ala Leu
    50                  55                  60

Arg Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Ile Tyr Tyr Cys
                85                  90                  95

Ala Arg Ile Tyr Asn Gly Asn Ser Phe Pro Tyr Trp Gly Gln Gly Thr
            100                 105                 110

Thr Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Asp Ser Leu
    130                 135                 140

Ala Val Ser Leu Gly Glu Arg Ala Thr Ile Asn Cys Lys Ser Ser Gln
145                 150                 155                 160

Ser Leu Phe Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr
            180                 185                 190

Arg Glu Ser Gly Val Pro Asp Arg Phe Ser Gly Ser Gly Ser Gly Thr
        195                 200                 205

Asp Phe Thr Leu Thr Ile Ser Ser Leu Gln Ala Glu Asp Val Ala Val
    210                 215                 220

Tyr Tyr Cys Gln Asn Ala Tyr Ser Phe Pro Tyr Thr Phe Gly Gly Gly
225                 230                 235                 240

Thr Lys Leu Glu Ile Lys Arg
                245

<210>  2
<211>  29
<212>  PRT
<213>  Artificial sequence

<220>
<223>  Synthesized polypeptide

<400>  2

Ala Ala Ile Glu Val Met Tyr Pro Pro Pro Tyr Leu Gly Gly Gly Gly
1               5                   10                  15

Ser Gly Gly Gly Gly Ser Gly Gly Gly Gly Ser Leu Glu
            20                  25

<210>  3
<211>  185
<212>  PRT
<213>  Artificial sequence

<220>
<223>  Synthesized polypeptide

<400>  3

Asp Gly Asn Glu Glu Met Gly Gly Ile Thr Gln Thr Pro Tyr Lys Val
1               5                   10                  15

Ser Ile Ser Gly Thr Thr Val Ile Leu Thr Cys Pro Gln Tyr Pro Gly

20                  25                  30

Ser Glu Ile Leu Trp Gln His Asn Asp Lys Asn Ile Gly Gly Asp Glu
        35                  40                  45

Asp Asp Lys Asn Ile Gly Ser Asp Glu Asp His Leu Ser Leu Lys Glu
    50                  55                  60

Phe Ser Glu Leu Glu Gln Ser Gly Tyr Tyr Val Cys Tyr Pro Arg Gly
65                  70                  75                  80

Ser Lys Pro Glu Asp Ala Asn Phe Tyr Leu Tyr Leu Arg Ala Arg Val
                85                  90                  95

Cys Glu Asn Cys Met Glu Met Asp Val Met Ser Val Ala Thr Ile Val
            100                 105                 110

Ile Val Asp Ile Cys Ile Thr Gly Gly Leu Leu Leu Leu Val Tyr Tyr
        115                 120                 125

Trp Ser Lys Asn Arg Lys Ala Lys Ala Lys Pro Val Thr Arg Gly Ala
    130                 135                 140

Gly Ala Gly Gly Arg Gln Arg Gly Gln Asn Lys Glu Arg Pro Pro Pro
145                 150                 155                 160

Val Pro Asn Pro Asp Tyr Glu Pro Ile Arg Lys Gly Gln Arg Asp Leu
                165                 170                 175

Tyr Ser Gly Leu Asn Gln Arg Arg Ile
            180                 185

<210>  4
<211>  741
<212>  DNA
<213>  Artificial sequence

<220>
<223>  Synthesized polynucleotide

<400>  4
caggtgcagc tgcaggagag cggccccggc ctgatcaagc ccagccagac cctgagcctg      60

acctgcaccg tgagcggcgg cagcatcagc agcggctaca actggcactg gatccggcag     120

ccccccggca agggcctgga gtggatcggc tacatccact acaccggcag caccaactac     180

aaccccgccc tgcggagccg ggtgaccatc agcgtggaca ccagcaagaa ccagttcagc     240

ctgaagctga gcagcgtgac cgccgccgac accgccatct actactgcgc ccggatctac     300

aacggcaaca gcttccccta ctggggccag ggcaccaccg tgaccgtgag cagcggtgga     360

ggcggttcag gcggaggtgg ttctggcggt ggcggatcgg acatcgtgat gacccagagc 420

cccgacagcc tggccgtgag cctgggcgag cgggccacca tcaactgcaa gagcagccag 480

agcctgttca acagcggcaa ccagaagaac tacctgacct ggtaccagca gaagcccggc 540

cagcccccca agctgctgat ctactgggcc agcacccggg agagcggcgt gcccgaccgg 600

ttcagcggca gcggcagcgg caccgacttc accctgacca tcagcagcct gcaggccgag 660

gacgtggccg tgtactactg ccagaacgcc tacagcttcc cctacacctt cggcggcggc 720

accaagctgg agatcaagcg g 741

<210> 5
<211> 90
<212> DNA
<213> Artificial sequence

<220>
<223> Synthesized polynucleotide

<400> 5
gcggccgcaa ttgaagttat gtatcctcct ccttacctag gtggcggcgg ttctggtggc 60

ggcggttctg gtggcggcgg ttctctcgag 90

<210> 6
<211> 555
<212> DNA
<213> Artificial sequence

<220>
<223> Synthesized polynucleotide

<400> 6
gatggtaatg aagaaatggg tggtattaca cagacaccat ataaagtctc catctctgga 60

accacagtaa tattgacatg ccctcagtat cctggatctg aaatactatg gcaacacaat 120

gataaaaaca taggcggtga tgaggatgat aaaaacatag gcagtgatga ggatcacctg 180

tcactgaagg aattttcaga attggagcaa agtggttatt atgtctgcta ccccagagga 240

agcaaaccag aagatgcgaa cttttatctc tacctgaggg caagagtgtg tgagaactgc 300

atggagatgg atgtgatgtc ggtggccaca attgtcatag tggacatctg catcactggg 360

ggcttgctgc tgctggttta ctactggagc aagaatagaa aggccaaggc caagcctgtg 420

acacgaggag cgggtgctgg cggcaggcaa aggggacaaa acaaggagag gccaccacct 480

gttcccaacc cagactatga gcccatccgg aaaggccagc gggacctgta ttctggcctg 540

aatcagagac gcatc 555

<210> 7
<211> 1386
<212> DNA

<213>  Artificial sequence

<220>
<223>  Synthesized polynucleotide

<400>  7
caggtgcagc tgcaggagag cggccccggc ctgatcaagc ccagccagac cctgagcctg      60

acctgcaccg tgagcggcgg cagcatcagc agcggctaca actggcactg gatccggcag     120

ccccccggca agggcctgga gtggatcggc tacatccact acaccggcag caccaactac     180

aaccccgccc tgcggagccg ggtgaccatc agcgtggaca ccagcaagaa ccagttcagc     240

ctgaagctga gcagcgtgac cgccgccgac accgccatct actactgcgc ccggatctac     300

aacggcaaca gcttccccta ctggggccag ggcaccaccg tgaccgtgag cagcggtgga     360

ggcggttcag gcggaggtgg ttctggcggt ggcggatcgg acatcgtgat gacccagagc     420

cccgacagcc tggccgtgag cctgggcgag cgggccacca tcaactgcaa gagcagccag     480

agcctgttca acagcggcaa ccagaagaac tacctgacct ggtaccagca gaagcccggc     540

cagcccccca agctgctgat ctactgggcc agcacccggg agagcggcgt gcccgaccgg     600

ttcagcggca gcggcagcgg caccgacttc accctgacca tcagcagcct gcaggccgag     660

gacgtggccg tgtactactg ccagaacgcc tacagcttcc cctacacctt cggcggcggc     720

accaagctgg agatcaagcg ggcggccgca attgaagtta tgtatcctcc tccttaccta     780

ggtggcggcg gttctggtgg cggcggttct ggtggcggcg gttctctcga ggatggtaat     840

gaagaaatgg gtggtattac acagacacca tataaagtct ccatctctgg aaccacagta     900

atattgacat gccctcagta tcctggatct gaaatactat ggcaacacaa tgataaaaac     960

ataggcggtg atgaggatga taaaaacata ggcagtgatg aggatcacct gtcactgaag    1020

gaattttcag aattggagca aagtggttat tatgtctgct accccagagg aagcaaacca    1080

gaagatgcga acttttatct ctacctgagg gcaagagtgt gtgagaactg catggagatg    1140

gatgtgatgt cggtggccac aattgtcata gtggacatct gcatcactgg gggcttgctg    1200

ctgctggttt actactggag caagaataga aaggccaagg ccaagcctgt gacacgagga    1260

gcgggtgctg gcggcaggca aaggggacaa aacaaggaga ggccaccacc tgttcccaac    1320

ccagactatg agcccatccg gaaaggccag cgggacctgt attctggcct gaatcagaga    1380

cgcatc                                                               1386

<210>  8
<211>  160
<212>  PRT
<213>  Artificial sequence

<220>
<223>  Synthesized polypeptide

<400>  8

Gln Ser Ile Lys Gly Asn His Leu Val Lys Val Tyr Asp Tyr Gln Glu
1               5                   10                  15

Asp Gly Ser Val Leu Leu Thr Cys Asp Ala Glu Ala Lys Asn Ile Thr
            20                  25                  30

Trp Phe Lys Asp Gly Lys Met Ile Gly Phe Leu Thr Glu Asp Lys Lys
        35                  40                  45

Lys Trp Asn Leu Gly Ser Asn Ala Lys Asp Pro Arg Gly Met Tyr Gln
    50                  55                  60

Cys Lys Gly Ser Gln Asn Lys Ser Lys Pro Leu Gln Val Tyr Tyr Arg
65                  70                  75                  80

Met Cys Gln Asn Cys Ile Glu Leu Asn Ala Ala Thr Ile Ser Gly Phe
                85                  90                  95

Leu Phe Ala Glu Ile Val Ser Ile Phe Val Leu Ala Val Gly Val Tyr
            100                 105                 110

Phe Ile Ala Gly Gln Asp Gly Val Arg Gln Ser Arg Ala Ser Asp Lys
        115                 120                 125

Gln Thr Leu Leu Pro Asn Asp Gln Leu Tyr Gln Pro Leu Lys Asp Arg
    130                 135                 140

Glu Asp Asp Gln Tyr Ser His Leu Gln Gly Asn Gln Leu Arg Arg Asn
145                 150                 155                 160

<210>  9
<211>  480
<212>  DNA
<213>  Artificial sequence

<220>
<223>  Synthesized polynucleotide

<400>  9
cagtcaatca aaggaaacca cttggttaag gtgtatgact atcaagaaga tggttcggta      60

cttctgactt gtgatgcaga agccaaaaat atcacatggt ttaaagatgg gaagatgatc     120

ggcttcctaa ctgaagataa aaaaaaatgg aatctgggaa gtaatgccaa ggacccacga     180

gggatgtatc agtgtaaagg atcacagaac aagtcaaaac cactccaagt gtattacaga     240

atgtgtcaga actgcattga actaaatgca gccaccatat ctggctttct ctttgctgaa     300

atcgtcagca ttttcgtcct tgctgttggg gtctacttca ttgctggaca ggatggagtt     360

cgccagtcga gagcttcaga caagcagact ctgttgccca atgaccagct ctaccagccc 420

ctcaaggatc gagaagatga ccagtacagc caccttcaag gaaaccagtt gaggaggaat 480

<210> 10
<211> 1311
<212> DNA
<213> Artificial sequence

<220>
<223> Synthesized polynucleotide

<400> 10
caggtgcagc tgcaggagag cggccccggc ctgatcaagc ccagccagac cctgagcctg 60

acctgcaccg tgagcggcgg cagcatcagc agcggctaca actggcactg gatccggcag 120

ccccccggca agggcctgga gtggatcggc tacatccact acaccggcag caccaactac 180

aaccccgccc tgcggagccg ggtgaccatc agcgtggaca ccagcaagaa ccagttcagc 240

ctgaagctga gcagcgtgac cgccgccgac accgccatct actactgcgc ccggatctac 300

aacggcaaca gcttccccta ctggggccag ggcaccaccg tgaccgtgag cagcggtgga 360

ggcggttcag gcggaggtgg ttctggcggt ggcggatcgg acatcgtgat gacccagagc 420

cccgacagcc tggccgtgag cctgggcgag cgggccacca tcaactgcaa gagcagccag 480

agcctgttca acagcggcaa ccagaagaac tacctgacct ggtaccagca gaagcccggc 540

cagcccccca agctgctgat ctactgggcc agcacccggg agagcggcgt gcccgaccgg 600

ttcagcggca gcggcagcgg caccgacttc accctgacca tcagcagcct gcaggccgag 660

gacgtggccg tgtactactg ccagaacgcc tacagcttcc cctacacctt cggcggcggc 720

accaagctgg agatcaagcg ggcggccgca attgaagtta tgtatcctcc tccttaccta 780

ggtggcggcg gttctggtgg cggcggttct ggtggcggcg gttctctcga gcagtcaatc 840

aaaggaaacc acttggttaa ggtgtatgac tatcaagaag atggttcggt acttctgact 900

tgtgatgcag aagccaaaaa tatcacatgg tttaaagatg ggaagatgat cggcttccta 960

actgaagata aaaaaaaatg gaatctggga agtaatgcca aggacccacg agggatgtat 1020

cagtgtaaag gatcacagaa caagtcaaaa ccactccaag tgtattacag aatgtgtcag 1080

aactgcattg aactaaatgc agccaccata tctggctttc tctttgctga aatcgtcagc 1140

attttcgtcc ttgctgttgg ggtctacttc attgctggac aggatggagt tcgccagtcg 1200

agagcttcag acaagcagac tctgttgccc aatgaccagc tctaccagcc cctcaaggat 1260

cgagaagatg accagtacag ccaccttcaa ggaaaccagt tgaggaggaa t 1311

<210> 11
<211> 20
<212> PRT
<213> Artificial sequence

<220>
<223>  Synthesized polypeptide

<400>  11

Ala Ala Ala Gly Gly Gly Gly Ser Gly Gly Gly Gly Ser Gly Gly Gly
1               5                   10                  15

Gly Ser Leu Glu
            20

<210>  12
<211>  60
<212>  DNA
<213>  Artificial sequence

<220>
<223>  Synthesized polynucleotide

<400>  12
gcggccgcag gtggcggcgg ttctggtggc ggcggttctg gtggcggcgg ttctctcgag        60

<210>  13
<211>  1356
<212>  DNA
<213>  Artificial sequence

<220>
<223>  Synthesized polynucleotide

<400>  13
caggtgcagc tgcaggagag cggccccggc ctgatcaagc ccagccagac cctgagcctg        60

acctgcaccg tgagcggcgg cagcatcagc agcggctaca actggcactg gatccggcag       120

ccccccggca agggcctgga gtggatcggc tacatccact acaccggcag caccaactac       180

aaccccgccc tgcggagccg ggtgaccatc agcgtggaca ccagcaagaa ccagttcagc       240

ctgaagctga gcagcgtgac cgccgccgac accgccatct actactgcgc ccggatctac       300

aacggcaaca gcttccccta ctggggccag ggcaccaccg tgaccgtgag cagcggtgga       360

ggcggttcag gcggaggtgg ttctggcggt ggcggatcgg acatcgtgat gacccagagc       420

cccgacagcc tggccgtgag cctgggcgag cgggccacca tcaactgcaa gagcagccag       480

agcctgttca acagcggcaa ccagaagaac tacctgacct ggtaccagca gaagcccggc       540

cagcccccca agctgctgat ctactgggcc agcacccggg agagcggcgt gcccgaccgg       600

ttcagcggca gcggcagcgg caccgacttc accctgacca tcagcagcct gcaggccgag       660

gacgtggccg tgtactactg ccagaacgcc tacagcttcc cctacacctt cggcggcggc       720

accaagctgg agatcaagcg ggcggccgca ggtggcggcg gttctggtgg cggcggttct       780

ggtggcggcg gttctctcga ggatggtaat gaagaaatgg gtggtattac acagacacca       840

tataaagtct ccatctctgg aaccacagta atattgacat gccctcagta tcctggatct 900

gaaatactat ggcaacacaa tgataaaaac ataggcggtg atgaggatga taaaaacata 960

ggcagtgatg aggatcacct gtcactgaag gaattttcag aattggagca aagtggttat 1020

tatgtctgct accccagagg aagcaaacca gaagatgcga acttttatct ctacctgagg 1080

gcaagagtgt gtgagaactg catggagatg gatgtgatgt cggtggccac aattgtcata 1140

gtggacatct gcatcactgg gggcttgctg ctgctggttt actactggag caagaataga 1200

aaggccaagg ccaagcctgt gacacgagga gcgggtgctg gcggcaggca aaggggacaa 1260

aacaaggaga ggccaccacc tgttcccaac ccagactatg agcccatccg gaaaggccag 1320

cgggacctgt attctggcct gaatcagaga cgcatc 1356

<210> 14
<211> 1281
<212> DNA
<213> Artificial sequence

<220>
<223> Synthesized polynucleotide

<400> 14
caggtgcagc tgcaggagag cggccccggc ctgatcaagc ccagccagac cctgagcctg 60

acctgcaccg tgagcggcgg cagcatcagc agcggctaca actggcactg gatccggcag 120

ccccccggca agggcctgga gtggatcggc tacatccact acaccggcag caccaactac 180

aaccccgccc tgcggagccg ggtgaccatc agcgtggaca ccagcaagaa ccagttcagc 240

ctgaagctga gcagcgtgac cgccgccgac accgccatct actactgcgc ccggatctac 300

aacggcaaca gcttccccta ctggggccag ggcaccaccg tgaccgtgag cagcggtgga 360

ggcggttcag gcggaggtgg ttctggcggt ggcggatcgg acatcgtgat gacccagagc 420

cccgacagcc tggccgtgag cctgggcgag cgggccacca tcaactgcaa gagcagccag 480

agcctgttca acagcggcaa ccagaagaac tacctgacct ggtaccagca gaagcccggc 540

cagcccccca agctgctgat ctactgggcc agcacccggg agagcggcgt gcccgaccgg 600

ttcagcggca gcggcagcgg caccgacttc accctgacca tcagcagcct gcaggccgag 660

gacgtggccg tgtactactg ccagaacgcc tacagcttcc cctacacctt cggcggcggc 720

accaagctgg agatcaagcg ggcggccgca ggtggcggcg gttctggtgg cggcggttct 780

ggtggcggcg gttctctcga gcagtcaatc aaaggaaacc acttggttaa ggtgtatgac 840

tatcaagaag atggttcggt acttctgact tgtgatgcag aagccaaaaa tatcacatgg 900

tttaaagatg ggaagatgat cggcttccta actgaagata aaaaaaaatg gaatctggga 960

agtaatgcca aggacccacg agggatgtat cagtgtaaag gatcacagaa caagtcaaaa 1020

ccactccaag tgtattacag aatgtgtcag aactgcattg aactaaatgc agccaccata 1080

tctggctttc tctttgctga aatcgtcagc attttcgtcc ttgctgttgg ggtctacttc     1140

attgctggac aggatggagt tcgccagtcg agagcttcag acaagcagac tctgttgccc     1200

aatgaccagc tctaccagcc cctcaaggat cgagaagatg accagtacag ccaccttcaa     1260

ggaaaccagt tgaggaggaa t                                               1281

<210>  15
<211>  6
<212>  PRT
<213>  Artificial sequence

<220>
<223>  Synthesized polypeptide

<400>  15

Ser Gly Tyr Asn Trp His
1               5

<210>  16
<211>  16
<212>  PRT
<213>  Artificial sequence

<220>
<223>  Synthesized polypeptide

<400>  16

Tyr Ile His Tyr Thr Gly Ser Thr Asn Tyr Asn Pro Ala Leu Arg Ser
1               5                   10                  15

<210>  17
<211>  9
<212>  PRT
<213>  Artificial sequence

<220>
<223>  Synthesized polypeptide

<400>  17

Ile Tyr Asn Gly Asn Ser Phe Pro Tyr
1               5

<210>  18
<211>  17
<212>  PRT
<213>  Artificial sequence

<220>
<223>  Synthesized polypeptide

<400>  18

Lys Ser Ser Gln Ser Leu Phe Asn Ser Gly Asn Gln Lys Asn Tyr Leu

1               5                   10                  15

Thr

<210>  19
<211>  7
<212>  PRT
<213>  Artificial sequence

<220>
<223>  Synthesized polypeptide

<400>  19

Trp Ala Ser Thr Arg Glu Ser
1               5

<210>  20
<211>  9
<212>  PRT
<213>  Artificial sequence

<220>
<223>  Synthesized polypeptide

<400>  20

Gln Asn Ala Tyr Ser Phe Pro Tyr Thr
1               5

<210>  21
<211>  118
<212>  PRT
<213>  Artificial sequence

<220>
<223>  Synthesized polypeptide

<400>  21

Gln Val Gln Leu Gln Glu Ser Gly Pro Gly Leu Ile Lys Pro Ser Gln
1               5                   10                  15

Thr Leu Ser Leu Thr Cys Thr Val Ser Gly Gly Ser Ile Ser Ser Gly
            20                  25                  30

Tyr Asn Trp His Trp Ile Arg Gln Pro Pro Gly Lys Gly Leu Glu Trp
        35                  40                  45

Ile Gly Tyr Ile His Tyr Thr Gly Ser Thr Asn Tyr Asn Pro Ala Leu
    50                  55                  60

Arg Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Ile Tyr Tyr Cys
                85                  90                  95

Ala Arg Ile Tyr Asn Gly Asn Ser Phe Pro Tyr Trp Gly Gln Gly Thr
            100                 105                 110

Thr Val Thr Val Ser Ser
        115

<210>  22
<211>  114
<212>  PRT
<213>  Artificial sequence

<220>
<223>  Synthesized polypeptide

<400>  22

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Phe Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Asn
                85                  90                  95

Ala Tyr Ser Phe Pro Tyr Thr Phe Gly Gly Gly Thr Lys Leu Glu Ile
            100                 105                 110

Lys Arg

<210>  23
<211>  243
<212>  PRT
<213>  Artificial sequence

<220>
<223>  Synthesized polypeptide

<400>  23

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg

<210> 24
<211> 729
<212> DNA
<213> Artificial sequence

<220>
<223> Synthesized polynucleotide

<400> 24
gaggtgcagc tggtgcagag cggcgccgag gtgaagaagc ccggcgccag cgtgaaggtg 60

agctgcaagg ccagcggcta caccttcagc gactacgaga tgcactgggt gcggcaggcc 120

cccggccagg gcctggagtg gatgggcgcc atccaccccg gcagcggcga caccgcctac 180

aaccagcggt tcaagggccg ggtgaccatc accgccgaca agagcaccag caccgcctac 240

atggagctga gcagcctgcg gagcgaggac accgccgtgt actactgcgc ccggttctac 300

agctacgcct actggggcca gggcaccctg gtgaccgtga gcgccggtgg aggcggttca 360

ggcggaggtg gttctggcgg tggcggatcg gacatcgtga tgacccagac ccccctgagc 420

ctgcccgtga cccccggcga gcccgccagc atcagctgcc ggagcagcca gagcctggtg 480

cacagcaacg gcaacaccta cctgcagtgg tacctgcaga agcccggcca gagcccccag 540

ctgctgatct acaaggtgag caaccggttc agcggcgtgc ccgaccggtt cagcggcagc 600

ggcagcggca ccgacttcac cctgaagatc agccgggtgg aggccgagga cgtgggcgtg 660

tactactgca gccagagcat ctacgtgccc tacaccttcg gccagggcac caagctggag 720

atcaaacgt 729

<210> 25
<211> 1356
<212> DNA
<213> Artificial sequence

<220>
<223> Synthesized polynucleotide

<400> 25
gaggtgcagc tggtgcagag cggcgccgag gtgaagaagc ccggcgccag cgtgaaggtg 60

agctgcaagg ccagcggcta caccttcagc gactacgaga tgcactgggt gcggcaggcc 120

cccggccagg gcctggagtg gatgggcgcc atccaccccg gcagcggcga caccgcctac 180

aaccagcggt tcaagggccg ggtgaccatc accgccgaca agagcaccag caccgcctac 240

atggagctga gcagcctgcg gagcgaggac accgccgtgt actactgcgc ccggttctac 300

agctacgcct actggggcca gggcaccctg gtgaccgtga gcgccggtgg aggcggttca 360

ggcggaggtg gttctggcgg tggcggatcg gacatcgtga tgacccagac ccccctgagc 420

ctgcccgtga cccccggcga gcccgccagc atcagctgcc ggagcagcca gagcctggtg 480

cacagcaacg gcaacaccta cctgcagtgg tacctgcaga agcccggcca gagcccccag 540

ctgctgatct acaaggtgag caaccggttc agcggcgtgc ccgaccggtt cagcggcagc 600

ggcagcggca ccgacttcac cctgaagatc agccgggtgg aggccgagga cgtgggcgtg 660

tactactgca gccagagcat ctacgtgccc tacaccttcg gccagggcac caagctggag 720

atcaaacgtg cggccgcagg tggcggcggt tctggtggcg gcggttctgg tggcggcggt 780

tctctcgaga tgcggtggaa cactttctgg ggcatcctgt gcctcagcct cctagctgtt 840

ggcacttgcc aggacgatgc cgagaacatt gaatacaaag tctccatctc aggaaccagt 900

gtagagttga cgtgccctct agacagtgac gagaacttaa aatgggaaaa aaatggccaa 960

gagctgcctc agaagcatga taagcacctg gtgctccagg atttctcgga agtcgaggac 1020

agtggctact acgtctgcta cacaccagcc tcaaataaaa acacgtactt gtacctgaaa 1080

gctcgagtgt gtgagtactg tgtggaggtg gacctgacag cagtagccat aatcatcatt 1140

gttgacatct gtatcactct gggcttgctg atggtcattt attactggag caagaatagg 1200

aaggccaagg ccaagcctgt gacccgagga accggtgctg gtagcaggcc cagagggcaa 1260

aacaaggagc ggccaccacc tgttcccaac ccagactatg agcccatccg caaaggccag 1320

cgggacctgt attctggcct gaatcagaga gcagtc 1356

<210> 26
<211> 5
<212> PRT
<213> Artificial sequence

<220>
<223> Synthesized polypeptide

<400> 26

Asp Tyr Glu Met His
1               5

<210> 27
<211> 17
<212> PRT
<213> Artificial sequence

<220>
<223> Synthesized polypeptide

<400> 27

Ala Ile His Pro Gly Ser Gly Asp Thr Ala Tyr Asn Gln Arg Phe Lys
1               5                   10                  15

Gly

<210>  28
<211>  6
<212>  PRT
<213>  Artificial sequence

<220>
<223>  Synthesized polypeptide

<400>  28

Phe Tyr Ser Tyr Ala Tyr
1               5


<210>  29
<211>  16
<212>  PRT
<213>  Artificial sequence

<220>
<223>  Synthesized polypeptide

<400>  29

Arg Ser Ser Gln Ser Leu Val His Ser Asn Gly Asn Thr Tyr Leu Gln
1               5                   10                  15


<210>  30
<211>  7
<212>  PRT
<213>  Artificial sequence

<220>
<223>  Synthesized polypeptide

<400>  30

Lys Val Ser Asn Arg Phe Ser
1               5


<210>  31
<211>  9
<212>  PRT
<213>  Artificial sequence

<220>
<223>  Synthesized polypeptide

<400>  31

Ser Gln Ser Ile Tyr Val Pro Tyr Thr
1               5


<210>  32
<211>  115
<212>  PRT
<213>  Artificial sequence

<220>
<223>  Synthesized polypeptide

<400>  32

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala
        115


<210>  33
<211>  113
<212>  PRT
<213>  Artificial sequence

<220>
<223>  Synthesized polypeptide

<400>  33

Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu Gln Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Ile Tyr Val Pro Tyr Thr Phe Gly Gln Gly Thr Lys Leu Glu Ile Lys
            100                 105                 110

Arg

<210>  34
<211>  189
<212>  PRT
<213>  Artificial sequence

<220>
<223>  Synthesized polypeptide

<400>  34

Met Arg Trp Asn Thr Phe Trp Gly Ile Leu Cys Leu Ser Leu Leu Ala
1               5                   10                  15

Val Gly Thr Cys Gln Asp Asp Ala Glu Asn Ile Glu Tyr Lys Val Ser
            20                  25                  30

Ile Ser Gly Thr Ser Val Glu Leu Thr Cys Pro Leu Asp Ser Asp Glu
        35                  40                  45

Asn Leu Lys Trp Glu Lys Asn Gly Gln Glu Leu Pro Gln Lys His Asp
    50                  55                  60

Lys His Leu Val Leu Gln Asp Phe Ser Glu Val Glu Asp Ser Gly Tyr
65                  70                  75                  80

Tyr Val Cys Tyr Thr Pro Ala Ser Asn Lys Asn Thr Tyr Leu Tyr Leu
                85                  90                  95

Lys Ala Arg Val Cys Glu Tyr Cys Val Glu Val Asp Leu Thr Ala Val
            100                 105                 110

Ala Ile Ile Ile Ile Val Asp Ile Cys Ile Thr Leu Gly Leu Leu Met
        115                 120                 125

Val Ile Tyr Tyr Trp Ser Lys Asn Arg Lys Ala Lys Ala Lys Pro Val
    130                 135                 140

Thr Arg Gly Thr Gly Ala Gly Ser Arg Pro Arg Gly Gln Asn Lys Glu

145                 150                 155                 160

Arg Pro Pro Pro Val Pro Asn Pro Asp Tyr Glu Pro Ile Arg Lys Gly
                165                 170                 175

Gln Arg Asp Leu Tyr Ser Gly Leu Asn Gln Arg Ala Val
            180                 185

<210>  35
<211>  567
<212>  DNA
<213>  Artificial sequence

<220>
<223>  Synthesized polynucleotide

<400>  35
atgcggtgga acactttctg gggcatcctg tgcctcagcc tcctagctgt tggcacttgc      60

caggacgatg ccgagaacat tgaatacaaa gtctccatct caggaaccag tgtagagttg     120

acgtgccctc tagacagtga cgagaactta aaatgggaaa aaaatggcca agagctgcct     180

cagaagcatg ataagcacct ggtgctccag gatttctcgg aagtcgagga cagtggctac     240

tacgtctgct acacaccagc ctcaaataaa aacacgtact tgtacctgaa agctcgagtg     300

tgtgagtact gtgtggaggt ggacctgaca gcagtagcca taatcatcat tgttgacatc     360

tgtatcactc tgggcttgct gatggtcatt tattactgga gcaagaatag gaaggccaag     420

gccaagcctg tgacccgagg aaccggtgct ggtagcaggc ccagagggca aaacaaggag     480

cggccaccac ctgttcccaa cccagactat gagcccatcc gcaaaggcca gcgggacctg     540

tattctggcc tgaatcagag agcagtc                                         567

<210>  36
<211>  22
<212>  PRT
<213>  Artificial sequence

<220>
<223>  Synthesized polypeptide

<400>  36

Val Lys Gln Thr Leu Asn Phe Asp Leu Leu Lys Leu Ala Gly Asp Val
1               5                   10                  15

Glu Ser Asn Pro Gly Pro
            20

<210>  37
<211>  66
<212>  DNA
<213>  Artificial sequence

<220>
<223>  Synthesized polynucleotide

<400>  37
gtgaaacaga ctttgaattt tgaccttctg aagttggcag gagacgttga gtccaaccct      60

gggccc                                                                  66


<210>  38
<211>  133
<212>  PRT
<213>  Artificial sequence

<220>
<223>  Synthesized polypeptide

<400>  38

Met Ala Gln Met Met Thr Leu Ser Leu Leu Ser Leu Val Leu Ala Leu
1               5                   10                  15

Cys Ile Pro Trp Thr Gln Gly Ser Asp Gly Gly Gly Gln Asp Cys Cys
            20                  25                  30

Leu Lys Tyr Ser Gln Lys Lys Ile Pro Tyr Ser Ile Val Arg Gly Tyr
        35                  40                  45

Arg Lys Gln Glu Pro Ser Leu Gly Cys Pro Ile Pro Ala Ile Leu Phe
    50                  55                  60

Leu Pro Arg Lys His Ser Lys Pro Glu Leu Cys Ala Asn Pro Glu Glu
65                  70                  75                  80

Gly Trp Val Gln Asn Leu Met Arg Arg Leu Asp Gln Pro Pro Ala Pro
                85                  90                  95

Gly Lys Gln Ser Pro Gly Cys Arg Lys Asn Arg Gly Thr Ser Lys Ser
            100                 105                 110

Gly Lys Lys Gly Lys Gly Ser Lys Gly Cys Lys Arg Thr Glu Gln Thr
        115                 120                 125

Gln Pro Ser Arg Gly
    130


<210>  39
<211>  399
<212>  DNA
<213>  Artificial sequence

<220>
<223>  Synthesized polynucleotide

<400>  39
atggctcaga tgatgactct gagcctcctt agcctggtcc tggctctctg catcccctgg      60

acccaaggca gtgatggagg gggacaggac tgctgcctta agtacagcca gaagaaaatt     120

ccctacagta ttgtccgagg ctataggaag caagaaccaa gtttaggctg tcccatcccg     180

gcaatcctgt tcttaccccg gaagcactct aagcctgagc tatgtgcaaa ccctgaggaa     240

ggctgggtgc agaacctgat gcgccgcctg gaccagcctc cagccccagg gaaacaaagc     300

cccggctgca ggaagaaccg gggaacctct aagtctggaa agaaaggaaa gggctccaag     360

ggctgcaaga gaactgaaca gacacagccc tcaagagga                            399

<210>  40
<211>  154
<212>  PRT
<213>  Artificial sequence

<220>
<223>  Synthesized polypeptide

<400>  40

Met Phe His Val Ser Phe Arg Tyr Ile Phe Gly Ile Pro Pro Leu Ile
1               5                   10                  15

Leu Val Leu Leu Pro Val Thr Ser Ser Glu Cys His Ile Lys Asp Lys
            20                  25                  30

Glu Gly Lys Ala Tyr Glu Ser Val Leu Met Ile Ser Ile Asp Glu Leu
        35                  40                  45

Asp Lys Met Thr Gly Thr Asp Ser Asn Cys Pro Asn Asn Glu Pro Asn
    50                  55                  60

Phe Phe Arg Lys His Val Cys Asp Asp Thr Lys Glu Ala Ala Phe Leu
65                  70                  75                  80

Asn Arg Ala Ala Arg Lys Leu Lys Gln Phe Leu Lys Met Asn Ile Ser
                85                  90                  95

Glu Glu Phe Asn Val His Leu Leu Thr Val Ser Gln Gly Thr Gln Thr
            100                 105                 110

Leu Val Asn Cys Thr Ser Lys Glu Glu Lys Asn Val Lys Glu Gln Lys
        115                 120                 125

Lys Asn Asp Ala Cys Phe Leu Lys Arg Leu Leu Arg Glu Ile Lys Thr
    130                 135                 140

Cys Trp Asn Lys Ile Leu Lys Gly Ser Ile
145                 150

<210>  41
<211>  462
<212>  DNA
<213>  Artificial sequence

<220>
<223>  Synthesized polynucleotide

<400>  41
atgttccatg tttcttttag atatatcttt ggaattcctc cactgatcct tgttctgctg        60

cctgtcacat catctgagtg ccacattaaa gacaaagaag gtaaagcata tgagagtgta       120

ctgatgatca gcatcgatga attggacaaa atgacaggaa ctgatagtaa ttgcccgaat       180

aatgaaccaa acttttttag aaaacatgta tgtgatgata caaaggaagc tgcttttcta       240

aatcgtgctg ctcgcaagtt gaagcaattt cttaaaatga atatcagtga agaattcaat       300

gtccacttac taacagtatc acaaggcaca caaacactgg tgaactgcac aagtaaggaa       360

gaaaaaaacg taaaggaaca gaaaaagaat gatgcatgtt tcctaaagag actactgaga       420

gaaataaaaa cttgttggaa taaaattttg aagggcagta ta                          462

<210>  42
<211>  19
<212>  PRT
<213>  Artificial sequence

<220>
<223>  Synthesized polypeptide

<400>  42

Ala Thr Asn Phe Ser Leu Leu Lys Gln Ala Gly Asp Val Glu Glu Asn
1               5                   10                  15

Pro Gly Pro

<210>  43
<211>  57
<212>  DNA
<213>  Artificial sequence

<220>
<223>  Synthesized polynucleotide

<400>  43
gctactaact tcagcctgct gaagcaggct ggagacgtgg aggagaaccc tggacct          57

<210>  44
<211>  62
<212>  PRT

<213>  Artificial sequence

<220>
<223>  Synthesized polypeptide

<400>  44

Lys Arg Lys Ile Cys Phe Ile Gln Lys Ala Leu Arg Gly Lys Phe Val
1               5                   10                  15

Ser Tyr Arg Arg Arg Glu Glu Asn Leu Phe His Thr Glu Gly Val Lys
            20                  25                  30

Arg Lys Ile Cys Phe Ile Gln Lys Ala Leu Arg Gly Lys Phe Val Ser
        35                  40                  45

Tyr Arg Arg Arg Glu Glu Asn Leu Phe His Thr Glu Gly Val
    50                  55                  60

<210>  45
<211>  192
<212>  DNA
<213>  Artificial sequence

<220>
<223>  Synthesized polynucleotide

<400>  45
aagaggaaaa tttgtttcat acagaaggcg ttaagaggaa aatttgtttc atacagaagg      60

cgttaagagg aaaatttgtt tcatacagaa ggcgttaaga ggaaaatttg tttcatacag     120

aaggcgttaa gaggaaaatt tgtttcatac agaaggcgtt aagaggaaaa tttgtttcat     180

acagaaggcg tt                                                         192

<210>  46
<211>  36
<212>  PRT
<213>  Artificial sequence

<220>
<223>  Synthesized polypeptide

<400>  46

Asn Ile Val Thr Pro Pro Tyr Tyr Phe Ser Ser Ile Asn Ser Ile Asn
1               5                   10                  15

Cys Leu Pro Cys Arg Ala Ala Tyr His Pro Cys Ser Leu Leu Thr Val
            20                  25                  30

Thr Ser Ser Pro
        35

<210>  47
<211>  114
<212>  DNA
<213>  Artificial sequence

<220>
<223>  Synthesized polynucleotide

<400>  47
aacatcgtga cacccccata ttatttttcc agcattaaca gtataaattg cctcccatgc        60

tgaagagctg cctatcaccc ttgctaatca ctcctcacag tgacctcaag tcct              114


<210>  48
<211>  542
<212>  PRT
<213>  Artificial sequence

<220>
<223>  Synthesized polypeptide

<400>  48

Met Trp Glu Leu Glu Lys Asp Val Tyr Val Val Glu Val Asp Trp Thr
1               5                   10                  15

Pro Asp Ala Pro Gly Glu Thr Val Asn Leu Thr Cys Asp Thr Pro Glu
            20                  25                  30

Glu Asp Asp Ile Thr Trp Thr Ser Asp Gln Arg His Gly Val Ile Gly
        35                  40                  45

Ser Gly Lys Thr Leu Thr Ile Thr Val Lys Glu Phe Leu Asp Ala Gly
    50                  55                  60

Gln Tyr Thr Cys His Lys Gly Gly Glu Thr Leu Ser His Ser His Leu
65                  70                  75                  80

Leu Leu His Lys Lys Glu Asn Gly Ile Trp Ser Thr Glu Ile Leu Lys
                85                  90                  95

Asn Phe Lys Asn Lys Thr Phe Leu Lys Cys Glu Ala Pro Asn Tyr Ser
            100                 105                 110

Gly Arg Phe Thr Cys Ser Trp Leu Val Gln Arg Asn Met Asp Leu Lys
        115                 120                 125

Phe Asn Ile Lys Ser Ser Ser Ser Ser Pro Asp Ser Arg Ala Val Thr
    130                 135                 140

Cys Gly Met Ala Ser Leu Ser Ala Glu Lys Val Thr Leu Asp Gln Arg
145                 150                 155                 160

Asp Tyr Glu Lys Tyr Ser Val Ser Cys Gln Glu Asp Val Thr Cys Pro
                165                 170                 175

Thr Ala Glu Glu Thr Leu Pro Ile Glu Leu Ala Leu Glu Ala Arg Gln
            180                 185                 190

Gln Asn Lys Tyr Glu Asn Tyr Ser Thr Ser Phe Phe Ile Arg Asp Ile
        195                 200                 205

Ile Lys Pro Asp Pro Pro Lys Asn Leu Gln Met Lys Pro Leu Lys Asn
    210                 215                 220

Ser Gln Val Glu Val Ser Trp Glu Tyr Pro Asp Ser Trp Ser Thr Pro
225                 230                 235                 240

His Ser Tyr Phe Ser Leu Lys Phe Phe Val Arg Ile Gln Arg Lys Lys
                245                 250                 255

Glu Lys Met Lys Glu Thr Glu Glu Gly Cys Asn Gln Lys Gly Ala Phe
            260                 265                 270

Leu Val Glu Lys Thr Ser Thr Glu Val Gln Cys Lys Gly Gly Asn Val
        275                 280                 285

Cys Val Gln Ala Gln Asp Arg Tyr Tyr Asn Ser Ser Cys Ser Lys Trp
    290                 295                 300

Ala Cys Val Pro Cys Arg Val Arg Ser Gly Gly Gly Gly Ser Gly Gly
305                 310                 315                 320

Gly Gly Ser Gly Gly Gly Gly Ser Cys Gln Ser Arg Tyr Leu Leu Phe
                325                 330                 335

Leu Ala Thr Leu Ala Leu Leu Asn His Leu Ser Leu Ala Arg Val Ile
            340                 345                 350

Pro Val Ser Gly Pro Ala Arg Cys Leu Ser Gln Ser Arg Asn Leu Leu
        355                 360                 365

Lys Thr Thr Asp Asp Met Val Lys Thr Ala Arg Glu Lys Leu Lys His
    370                 375                 380

Tyr Ser Cys Thr Ala Glu Asp Ile Asp His Glu Asp Ile Thr Arg Asp
385                 390                 395                 400

Gln Thr Ser Thr Leu Lys Thr Cys Leu Pro Leu Glu Leu His Lys Asn
                405                 410                 415

Glu Ser Cys Leu Ala Thr Arg Glu Thr Ser Ser Thr Thr Arg Gly Ser
            420                 425                 430

Cys Leu Pro Pro Gln Lys Thr Ser Leu Met Met Thr Leu Cys Leu Gly
        435                 440                 445

Ser Ile Tyr Glu Asp Leu Lys Met Tyr Gln Thr Glu Phe Gln Ala Ile
    450                 455                 460

Asn Ala Ala Leu Gln Asn His Asn His Gln Gln Ile Ile Leu Asp Lys
465                 470                 475                 480

Gly Met Leu Val Ala Ile Asp Glu Leu Met Gln Ser Leu Asn His Asn
                485                 490                 495

Gly Glu Thr Leu Arg Gln Lys Pro Pro Val Gly Glu Ala Asp Pro Tyr
            500                 505                 510

Arg Val Lys Met Lys Leu Cys Ile Leu Leu His Ala Phe Ser Thr Arg
        515                 520                 525

Val Val Thr Ile Asn Arg Val Met Gly Tyr Leu Ser Ser Ala
    530                 535                 540

<210>  49
<211>  1626
<212>  DNA
<213>  Artificial sequence

<220>
<223>  Synthesized polynucleotide

<400>  49
atgtgggagc tggagaaaga cgtttatgtt gtagaggtgg actggactcc cgatgcccct      60

ggagaaacag tgaacctcac ctgtgacacg cctgaagaag atgacatcac ctggacctca     120

gaccagagac atggagtcat aggctctgga aagaccctga ccatcactgt caaagagttt     180

ctagatgctg gccagtacac ctgccacaaa ggaggcgaga ctctgagcca ctcacatctg     240

ctgctccaca agaaggaaaa tggaatttgg tccactgaaa ttttaaaaaa tttcaaaaac     300

aagactttcc tgaagtgtga agcaccaaat tactccggac ggttcacgtg ctcatggctg     360

gtgcaaagaa acatggactt gaagttcaac atcaagagca gtagcagttc ccctgactct     420

cgggcagtga catgtggaat ggcgtctctg tctgcagaga aggtcacact ggaccaaagg     480

gactatgaga agtattcagt gtcctgccag gaggatgtca cctgcccaac tgccgaggag     540

accctgccca ttgaactggc gttggaagca cggcagcaga ataaatatga gaactacagc     600

accagcttct tcatcaggga catcatcaaa ccagacccgc ccaagaactt gcagatgaag 660

cctttgaaga actcacaggt ggaggtcagc tgggagtacc ctgactcctg gagcactccc 720

cattcctact tctccctcaa gttctttgtt cgaatccagc gcaagaaaga aaagatgaag 780

gagacagagg aggggtgtaa ccagaaaggt gcgttcctcg tagagaagac atctaccgaa 840

gtccaatgca aaggcgggaa tgtctgcgtg caagctcagg atcgctatta caattcctca 900

tgcagcaagt gggcatgtgt tccctgcagg gtccgatccg gtggcggtgg ctcgggcggt 960

ggtgggtcgg gtggcggcgg atcttgtcaa tcacgctacc tcctcttttt ggccaccctt 1020

gccctcctaa accacctcag tttggccagg gtcattccag tctctggacc tgccaggtgt 1080

cttagccagt cccgaaacct gctgaagacc acagatgaca tggtgaagac ggccagagaa 1140

aaactgaaac attattcctg cactgctgaa gacatcgatc atgaagacat cacacgggac 1200

caaaccagca cattgaagac ctgtttacca ctggaactac acaagaacga gagttgcctg 1260

gctactagag agacttcttc cacaacaaga gggagctgcc tgcccccaca gaagacgtct 1320

ttgatgatga ccctgtgcct tggtagcatc tatgaggact tgaagatgta ccagacagag 1380

ttccaggcca tcaacgcagc acttcagaat cacaaccatc agcagatcat tctagacaag 1440

ggcatgctgg tggccatcga tgagctgatg cagtctctga atcataatgg cgagactctg 1500

cgccagaaac ctcctgtggg agaagcagac ccttacagag tgaaaatgaa gctctgcatc 1560

ctgcttcacg ccttcagcac ccgcgtcgtg accatcaaca gggtgatggg ctatctgagc 1620

tccgcc 1626

<210> 50
<211> 409
<212> PRT
<213> Artificial sequence

<220>
<223> Synthesized polypeptide

<400> 50

Met Arg Ile Pro Val Asp Pro Ser Thr Ser Arg Arg Phe Thr Pro Pro
1               5                   10                  15

Ser Thr Ala Phe Pro Cys Gly Gly Gly Gly Gly Gly Lys Met Gly Glu
            20                  25                  30

Asn Ser Gly Ala Leu Ser Ala Gln Ala Thr Ala Gly Pro Gly Gly Arg
        35                  40                  45

Thr Arg Pro Glu Val Arg Ser Met Val Asp Val Leu Ala Asp His Ala
    50                  55                  60

Gly Glu Leu Val Arg Thr Asp Ser Pro Asn Phe Leu Cys Ser Val Leu
65                  70                  75                  80

Pro Ser His Trp Arg Cys Asn Lys Thr Leu Pro Val Ala Phe Lys Val
                85                  90                  95

Val Ala Leu Gly Asp Val Pro Asp Gly Thr Val Val Thr Val Met Ala
            100                 105                 110

Gly Asn Asp Glu Asn Tyr Ser Ala Glu Leu Arg Asn Ala Ser Ala Val
        115                 120                 125

Met Lys Asn Gln Val Ala Arg Phe Asn Asp Leu Arg Phe Val Gly Arg
    130                 135                 140

Ser Gly Arg Gly Lys Ser Phe Thr Leu Thr Ile Thr Val Phe Thr Asn
145                 150                 155                 160

Pro Thr Gln Val Ala Thr Tyr His Arg Ala Ile Lys Val Thr Val Asp
                165                 170                 175

Gly Pro Arg Glu Pro Arg Arg His Arg Gln Lys Ile Glu Asp Gln Thr
            180                 185                 190

Lys Ala Phe Pro Asp Arg Phe Gly Asp Leu Arg Met Arg Val Thr Pro
        195                 200                 205

Ser Thr Pro Ser Pro Arg Gly Ser Leu Ser Thr Thr Ser His Phe Ser
    210                 215                 220

Ser Gln Ala Gln Thr Pro Ile Gln Gly Ser Ser Asp Leu Asn Pro Phe
225                 230                 235                 240

Ser Asp Pro Arg Gln Phe Asp Arg Ser Phe Pro Thr Leu Gln Ser Leu
                245                 250                 255

Thr Glu Ser Arg Phe Pro Asp Pro Arg Met His Tyr Pro Gly Ala Met
            260                 265                 270

Ser Ala Ala Phe Pro Tyr Ser Ala Thr Pro Ser Gly Thr Ser Leu Gly
        275                 280                 285

Ser Leu Ser Val Ala Gly Met Pro Ala Ser Ser Arg Phe His His Thr
    290                 295                 300

Tyr Leu Pro Pro Pro Tyr Pro Gly Ala Pro Gln Ser Gln Ser Gly Pro
305                 310                 315                 320

Phe Gln Ala Asn Pro Ala Pro Tyr His Leu Phe Tyr Gly Ala Ser Ser
                325                 330                 335

Gly Ser Tyr Gln Phe Ser Met Ala Ala Ala Gly Gly Gly Glu Arg Ser
            340                 345                 350

Pro Thr Arg Met Leu Thr Ser Cys Pro Ser Gly Ala Ser Val Ser Ala
        355                 360                 365

Gly Asn Leu Met Asn Pro Ser Leu Gly Gln Ala Asp Gly Val Glu Ala
    370                 375                 380

Asp Gly Ser His Ser Asn Ser Pro Thr Ala Leu Ser Thr Pro Gly Arg
385                 390                 395                 400

Met Asp Glu Ala Val Trp Arg Pro Tyr
                405

<210>  51
<211>  1227
<212>  DNA
<213>  Artificial sequence

<220>
<223>  Synthesized polynucleotide

<400>  51
atgcgtattc ccgtagaccc gagcaccagc cgccgcttca ctcccccctc cacggccttc      60

ccctgcggcg gcggcggcgg cggcaagatg ggcgagaaca gcggcgcgct aagcgcgcag     120

gcaaccgcgg gccccggcgg ccgcacccgg cccgaagtgc gctcgatggt ggacgtgctg     180

gccgaccacg cgggagagct cgtgcgcacc gacagcccca acttcctctg ctccgtgctg     240

ccctcgcact ggcgctgcaa caagacgctg ccggtcgcct tcaaggtggt ggccctgggg     300

gatgtgccgg atggaacggt ggtgaccgtg atggccggca atgatgagaa ctactccgcc     360

gagctgcgca acgcttccgc tgtcatgaag aaccaagtgg ccaggttcaa cgaccttcga     420

ttcgtgggcc gcagtgggcg agggaagagt ttcacgctca caatcaccgt gttcaccaac     480

cctacccaag tggctaccta ccaccgagcc atcaaggtca ctgtggatgg accccgggaa     540

ccccgacggc accggcagaa gatagaagac cagaccaagg ccttccccga ccgctttgga     600

gacctgcgca tgcgtgtaac accaagcaca cccagccccc gtggctctct cagcaccacg     660

agccacttca gcagccaggc ccagacccca atccaaggct cctcagacct gaaccccttc     720

tccgaccccc gccagtttga ccgctccttc cctacgctgc agagcctcac agagagccgc     780

ttcccggacc ccaggatgca ctacccggga gccatgtctg ccgccttccc ctacagcgcc     840

acaccatcgg gcaccagcct gggcagcctg agcgtggcgg gcatgccggc cagcagccgc     900

ttccaccaca cctacctccc tccgccctac cccggggccc cacagagcca gagcgggccc 960

tttcaggcca accccgcgcc ctaccacctc ttttacggcg cctcctccgg ctcctaccag 1020

ttctccatgg cagccgcggg aggtggtgag cgctcgccca cccgcatgct gacctcctgc 1080

cccagcggcg cttcggtgtc agcaggcaac ctcatgaacc ccagcctggg ccaggctgat 1140

ggcgtggaag ccgacggcag ccacagcaac tcgcccacgg ccctgagcac gccgggccgc 1200

atggacgagg ccgtgtggcg gccctac 1227

<210> 52
<211> 17
<212> PRT
<213> Artificial sequence

<220>
<223> Synthesized polypeptide

<400> 52

Asn Lys Ile Ser Leu Phe Ser Leu His Leu Cys Val Gly Phe Leu Cys
1               5                   10                  15

Glu

<210> 53
<211> 51
<212> DNA
<213> Artificial sequence

<220>
<223> Synthesized polynucleotide

<400> 53
aataaaatat ctttattttc attacatctg tgtgttggtt ttttgtgtga g 51

<210> 54
<211> 261
<212> PRT
<213> Artificial sequence

<220>
<223> Synthesized polypeptide

<400> 54

Met Ala Val Thr Ala Cys Gln Gly Leu Gly Phe Val Val Ser Leu Ile
1               5                   10                  15

Gly Ile Ala Gly Ile Ile Ala Ala Thr Cys Met Asp Gln Trp Ser Thr
            20                  25                  30

Gln Asp Leu Tyr Asn Asn Pro Val Thr Ala Val Phe Asn Tyr Gln Gly

35                  40                  45

Leu Trp Arg Ser Cys Val Arg Glu Ser Ser Gly Phe Thr Glu Cys Arg
    50                  55                  60

Gly Tyr Phe Thr Leu Leu Gly Leu Pro Ala Met Leu Gln Ala Val Arg
65                  70                  75                  80

Ala Leu Met Ile Val Gly Ile Val Leu Gly Ala Ile Gly Leu Leu Val
                85                  90                  95

Ser Ile Phe Ala Leu Lys Cys Ile Arg Ile Gly Ser Met Glu Asp Ser
            100                 105                 110

Ala Lys Ala Asn Met Thr Leu Thr Ser Gly Ile Met Phe Ile Val Ser
        115                 120                 125

Gly Leu Cys Ala Ile Ala Gly Val Ser Val Phe Ala Asn Met Leu Val
    130                 135                 140

Thr Asn Phe Trp Met Ser Thr Ala Asn Met Tyr Thr Gly Met Gly Gly
145                 150                 155                 160

Met Val Gln Thr Val Gln Thr Arg Tyr Thr Phe Gly Ala Ala Leu Phe
                165                 170                 175

Val Gly Trp Val Ala Gly Gly Leu Thr Leu Ile Gly Gly Val Met Met
            180                 185                 190

Cys Ile Ala Cys Arg Gly Leu Ala Pro Glu Glu Thr Asn Tyr Lys Ala
        195                 200                 205

Val Ser Tyr His Ala Ser Gly His Ser Val Ala Tyr Lys Pro Gly Gly
    210                 215                 220

Phe Lys Ala Ser Thr Gly Phe Gly Ser Asn Thr Lys Asn Lys Lys Ile
225                 230                 235                 240

Tyr Asp Gly Gly Ala Arg Thr Glu Asp Glu Val Gln Ser Tyr Pro Ser
                245                 250                 255

Lys His Asp Tyr Val
            260

<210>  55
<211>  579
<212>  PRT
<213>  Artificial sequence

<220>
<223>  Synthesized polypeptide

<400>  55

Met Ala Gly Thr Val Arg Thr Ala Cys Leu Leu Val Ala Met Leu Leu
1               5                   10                  15

Gly Leu Gly Cys Leu Gly Gln Ala Gln Pro Pro Pro Pro Pro Asp Ala
            20                  25                  30

Thr Cys His Gln Val Arg Ser Phe Phe Gln Arg Leu Gln Pro Gly Leu
        35                  40                  45

Lys Trp Val Pro Glu Thr Pro Val Pro Gly Ser Asp Leu Gln Val Cys
    50                  55                  60

Leu Pro Lys Gly Pro Thr Cys Cys Ser Arg Lys Met Glu Glu Lys Tyr
65                  70                  75                  80

Gln Leu Thr Ala Arg Leu Asn Met Glu Gln Leu Leu Gln Ser Ala Ser
                85                  90                  95

Met Glu Leu Lys Phe Leu Ile Ile Gln Asn Ala Ala Val Phe Gln Glu
            100                 105                 110

Ala Phe Glu Ile Val Val Arg His Ala Lys Asn Tyr Thr Asn Ala Met
        115                 120                 125

Phe Lys Asn Asn Tyr Pro Ser Leu Thr Pro Gln Ala Phe Glu Phe Val
    130                 135                 140

Gly Glu Phe Phe Thr Asp Val Ser Leu Tyr Ile Leu Gly Ser Asp Ile
145                 150                 155                 160

Asn Val Asp Asp Met Val Asn Glu Leu Phe Asp Ser Leu Phe Pro Val
                165                 170                 175

Ile Tyr Thr Gln Met Met Asn Pro Gly Leu Pro Glu Ser Val Leu Asp
            180                 185                 190

Ile Asn Glu Cys Leu Arg Gly Ala Arg Arg Asp Leu Lys Val Phe Gly
        195                 200                 205

Ser Phe Pro Lys Leu Ile Met Thr Gln Val Ser Lys Ser Leu Gln Val
    210                 215                 220

Thr Arg Ile Phe Leu Gln Ala Leu Asn Leu Gly Ile Glu Val Ile Asn

225                 230                 235                 240

Thr Thr Asp His Leu Lys Phe Ser Lys Asp Cys Gly Arg Met Leu Thr
                245                 250                 255

Arg Met Trp Tyr Cys Ser Tyr Cys Gln Gly Leu Met Met Val Lys Pro
            260                 265                 270

Cys Gly Gly Tyr Cys Asn Val Val Met Gln Gly Cys Met Ala Gly Val
        275                 280                 285

Val Glu Ile Asp Lys Tyr Trp Arg Glu Tyr Ile Leu Ser Leu Glu Glu
    290                 295                 300

Leu Val Asn Gly Met Tyr Arg Ile Tyr Asp Met Glu Asn Val Leu Leu
305                 310                 315                 320

Gly Leu Phe Ser Thr Ile His Asp Ser Ile Gln Tyr Val Gln Lys Asn
                325                 330                 335

Gly Gly Lys Leu Thr Thr Thr Ile Gly Lys Leu Cys Ala His Ser Gln
            340                 345                 350

Gln Arg Gln Tyr Arg Ser Ala Tyr Tyr Pro Glu Asp Leu Phe Ile Asp
        355                 360                 365

Lys Lys Ile Leu Lys Val Ala His Val Glu His Glu Glu Thr Leu Ser
    370                 375                 380

Ser Arg Arg Arg Glu Leu Ile Gln Lys Leu Lys Ser Phe Ile Asn Phe
385                 390                 395                 400

Tyr Ser Ala Leu Pro Gly Tyr Ile Cys Ser His Ser Pro Val Ala Glu
                405                 410                 415

Asn Asp Thr Leu Cys Trp Asn Gly Gln Glu Leu Val Glu Arg Tyr Ser
            420                 425                 430

Gln Lys Ala Ala Arg Asn Gly Met Lys Asn Gln Phe Asn Leu His Glu
        435                 440                 445

Leu Lys Met Lys Gly Pro Glu Pro Val Val Ser Gln Ile Ile Asp Lys
    450                 455                 460

Leu Lys His Ile Asn Gln Leu Leu Arg Thr Met Ser Val Pro Lys Gly
465                 470                 475                 480

Lys Val Leu Asp Lys Ser Leu Asp Glu Glu Gly Leu Glu Ser Gly Asp
                485                 490                 495

Cys Gly Asp Asp Glu Asp Glu Cys Ile Gly Ser Ser Gly Asp Gly Met
            500                 505                 510

Val Lys Val Lys Asn Gln Leu Arg Phe Leu Ala Glu Leu Ala Tyr Asp
        515                 520                 525

Leu Asp Val Asp Asp Ala Pro Gly Asn Lys Gln His Gly Asn Gln Lys
    530                 535                 540

Asp Asn Glu Ile Thr Thr Ser His Ser Val Gly Asn Met Pro Ser Pro
545                 550                 555                 560

Leu Lys Ile Leu Ile Ser Val Ala Ile Tyr Val Ala Cys Phe Phe Phe
                565                 570                 575

Leu Val His

<210>  56
<211>  247
<212>  PRT
<213>  Artificial sequence

<220>
<223>  Synthesized polypeptide

<400>  56

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asn Pro Ser Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr

100                 105                 110

Thr Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Ser Ser Leu
    130                 135                 140

Thr Val Thr Ala Gly Glu Lys Val Thr Met Ser Cys Lys Ser Ser Gln
145                 150                 155                 160

Ser Leu Leu Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr
            180                 185                 190

Arg Glu Ser Gly Val Pro Asp Arg Phe Thr Gly Ser Gly Ser Gly Thr
        195                 200                 205

Asp Phe Thr Leu Thr Ile Ser Ser Val Gln Ala Glu Asp Leu Ala Val
    210                 215                 220

Tyr Tyr Cys Gln Asn Asp Tyr Ser Tyr Pro Leu Thr Phe Gly Ala Gly
225                 230                 235                 240

Thr Lys Leu Glu Leu Lys Arg
                245

<210>  57
<211>  5
<212>  PRT
<213>  Artificial sequence

<220>
<223>  Synthesized polypeptide

<400>  57

Ser Tyr Thr Met His
1               5

<210>  58
<211>  17
<212>  PRT
<213>  Artificial sequence

<220>
<223>  Synthesized polypeptide

<400>  58

Tyr Ile Asn Pro Ser Ser Gly Tyr Thr Asn Tyr Asn Gln Lys Phe Lys
1               5                   10                  15

Asp

<210>  59
<211>  9
<212>  PRT
<213>  Artificial sequence

<220>
<223>  Synthesized polypeptide

<400>  59

Ile Tyr Tyr Gly Asn Ser Phe Ala Tyr
1               5

<210>  60
<211>  17
<212>  PRT
<213>  Artificial sequence

<220>
<223>  Synthesized polypeptide

<400>  60

Lys Ser Ser Gln Ser Leu Leu Asn Ser Gly Asn Gln Lys Asn Tyr Leu
1               5                   10                  15

Thr

<210>  61
<211>  7
<212>  PRT
<213>  Artificial sequence

<220>
<223>  Synthesized polypeptide

<400>  61

Trp Ala Ser Thr Arg Glu Ser
1               5

<210>  62
<211>  9
<212>  PRT
<213>  Artificial sequence

<220>
<223>  Synthesized polypeptide

<400>  62

Gln Asn Asp Tyr Ser Tyr Pro Leu Thr
1               5

<210>  63
<211>  118
<212>  PRT
<213>  Artificial sequence

<220>
<223>  Synthesized polypeptide

<400>  63

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asn Pro Ser Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser
        115

<210>  64
<211>  114
<212>  PRT
<213>  Artificial sequence

<220>
<223>  Synthesized polypeptide

<400>  64

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Leu Thr Phe Gly Ala Gly Thr Lys Leu Glu Leu
            100                 105                 110

Lys Arg

<210>  65
<211>  242
<212>  PRT
<213>  Artificial sequence

<220>
<223>  Synthesized polypeptide

<400>  65

Gln Val Gln Leu Gln Gln Ser Gly Pro Glu Leu Val Lys Pro Gly Ala
1               5                   10                  15

Leu Val Lys Ile Ser Cys Lys Ala Ser Gly Tyr Thr Phe Thr Ser Tyr
            20                  25                  30

Asp Ile Asn Trp Val Lys Gln Arg Pro Gly Gln Gly Leu Glu Trp Ile
        35                  40                  45

Gly Trp Ile Tyr Pro Gly Asp Gly Ser Thr Lys Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asn Ser Ala Val Tyr Phe Cys
                85                  90                  95

Ala Arg Gly Gly Tyr Arg Tyr Asp Glu Ala Met Asp Tyr Trp Gly Gln
            100                 105                 110

Gly Thr Thr Val Thr Val Ser Ser Gly Gly Gly Gly Ser Gly Gly Gly
        115                 120                 125

Gly Ser Gly Gly Gly Gly Ser Gln Ile Val Leu Thr Gln Ser Pro Ala
    130                 135                 140

Ile Met Ser Ala Ser Pro Gly Glu Lys Val Thr Met Thr Cys Ser Ala
145                 150                 155                 160

Ser Ser Ser Ile Ser Tyr Met His Trp Tyr Gln Gln Lys Pro Gly Thr
                165                 170                 175

Ser Pro Lys Arg Trp Ile Tyr Asp Thr Ser Lys Leu Ala Ser Gly Val
            180                 185                 190

Pro Ala Arg Phe Ser Gly Ser Gly Ser Gly Thr Ser Tyr Ser Leu Thr
        195                 200                 205

Ile Ser Ser Met Glu Ala Glu Asp Ala Ala Thr Tyr Tyr Cys His Gln
    210                 215                 220

Arg Ser Ser Tyr Pro Tyr Thr Phe Gly Gly Gly Thr Lys Leu Glu Ile
225                 230                 235                 240

Lys Arg

<210>  66
<211>  5
<212>  PRT
<213>  Artificial sequence

<220>
<223>  Synthesized polypeptide

<400>  66

Ser Tyr Asp Ile Asn
1               5

<210>  67
<211>  17
<212>  PRT
<213>  Artificial sequence

<220>
<223>  Synthesized polypeptide

<400>  67

Trp Ile Tyr Pro Gly Asp Gly Ser Thr Lys Tyr Asn Glu Lys Phe Lys
1               5                   10                  15

Gly

<210> 68
<211> 11
<212> PRT
<213> Artificial sequence

<220>
<223> Synthesized polypeptide

<400> 68

Gly Gly Tyr Arg Tyr Asp Glu Ala Met Asp Tyr
1               5                   10

<210> 69
<211> 10
<212> PRT
<213> Artificial sequence

<220>
<223> Synthesized polypeptide

<400> 69

Ser Ala Ser Ser Ser Ile Ser Tyr Met His
1               5                   10

<210> 70
<211> 7
<212> PRT
<213> Artificial sequence

<220>
<223> Synthesized polypeptide

<400> 70

Asp Thr Ser Lys Leu Ala Ser
1               5

<210> 71
<211> 9
<212> PRT
<213> Artificial sequence

<220>
<223> Synthesized polypeptide

<400> 71

His Gln Arg Ser Ser Tyr Pro Tyr Thr
1               5

<210> 72

<211>  120
<212>  PRT
<213>  Artificial sequence

<220>
<223>  Synthesized polypeptide

<400>  72

Gln Val Gln Leu Gln Gln Ser Gly Pro Glu Leu Val Lys Pro Gly Ala
1               5                   10                  15

Leu Val Lys Ile Ser Cys Lys Ala Ser Gly Tyr Thr Phe Thr Ser Tyr
            20                  25                  30

Asp Ile Asn Trp Val Lys Gln Arg Pro Gly Gln Gly Leu Glu Trp Ile
        35                  40                  45

Gly Trp Ile Tyr Pro Gly Asp Gly Ser Thr Lys Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asn Ser Ala Val Tyr Phe Cys
                85                  90                  95

Ala Arg Gly Gly Tyr Arg Tyr Asp Glu Ala Met Asp Tyr Trp Gly Gln
            100                 105                 110

Gly Thr Thr Val Thr Val Ser Ser
        115                 120


<210>  73
<211>  107
<212>  PRT
<213>  Artificial sequence

<220>
<223>  Synthesized polypeptide

<400>  73

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Ile Ser Tyr Met
            20                  25                  30

His Trp Tyr Gln Gln Lys Pro Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys His Gln Arg Ser Ser Tyr Pro Tyr Thr
                85                  90                  95

Phe Gly Gly Gly Thr Lys Leu Glu Ile Lys Arg
            100                 105

<210>  74
<211>  247
<212>  PRT
<213>  Artificial sequence

<220>
<223>  Synthesized polypeptide

<400>  74

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Tyr
            20                  25                  30

Gly Met Asn Trp Val Lys Gln Ala Pro Gly Lys Gly Leu Lys Trp Met
        35                  40                  45

Gly Trp Ile Asn Thr Asn Thr Gly Glu Pro Thr Tyr Ala Glu Glu Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Phe Ser Tyr Gly Asn Ser Phe Ala Tyr Trp Gly Gln Gly Thr
            100                 105                 110

Thr Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Ser Ser Leu
    130                 135                 140

Ser Val Ser Ala Gly Glu Lys Val Thr Met Ser Cys Lys Ser Ser Gln

145                 150                 155                 160

Ser Leu Leu Asn Ser Gly Asn Gln Lys Asn Tyr Leu Ala Trp Tyr Gln
                165                 170                 175

Gln Lys Pro Gly Gln Pro Pro Lys Leu Leu Ile Tyr Gly Ala Ser Thr
            180                 185                 190

Arg Glu Ser Gly Val Pro Asp Arg Phe Thr Gly Ser Gly Ser Gly Thr
        195                 200                 205

Asp Phe Thr Leu Thr Ile Ser Ser Val Gln Ala Glu Asp Leu Ala Val
    210                 215                 220

Tyr Tyr Cys Gln Asn Asp His Ser Tyr Pro Leu Thr Phe Gly Ala Gly
225                 230                 235                 240

Thr Lys Leu Glu Leu Lys Arg
                245

<210>  75
<211>  5
<212>  PRT
<213>  Artificial sequence

<220>
<223>  Synthesized polypeptide

<400>  75

Asn Tyr Gly Met Asn
1               5

<210>  76
<211>  17
<212>  PRT
<213>  Artificial sequence

<220>
<223>  Synthesized polypeptide

<400>  76

Trp Ile Asn Thr Asn Thr Gly Glu Pro Thr Tyr Ala Glu Glu Phe Lys
1               5                   10                  15

Gly

<210>  77
<211>  9
<212>  PRT
<213>  Artificial sequence

<220>
<223>  Synthesized polypeptide

<400>  77

Phe Ser Tyr Gly Asn Ser Phe Ala Tyr
1               5


<210>  78
<211>  17
<212>  PRT
<213>  Artificial sequence

<220>
<223>  Synthesized polypeptide

<400>  78

Lys Ser Ser Gln Ser Leu Leu Asn Ser Gly Asn Gln Lys Asn Tyr Leu
1               5                   10                  15


Ala




<210>  79
<211>  7
<212>  PRT
<213>  Artificial sequence

<220>
<223>  Synthesized polypeptide

<400>  79

Gly Ala Ser Thr Arg Glu Ser
1               5


<210>  80
<211>  9
<212>  PRT
<213>  Artificial sequence

<220>
<223>  Synthesized polypeptide

<400>  80

Gln Asn Asp His Ser Tyr Pro Leu Thr
1               5


<210>  81
<211>  118
<212>  PRT
<213>  Artificial sequence

<220>
<223>  Synthesized polypeptide

<400>  81

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Tyr
            20                  25                  30

Gly Met Asn Trp Val Lys Gln Ala Pro Gly Lys Gly Leu Lys Trp Met
        35                  40                  45

Gly Trp Ile Asn Thr Asn Thr Gly Glu Pro Thr Tyr Ala Glu Glu Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Phe Ser Tyr Gly Asn Ser Phe Ala Tyr Trp Gly Gln Gly Thr
            100                 105                 110

Thr Val Thr Val Ser Ser
        115


<210>  82
<211>  114
<212>  PRT
<213>  Artificial sequence

<220>
<223>  Synthesized polypeptide

<400>  82

Asp Ile Val Met Thr Gln Ser Pro Ser Ser Leu Ser Val Ser Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Ala Trp Tyr Gln Gln Lys Pro Gly Gln
        35                  40                  45

Pro Pro Lys Leu Leu Ile Tyr Gly Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp His Ser Tyr Pro Leu Thr Phe Gly Ala Gly Thr Lys Leu Glu Leu
            100                 105                 110

Lys Arg

<210>  83
<211>  247
<212>  PRT
<213>  Artificial sequence

<220>
<223>  Synthesized polypeptide

<400>  83

Asp Val Gln Leu Gln Glu Ser Gly Pro Asp Leu Val Lys Pro Ser Gln
1               5                   10                  15

Ser Leu Ser Leu Thr Cys Thr Val Thr Gly Tyr Ser Ile Thr Ser Gly
            20                  25                  30

Tyr Asn Trp His Trp Ile Arg Gln Phe Pro Gly Asn Lys Met Glu Trp
        35                  40                  45

Met Gly Tyr Ile His Tyr Thr Gly Ser Thr Asn Tyr Asn Pro Ser Leu
    50                  55                  60

Arg Ser Arg Ile Ser Ile Thr Arg Asp Thr Ser Lys Asn Gln Phe Phe
65                  70                  75                  80

Leu Gln Leu Asn Ser Val Thr Thr Asp Asp Thr Ala Thr Tyr Tyr Cys
                85                  90                  95

Thr Arg Ile Tyr Asn Gly Asn Ser Phe Pro Tyr Trp Gly Gln Gly Thr
            100                 105                 110

Ser Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Ser Ser Leu
    130                 135                 140

Thr Val Thr Pro Gly Glu Lys Val Thr Met Thr Cys Lys Ser Ser Gln
145                 150                 155                 160

Ser Leu Phe Asn Ser Gly Asn Gln Lys Asn Tyr Leu Thr Trp Tyr Gln
                165                 170                 175

Gln Arg Pro Gly Gln Pro Pro Lys Met Leu Ile Tyr Trp Ala Ser Thr
            180                 185                 190

Arg Glu Ser Gly Val Pro Asp Arg Phe Thr Gly Ser Gly Ser Gly Thr
        195                 200                 205

Asp Phe Thr Leu Thr Ile Ser Ser Val Gln Ala Glu Asp Leu Ala Val
    210                 215                 220

Phe Tyr Cys Gln Asn Ala Tyr Ser Phe Pro Tyr Thr Phe Gly Gly Gly
225                 230                 235                 240

Thr Lys Leu Glu Ile Lys Arg
                245

<210>  84
<211>  6
<212>  PRT
<213>  Artificial sequence

<220>
<223>  Synthesized polypeptide

<400>  84

Ser Gly Tyr Asn Trp His
1               5

<210>  85
<211>  16
<212>  PRT
<213>  Artificial sequence

<220>
<223>  Synthesized polypeptide

<400>  85

Tyr Ile His Tyr Thr Gly Ser Thr Asn Tyr Asn Pro Ser Leu Arg Ser
1               5                   10                  15

<210>  86
<211>  9
<212>  PRT
<213>  Artificial sequence

<220>
<223>  Synthesized polypeptide

<400>  86

Ile Tyr Asn Gly Asn Ser Phe Pro Tyr
1               5

<210>  87
<211>  17
<212>  PRT
<213>  Artificial sequence

<220>
<223>  Synthesized polypeptide

<400>  87

Lys Ser Ser Gln Ser Leu Phe Asn Ser Gly Asn Gln Lys Asn Tyr Leu
1               5                   10                  15

Thr

<210>  88
<211>  7
<212>  PRT
<213>  Artificial sequence

<220>
<223>  Synthesized polypeptide

<400>  88

Trp Ala Ser Thr Arg Glu Ser
1               5

<210>  89
<211>  9
<212>  PRT
<213>  Artificial sequence

<220>
<223>  Synthesized polypeptide

<400>  89

Gln Asn Ala Tyr Ser Phe Pro Tyr Thr
1               5

<210>  90
<211>  118
<212>  PRT
<213>  Artificial sequence

<220>
<223>  Synthesized polypeptide

<400>  90

Asp Val Gln Leu Gln Glu Ser Gly Pro Asp Leu Val Lys Pro Ser Gln
1               5                   10                  15

Ser Leu Ser Leu Thr Cys Thr Val Thr Gly Tyr Ser Ile Thr Ser Gly
            20                  25                  30

Tyr Asn Trp His Trp Ile Arg Gln Phe Pro Gly Asn Lys Met Glu Trp
        35                  40                  45

Met Gly Tyr Ile His Tyr Thr Gly Ser Thr Asn Tyr Asn Pro Ser Leu
    50                  55                  60

Arg Ser Arg Ile Ser Ile Thr Arg Asp Thr Ser Lys Asn Gln Phe Phe
65                  70                  75                  80

Leu Gln Leu Asn Ser Val Thr Thr Asp Asp Thr Ala Thr Tyr Tyr Cys
                85                  90                  95

Thr Arg Ile Tyr Asn Gly Asn Ser Phe Pro Tyr Trp Gly Gln Gly Thr
            100                 105                 110

Ser Val Thr Val Ser Ser
        115

<210> 91
<211> 114
<212> PRT
<213> Artificial sequence

<220>
<223> Synthesized polypeptide

<400> 91

Asp Ile Val Met Thr Gln Ser Pro Ser Ser Leu Thr Val Thr Pro Gly
1               5                   10                  15

Glu Lys Val Thr Met Thr Cys Lys Ser Ser Gln Ser Leu Phe Asn Ser
            20                  25                  30

Gly Asn Gln Lys Asn Tyr Leu Thr Trp Tyr Gln Gln Arg Pro Gly Gln
        35                  40                  45

Pro Pro Lys Met Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Phe Tyr Cys Gln Asn
                85                  90                  95

Ala Tyr Ser Phe Pro Tyr Thr Phe Gly Gly Gly Thr Lys Leu Glu Ile
            100                 105                 110

Lys Arg

<210>  92
<211>  247
<212>  PRT
<213>  Artificial sequence

<220>
<223>  Synthesized polypeptide

<400>  92

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asp Pro Ser Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Ser Ser Leu
    130                 135                 140

Thr Val Thr Ala Gly Glu Lys Val Thr Met Ser Cys Lys Ser Ser Gln
145                 150                 155                 160

Ser Leu Leu Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr

                180                 185                 190

Arg Glu Ser Gly Val Pro Asp Arg Phe Thr Gly Ser Gly Ser Gly Thr
        195                 200                 205

Asp Phe Thr Leu Thr Ile Ser Ser Val Gln Ala Glu Asp Leu Ala Val
    210                 215                 220

Tyr Tyr Cys Gln Asn Asp Tyr Ser Tyr Pro Leu Thr Phe Gly Ala Gly
225                 230                 235                 240

Thr Lys Leu Glu Leu Lys Arg
                245

<210>  93
<211>  5
<212>  PRT
<213>  Artificial sequence

<220>
<223>  Synthesized polypeptide

<400>  93

Ser Tyr Thr Met His
1               5

<210>  94
<211>  17
<212>  PRT
<213>  Artificial sequence

<220>
<223>  Synthesized polypeptide

<400>  94

Tyr Ile Asp Pro Ser Ser Gly Tyr Thr Asn Tyr Asn Gln Lys Phe Lys
1               5                   10                  15

Asp

<210>  95
<211>  9
<212>  PRT
<213>  Artificial sequence

<220>
<223>  Synthesized polypeptide

<400>  95

Ile Tyr Tyr Gly Asn Ser Phe Ala Tyr
1               5

<210>  96
<211>  17
<212>  PRT
<213>  Artificial sequence

<220>
<223>  Synthesized polypeptide

<400>  96

Lys Ser Ser Gln Ser Leu Leu Asn Ser Gly Asn Gln Lys Asn Tyr Leu
1               5                   10                  15

Thr

<210>  97
<211>  7
<212>  PRT
<213>  Artificial sequence

<220>
<223>  Synthesized polypeptide

<400>  97

Trp Ala Ser Thr Arg Glu Ser
1               5

<210>  98
<211>  9
<212>  PRT
<213>  Artificial sequence

<220>
<223>  Synthesized polypeptide

<400>  98

Gln Asn Asp Tyr Ser Tyr Pro Leu Thr
1               5

<210>  99
<211>  118
<212>  PRT
<213>  Artificial sequence

<220>
<223>  Synthesized polypeptide

<400>  99

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr

20                  25                  30

Thr Met His Trp Val Lys Gln Arg Pro Gly Gln Gly Leu Glu Trp Ile
        35                  40                  45

Gly Tyr Ile Asp Pro Ser Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser
        115

<210>  100
<211>  114
<212>  PRT
<213>  Artificial sequence

<220>
<223>  Synthesized polypeptide

<400>  100

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Leu Thr Phe Gly Ala Gly Thr Lys Leu Glu Leu
            100                 105                 110

Lys Arg

<210> 101
<211> 247
<212> PRT
<213> Artificial sequence

<220>
<223> Synthesized polypeptide

<400> 101

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asn Pro Ala Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Ser Ser Leu
    130                 135                 140

Thr Val Thr Ala Gly Glu Lys Val Thr Met Ser Cys Lys Ser Ser Gln
145                 150                 155                 160

Ser Leu Leu Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr
            180                 185                 190

Arg Glu Ser Gly Val Pro Asp Arg Phe Thr Gly Ser Gly Ser Gly Thr
        195                 200                 205

Asp Phe Thr Leu Thr Ile Ser Ser Val Gln Ala Glu Asp Leu Ala Val
    210                 215                 220

Tyr Tyr Cys Gln Asn Asp Tyr Ser Tyr Pro Leu Thr Phe Gly Ala Gly
225                 230                 235                 240

Thr Lys Leu Glu Leu Lys Arg
                245

<210>  102
<211>  5
<212>  PRT
<213>  Artificial sequence

<220>
<223>  Synthesized polypeptide

<400>  102

Ser Tyr Thr Met His
1               5

<210>  103
<211>  17
<212>  PRT
<213>  Artificial sequence

<220>
<223>  Synthesized polypeptide

<400>  103

Tyr Ile Asn Pro Ala Ser Gly Tyr Thr Asn Tyr Asn Gln Lys Phe Lys
1               5                   10                  15

Asp

<210>  104
<211>  9
<212>  PRT
<213>  Artificial sequence

<220>
<223>  Synthesized polypeptide

<400>  104

Ile Tyr Tyr Gly Asn Ser Phe Ala Tyr
1               5

<210>  105
<211>  17
<212>  PRT
<213>  Artificial sequence

<220>
<223>  Synthesized polypeptide

<400>  105

Lys Ser Ser Gln Ser Leu Leu Asn Ser Gly Asn Gln Lys Asn Tyr Leu
1               5                   10                  15

Thr

<210>  106
<211>  7
<212>  PRT
<213>  Artificial sequence

<220>
<223>  Synthesized polypeptide

<400>  106

Trp Ala Ser Thr Arg Glu Ser
1               5

<210>  107
<211>  9
<212>  PRT
<213>  Artificial sequence

<220>
<223>  Synthesized polypeptide

<400>  107

Gln Asn Asp Tyr Ser Tyr Pro Leu Thr
1               5

<210>  108
<211>  118
<212>  PRT
<213>  Artificial sequence

<220>
<223>  Synthesized polypeptide

<400>  108

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asn Pro Ala Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser
        115

<210>  109
<211>  114
<212>  PRT
<213>  Artificial sequence

<220>
<223>  Synthesized polypeptide

<400>  109

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Leu Thr Phe Gly Ala Gly Thr Lys Leu Glu Leu
            100                 105                 110

Lys Arg

<210> 110
<211> 247
<212> PRT
<213> Artificial sequence

<220>
<223> Synthesized polypeptide

<400> 110

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asn Pro Ala Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Ser Ser Leu
    130                 135                 140

Thr Val Thr Ala Gly Glu Lys Val Thr Met Ser Cys Lys Ser Ser Gln
145                 150                 155                 160

Ser Leu Leu Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr
            180                 185                 190

Arg Glu Ser Gly Val Pro Asp Arg Phe Thr Gly Ser Gly Ser Gly Thr

195                 200                 205

Asp Phe Thr Leu Thr Ile Ser Ser Val Gln Ala Glu Asp Leu Ala Val
    210                 215                 220

Tyr Tyr Cys Gln Asn Asp Tyr Ser Tyr Pro Leu Thr Phe Gly Ala Gly
225                 230                 235                 240

Thr Lys Leu Glu Leu Lys Arg
                245

<210>  111
<211>  5
<212>  PRT
<213>  Artificial sequence

<220>
<223>  Synthesized polypeptide

<400>  111

Ser Tyr Thr Met His
1               5

<210>  112
<211>  17
<212>  PRT
<213>  Artificial sequence

<220>
<223>  Synthesized polypeptide

<400>  112

Tyr Ile Asn Pro Ala Ser Gly Tyr Thr Asn Tyr Asn Gln Lys Phe Lys
1               5                   10                  15

Asp

<210>  113
<211>  9
<212>  PRT
<213>  Artificial sequence

<220>
<223>  Synthesized polypeptide

<400>  113

Ile Tyr Tyr Gly Asn Ser Phe Ala Tyr
1               5

<210>  114
<211>  17

<212>  PRT
<213>  Artificial sequence

<220>
<223>  Synthesized polypeptide

<400>  114

Lys Ser Ser Gln Ser Leu Leu Asn Ser Gly Asn Gln Lys Asn Tyr Leu
1               5                   10                  15

Thr

<210>  115
<211>  7
<212>  PRT
<213>  Artificial sequence

<220>
<223>  Synthesized polypeptide

<400>  115

Trp Ala Ser Thr Arg Glu Ser
1               5

<210>  116
<211>  9
<212>  PRT
<213>  Artificial sequence

<220>
<223>  Synthesized polypeptide

<400>  116

Gln Asn Asp Tyr Ser Tyr Pro Leu Thr
1               5

<210>  117
<211>  118
<212>  PRT
<213>  Artificial sequence

<220>
<223>  Synthesized polypeptide

<400>  117

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile

35                  40                  45

Gly Tyr Ile Asn Pro Ala Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser
        115

<210>  118
<211>  114
<212>  PRT
<213>  Artificial sequence

<220>
<223>  Synthesized polypeptide

<400>  118

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Leu Thr Phe Gly Ala Gly Thr Lys Leu Glu Leu
            100                 105                 110

Lys Arg

<210>  119
<211>  247
<212>  PRT
<213>  Artificial sequence

<220>
<223>  Synthesized polypeptide

<400>  119

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Thr Met His Trp Val Arg Gln Ala Pro Gly Gln Gly Leu Glu Trp Met
        35                  40                  45

Gly Tyr Ile Asn Pro Ala Ser Gly Tyr Thr Asn Tyr Asn Gln Lys Phe
    50                  55                  60

Lys Asp Arg Val Thr Met Thr Arg Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ile Tyr Tyr Gly Asn Ser Phe Ala Tyr Trp Gly Gln Gly Thr
            100                 105                 110

Leu Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Asp Ser Leu
    130                 135                 140

Ala Val Ser Leu Gly Glu Arg Ala Thr Ile Asn Cys Lys Ser Ser Gln
145                 150                 155                 160

Ser Leu Leu Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr
            180                 185                 190

Arg Glu Ser Gly Val Pro Asp Arg Phe Ser Gly Ser Gly Ser Gly Thr
        195                 200                 205

Asp Phe Thr Leu Thr Ile Ser Ser Leu Gln Ala Glu Asp Val Ala Val
    210                 215                 220

Tyr Tyr Cys Gln Asn Asp Tyr Ser Tyr Pro Leu Thr Phe Gly Gly Gly
225                 230                 235                 240

Thr Lys Val Glu Ile Lys Arg
                245

<210>  120
<211>  5
<212>  PRT
<213>  Artificial sequence

<220>
<223>  Synthesized polypeptide

<400>  120

Ser Tyr Thr Met His
1               5

<210>  121
<211>  17
<212>  PRT
<213>  Artificial sequence

<220>
<223>  Synthesized polypeptide

<400>  121

Tyr Ile Asn Pro Ala Ser Gly Tyr Thr Asn Tyr Asn Gln Lys Phe Lys
1               5                   10                  15

Asp

<210>  122
<211>  9
<212>  PRT
<213>  Artificial sequence

<220>
<223>  Synthesized polypeptide

<400>  122

Ile Tyr Tyr Gly Asn Ser Phe Ala Tyr
1               5

<210>  123
<211>  17
<212>  PRT
<213>  Artificial sequence

<220>
<223>  Synthesized polypeptide

<400>  123

Lys Ser Ser Gln Ser Leu Leu Asn Ser Gly Asn Gln Lys Asn Tyr Leu
1               5                   10                  15

Thr

<210>  124
<211>  7
<212>  PRT
<213>  Artificial sequence

<220>
<223>  Synthesized polypeptide

<400>  124

Trp Ala Ser Thr Arg Glu Ser
1               5

<210>  125
<211>  9
<212>  PRT
<213>  Artificial sequence

<220>
<223>  Synthesized polypeptide

<400>  125

Gln Asn Asp Tyr Ser Tyr Pro Leu Thr
1               5

<210>  126
<211>  118
<212>  PRT
<213>  Artificial sequence

<220>
<223>  Synthesized polypeptide

<400>  126

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Thr Met His Trp Val Arg Gln Ala Pro Gly Gln Gly Leu Glu Trp Met
        35                  40                  45

Gly Tyr Ile Asn Pro Ala Ser Gly Tyr Thr Asn Tyr Asn Gln Lys Phe
    50                  55                  60

Lys Asp Arg Val Thr Met Thr Arg Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ile Tyr Tyr Gly Asn Ser Phe Ala Tyr Trp Gly Gln Gly Thr
            100                 105                 110

Leu Val Thr Val Ser Ser
        115

<210>  127
<211>  114
<212>  PRT
<213>  Artificial sequence

<220>
<223>  Synthesized polypeptide

<400>  127

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Asn
                85                  90                  95

Asp Tyr Ser Tyr Pro Leu Thr Phe Gly Gly Gly Thr Lys Val Glu Ile
            100                 105                 110

Lys Arg

<210>  128
<211>  247
<212>  PRT
<213>  Artificial sequence

<220>
<223>  Synthesized polypeptide

<400>  128

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Tyr Asn Trp His Trp Ile Arg Gln Pro Pro Gly Lys Gly Leu Glu Trp
        35                  40                  45

Ile Gly Tyr Ile His Tyr Thr Gly Ser Thr Asn Tyr Asn Pro Ala Leu
    50                  55                  60

Arg Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Ile Tyr Asn Gly Asn Ser Phe Pro Tyr Trp Gly Gln Gly Thr
            100                 105                 110

Thr Val Thr Val Ser Ser Gly Gly Gly Gly Ser Gly Gly Gly Gly Ser
        115                 120                 125

Gly Gly Gly Gly Ser Asp Ile Val Met Thr Gln Ser Pro Asp Ser Leu
    130                 135                 140

Ala Val Ser Leu Gly Glu Arg Ala Thr Ile Asn Cys Lys Ser Ser Gln
145                 150                 155                 160

Ser Leu Phe Asn Ser Gly Asn Gln Lys Asn Tyr Leu Thr Trp Tyr Gln
                165                 170                 175

Gln Lys Pro Gly Gln Pro Pro Lys Leu Leu Ile Tyr Trp Ala Ser Thr
            180                 185                 190

Arg Glu Ser Gly Val Pro Asp Arg Phe Ser Gly Ser Gly Ser Gly Thr
        195                 200                 205

Asp Phe Thr Leu Thr Ile Ser Ser Leu Gln Ala Glu Asp Val Ala Val

210                 215                 220

Tyr Tyr Cys Gln Asn Ala Tyr Ser Phe Pro Tyr Thr Phe Gly Gly Gly
225                 230                 235                 240

Thr Lys Leu Glu Ile Lys Arg
                245

<210>  129
<211>  6
<212>  PRT
<213>  Artificial sequence

<220>
<223>  Synthesized polypeptide

<400>  129

Ser Gly Tyr Asn Trp His
1               5

<210>  130
<211>  16
<212>  PRT
<213>  Artificial sequence

<220>
<223>  Synthesized polypeptide

<400>  130

Tyr Ile His Tyr Thr Gly Ser Thr Asn Tyr Asn Pro Ala Leu Arg Ser
1               5                   10                  15

<210>  131
<211>  9
<212>  PRT
<213>  Artificial sequence

<220>
<223>  Synthesized polypeptide

<400>  131

Ile Tyr Asn Gly Asn Ser Phe Pro Tyr
1               5

<210>  132
<211>  17
<212>  PRT
<213>  Artificial sequence

<220>
<223>  Synthesized polypeptide

<400>  132

Lys Ser Ser Gln Ser Leu Phe Asn Ser Gly Asn Gln Lys Asn Tyr Leu
1               5                   10                  15

Thr

<210>  133
<211>  7
<212>  PRT
<213>  Artificial sequence

<220>
<223>  Synthesized polypeptide

<400>  133

Trp Ala Ser Thr Arg Glu Ser
1               5

<210>  134
<211>  9
<212>  PRT
<213>  Artificial sequence

<220>
<223>  Synthesized polypeptide

<400>  134

Gln Asn Ala Tyr Ser Phe Pro Tyr Thr
1               5

<210>  135
<211>  118
<212>  PRT
<213>  Artificial sequence

<220>
<223>  Synthesized polypeptide

<400>  135

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Tyr Asn Trp His Trp Ile Arg Gln Pro Pro Gly Lys Gly Leu Glu Trp
        35                  40                  45

Ile Gly Tyr Ile His Tyr Thr Gly Ser Thr Asn Tyr Asn Pro Ala Leu
    50                  55                  60

Arg Ser Arg Val Thr Ile Ser Val Asp Thr Ser Lys Asn Gln Phe Ser

65                  70                  75                  80

Leu Lys Leu Ser Ser Val Thr Ala Ala Asp Thr Ala Val Tyr Tyr Cys
                85                  90                  95

Ala Arg Ile Tyr Asn Gly Asn Ser Phe Pro Tyr Trp Gly Gln Gly Thr
            100                 105                 110

Thr Val Thr Val Ser Ser
        115

<210>  136
<211>  114
<212>  PRT
<213>  Artificial sequence

<220>
<223>  Synthesized polypeptide

<400>  136

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Phe Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Asn
                85                  90                  95

Ala Tyr Ser Phe Pro Tyr Thr Phe Gly Gly Gly Thr Lys Leu Glu Ile
            100                 105                 110

Lys Arg

<210>  137
<211>  247
<212>  PRT
<213>  Artificial sequence

<220>

<223>  Synthesized polypeptide

<400>  137

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Gln Ser Ala Leu Thr Gln Pro Pro
    130                 135                 140

Ser Ala Ser Gly Ser Pro Gly Gln Ser Val Thr Ile Ser Cys Thr Gly
145                 150                 155                 160

Thr Ser Ser Asp Val Gly Gly Tyr Asn Tyr Val Ser Trp Tyr Gln Gln
                165                 170                 175

Tyr Pro Gly Lys Ala Pro Lys Leu Leu Ile Tyr Gly Asn Ser Asn Arg
            180                 185                 190

Pro Ser Gly Val Pro Asp Arg Phe Ser Gly Ser Lys Ser Gly Thr Ser
        195                 200                 205

Ala Ser Leu Ala Ile Thr Gly Leu Gln Ala Glu Asp Gly Ala Asp Tyr
    210                 215                 220

Tyr Cys Gln Ser Tyr Asp Ser Ser Leu Arg Val Val Phe Gly Gly Gly
225                 230                 235                 240

Thr Lys Val Thr Val Leu Gly
                245

<210>  138
<211>  10
<212>  PRT
<213>  Artificial sequence

<220>
<223>  Synthesized polypeptide

<400>  138

Gly Phe Thr Phe Ser Ser Tyr Ala Met His
1               5                   10

<210>  139
<211>  17
<212>  PRT
<213>  Artificial sequence

<220>
<223>  Synthesized polypeptide

<400>  139

Ala Ile Ser Gly Ser Gly Gly Ser Thr Tyr Tyr Ala Asp Ser Val Lys
1               5                   10                  15

Gly

<210>  140
<211>  12
<212>  PRT
<213>  Artificial sequence

<220>
<223>  Synthesized polypeptide

<400>  140

Asp Arg Arg Gly Ser His Ala Asp Ala Phe Asp Val
1               5                   10

<210>  141
<211>  14
<212>  PRT
<213>  Artificial sequence

<220>
<223>  Synthesized polypeptide

<400>  141

Thr Gly Thr Ser Ser Asp Val Gly Gly Tyr Asn Tyr Val Ser
1               5                   10

<210>  142
<211>  7
<212>  PRT
<213>  Artificial sequence

<220>
<223>  Synthesized polypeptide

<400>  142

Gly Asn Ser Asn Arg Pro Ser
1               5


<210>  143
<211>  10
<212>  PRT
<213>  Artificial sequence

<220>
<223>  Synthesized polypeptide

<400>  143

Gln Ser Tyr Asp Ser Ser Leu Arg Val Val
1               5                   10


<210>  144
<211>  121
<212>  PRT
<213>  Artificial sequence

<220>
<223>  Synthesized polypeptide

<400>  144

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser
        115                 120

<210>  145
<211>  111
<212>  PRT
<213>  Artificial sequence

<220>
<223>  Synthesized polypeptide

<400>  145

Gln Ser Ala Leu Thr Gln Pro Pro Ser Ala Ser Gly Ser Pro Gly Gln
1               5                   10                  15

Ser Val Thr Ile Ser Cys Thr Gly Thr Ser Ser Asp Val Gly Gly Tyr
            20                  25                  30

Asn Tyr Val Ser Trp Tyr Gln Gln Tyr Pro Gly Lys Ala Pro Lys Leu
        35                  40                  45

Leu Ile Tyr Gly Asn Ser Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Gly Ala Asp Tyr Tyr Cys Gln Ser Tyr Asp Ser Ser
                85                  90                  95

Leu Arg Val Val Phe Gly Gly Gly Thr Lys Val Thr Val Leu Gly
            100                 105                 110

<210>  146
<211>  247
<212>  PRT
<213>  Artificial sequence

<220>
<223>  Synthesized polypeptide

<400>  146

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Thr Tyr
            20                  25                  30

Ala Met Thr Trp Val Arg Gln Ala Pro Gly Lys Gly Leu Glu Trp Val
        35                  40                  45

Ser Ser Ile Ser Ser Ser Gly Glu Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Gln Ser Ala Leu Thr Gln Pro Pro
    130                 135                 140

Ser Ala Ser Gly Ser Pro Gly Gln Ser Val Thr Ile Ser Cys Thr Gly
145                 150                 155                 160

Thr Ser Ser Asp Val Gly Gly Tyr Asn Tyr Val Ser Trp Tyr Gln Gln
                165                 170                 175

Tyr Pro Gly Lys Ala Pro Lys Leu Leu Ile Tyr Gly Asn Ser Asn Arg
            180                 185                 190

Pro Ser Gly Val Pro Asp Arg Phe Ser Gly Ser Lys Ser Gly Thr Ser
        195                 200                 205

Ala Ser Leu Ala Ile Thr Gly Leu Gln Ala Glu Asp Gly Ala Asp Tyr
    210                 215                 220

Tyr Cys Gln Ser Tyr Asp Ser Ser Leu Arg Val Val Phe Gly Gly Gly
225                 230                 235                 240

Thr Lys Val Thr Val Leu Gly
                245

<210>  147
<211>  10
<212>  PRT
<213>  Artificial sequence

<220>
<223>  Synthesized polypeptide

<400>  147

Gly Phe Thr Phe Ser Thr Tyr Ala Met Thr
1               5                   10


<210>  148
<211>  17
<212>  PRT
<213>  Artificial sequence

<220>
<223>  Synthesized polypeptide

<400>  148

Ser Ile Ser Ser Ser Gly Glu Ser Thr Tyr Tyr Ala Asp Ser Val Lys
1               5                   10                  15


Gly




<210>  149
<211>  12
<212>  PRT
<213>  Artificial sequence

<220>
<223>  Synthesized polypeptide

<400>  149

Asp Arg Arg Gly Ser His Ala Asp Ala Phe Asp Val
1               5                   10


<210>  150
<211>  14
<212>  PRT
<213>  Artificial sequence

<220>
<223>  Synthesized polypeptide

<400>  150

Thr Gly Thr Ser Ser Asp Val Gly Gly Tyr Asn Tyr Val Ser
1               5                   10


<210>  151
<211>  7
<212>  PRT
<213>  Artificial sequence

<220>
<223>  Synthesized polypeptide

<400>  151

Gly Asn Ser Asn Arg Pro Ser
1               5


<210>  152
<211>  10
<212>  PRT
<213>  Artificial sequence

<220>
<223>  Synthesized polypeptide

<400>  152

Gln Ser Tyr Asp Ser Ser Leu Arg Val Val
1               5                   10


<210>  153
<211>  121
<212>  PRT
<213>  Artificial sequence

<220>
<223>  Synthesized polypeptide

<400>  153

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Thr Tyr
            20                  25                  30

Ala Met Thr Trp Val Arg Gln Ala Pro Gly Lys Gly Leu Glu Trp Val
        35                  40                  45

Ser Ser Ile Ser Ser Ser Gly Glu Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser
        115                 120


<210>  154

<211>  111
<212>  PRT
<213>  Artificial sequence

<220>
<223>  Synthesized polypeptide

<400>  154

Gln Ser Ala Leu Thr Gln Pro Pro Ser Ala Ser Gly Ser Pro Gly Gln
1               5                   10                  15

Ser Val Thr Ile Ser Cys Thr Gly Thr Ser Ser Asp Val Gly Gly Tyr
            20                  25                  30

Asn Tyr Val Ser Trp Tyr Gln Gln Tyr Pro Gly Lys Ala Pro Lys Leu
        35                  40                  45

Leu Ile Tyr Gly Asn Ser Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Gly Ala Asp Tyr Tyr Cys Gln Ser Tyr Asp Ser Ser
                85                  90                  95

Leu Arg Val Val Phe Gly Gly Gly Thr Lys Val Thr Val Leu Gly
            100                 105                 110


<210>  155
<211>  247
<212>  PRT
<213>  Artificial sequence

<220>
<223>  Synthesized polypeptide

<400>  155

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Thr Tyr
            20                  25                  30

Ala Met Ala Trp Val Arg Gln Ala Pro Gly Lys Gly Leu Glu Trp Val
        35                  40                  45

Ser Glu Ile Ser Ser Ser Gly Ser Arg Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Gln Ser Ala Leu Thr Gln Pro Pro
    130                 135                 140

Ser Ala Ser Gly Ser Pro Gly Gln Ser Val Thr Ile Ser Cys Thr Gly
145                 150                 155                 160

Thr Ser Ser Asp Val Gly Gly Tyr Asn Tyr Val Ser Trp Tyr Gln Gln
                165                 170                 175

Tyr Pro Gly Lys Ala Pro Lys Leu Leu Ile Tyr Gly Asn Ser Asn Arg
            180                 185                 190

Pro Ser Gly Val Pro Asp Arg Phe Ser Gly Ser Lys Ser Gly Thr Ser
        195                 200                 205

Ala Ser Leu Ala Ile Thr Gly Leu Gln Ala Glu Asp Gly Ala Asp Tyr
    210                 215                 220

Tyr Cys Gln Ser Tyr Asp Ser Ser Leu Arg Val Val Phe Gly Gly Gly
225                 230                 235                 240

Thr Lys Val Thr Val Leu Gly
                245

<210>  156
<211>  10
<212>  PRT
<213>  Artificial sequence

<220>
<223>  Synthesized polypeptide

<400>  156

Gly Phe Thr Phe Ser Thr Tyr Ala Met Ala
1               5                   10

<210>  157

<211>  17
<212>  PRT
<213>  Artificial sequence

<220>
<223>  Synthesized polypeptide

<400>  157

Glu Ile Ser Ser Ser Gly Ser Arg Thr Tyr Tyr Ala Asp Ser Val Lys
1               5                   10                  15

Gly

<210>  158
<211>  12
<212>  PRT
<213>  Artificial sequence

<220>
<223>  Synthesized polypeptide

<400>  158

Asp Arg Arg Gly Ser His Ala Asp Ala Phe Asp Val
1               5                   10

<210>  159
<211>  14
<212>  PRT
<213>  Artificial sequence

<220>
<223>  Synthesized polypeptide

<400>  159

Thr Gly Thr Ser Ser Asp Val Gly Gly Tyr Asn Tyr Val Ser
1               5                   10

<210>  160
<211>  7
<212>  PRT
<213>  Artificial sequence

<220>
<223>  Synthesized polypeptide

<400>  160

Gly Asn Ser Asn Arg Pro Ser
1               5

<210>  161
<211>  10
<212>  PRT
<213>  Artificial sequence

<220>
<223>  Synthesized polypeptide

<400>  161

Gln Ser Tyr Asp Ser Ser Leu Arg Val Val
1               5                   10


<210>  162
<211>  121
<212>  PRT
<213>  Artificial sequence

<220>
<223>  Synthesized polypeptide

<400>  162

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Thr Tyr
            20                  25                  30

Ala Met Ala Trp Val Arg Gln Ala Pro Gly Lys Gly Leu Glu Trp Val
        35                  40                  45

Ser Glu Ile Ser Ser Ser Gly Ser Arg Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser
        115                 120


<210>  163
<211>  111
<212>  PRT
<213>  Artificial sequence

<220>
<223>  Synthesized polypeptide

<400>  163

Gln Ser Ala Leu Thr Gln Pro Pro Ser Ala Ser Gly Ser Pro Gly Gln

1               5                   10                  15

Ser Val Thr Ile Ser Cys Thr Gly Thr Ser Ser Asp Val Gly Gly Tyr
            20                  25                  30

Asn Tyr Val Ser Trp Tyr Gln Gln Tyr Pro Gly Lys Ala Pro Lys Leu
        35                  40                  45

Leu Ile Tyr Gly Asn Ser Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Gly Ala Asp Tyr Tyr Cys Gln Ser Tyr Asp Ser Ser
                85                  90                  95

Leu Arg Val Val Phe Gly Gly Gly Thr Lys Val Thr Val Leu Gly
            100                 105                 110

<210>  164
<211>  247
<212>  PRT
<213>  Artificial sequence

<220>
<223>  Synthesized polypeptide

<400>  164

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Ser Ala Ile Ser Met Ser Gly Glu Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Gln Ser Ala Leu Thr Gln Pro Pro
    130                 135                 140

Ser Ala Ser Gly Ser Pro Gly Gln Ser Val Thr Ile Ser Cys Thr Gly
145                 150                 155                 160

Thr Ser Ser Asp Val Gly Gly Tyr Asn Tyr Val Ser Trp Tyr Gln Gln
                165                 170                 175

Tyr Pro Gly Lys Ala Pro Lys Leu Leu Ile Tyr Gly Asn Ser Asn Arg
            180                 185                 190

Pro Ser Gly Val Pro Asp Arg Phe Ser Gly Ser Lys Ser Gly Thr Ser
        195                 200                 205

Ala Ser Leu Ala Ile Thr Gly Leu Gln Ala Glu Asp Gly Ala Asp Tyr
    210                 215                 220

Tyr Cys Gln Ser Tyr Asp Ser Ser Leu Arg Val Val Phe Gly Gly Gly
225                 230                 235                 240

Thr Lys Val Thr Val Leu Gly
                245

<210>  165
<211>  10
<212>  PRT
<213>  Artificial sequence

<220>
<223>  Synthesized polypeptide

<400>  165

Gly Phe Thr Phe Ser Thr Tyr Ala Met Ala
1               5                   10

<210>  166
<211>  17
<212>  PRT
<213>  Artificial sequence

<220>
<223>  Synthesized polypeptide

<400>  166

Ala Ile Ser Met Ser Gly Glu Ser Thr Tyr Tyr Ala Asp Ser Val Lys

1               5                   10                  15

Gly

<210>  167
<211>  12
<212>  PRT
<213>  Artificial sequence

<220>
<223>  Synthesized polypeptide

<400>  167

Asp Arg Arg Gly Ser His Ala Asp Ala Phe Asp Val
1               5                   10

<210>  168
<211>  14
<212>  PRT
<213>  Artificial sequence

<220>
<223>  Synthesized polypeptide

<400>  168

Thr Gly Thr Ser Ser Asp Val Gly Gly Tyr Asn Tyr Val Ser
1               5                   10

<210>  169
<211>  7
<212>  PRT
<213>  Artificial sequence

<220>
<223>  Synthesized polypeptide

<400>  169

Gly Asn Ser Asn Arg Pro Ser
1               5

<210>  170
<211>  10
<212>  PRT
<213>  Artificial sequence

<220>
<223>  Synthesized polypeptide

<400>  170

Gln Ser Tyr Asp Ser Ser Leu Arg Val Val
1               5                   10

<210>  171
<211>  121
<212>  PRT
<213>  Artificial sequence

<220>
<223>  Synthesized polypeptide

<400>  171

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Ser Ala Ile Ser Met Ser Gly Glu Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser
        115                 120


<210>  172
<211>  111
<212>  PRT
<213>  Artificial sequence

<220>
<223>  Synthesized polypeptide

<400>  172

Gln Ser Ala Leu Thr Gln Pro Pro Ser Ala Ser Gly Ser Pro Gly Gln
1               5                   10                  15

Ser Val Thr Ile Ser Cys Thr Gly Thr Ser Ser Asp Val Gly Gly Tyr
            20                  25                  30

Asn Tyr Val Ser Trp Tyr Gln Gln Tyr Pro Gly Lys Ala Pro Lys Leu
        35                  40                  45

Leu Ile Tyr Gly Asn Ser Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Gly Ala Asp Tyr Tyr Cys Gln Ser Tyr Asp Ser Ser
                85                  90                  95

Leu Arg Val Val Phe Gly Gly Gly Thr Lys Val Thr Val Leu Gly
            100                 105                 110

<210>  173
<211>  247
<212>  PRT
<213>  Artificial sequence

<220>
<223>  Synthesized polypeptide

<400>  173

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ala Ile Ser Ser Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Gln Ser Ala Leu Thr Gln Pro Pro
    130                 135                 140

Ser Ala Ser Gly Ser Pro Gly Gln Ser Val Thr Ile Ser Cys Thr Gly
145                 150                 155                 160

Thr Ser Ser Asp Val Gly His Lys Phe Pro Val Ser Trp Tyr Gln Gln
                165                 170                 175

Tyr Pro Gly Lys Ala Pro Lys Leu Leu Ile Tyr Lys Asn Leu Leu Arg
            180                 185                 190

Pro Ser Gly Val Pro Asp Arg Phe Ser Gly Ser Lys Ser Gly Thr Ser
        195                 200                 205

Ala Ser Leu Ala Ile Thr Gly Leu Gln Ala Glu Asp Gly Ala Asp Tyr
    210                 215                 220

Tyr Cys Gln Ser Tyr Asp Ser Ser Leu Arg Val Val Phe Gly Gly Gly
225                 230                 235                 240

Thr Lys Val Thr Val Leu Gly
                245

<210>  174
<211>  10
<212>  PRT
<213>  Artificial sequence

<220>
<223>  Synthesized polypeptide

<400>  174

Gly Phe Thr Phe Ser Ser Tyr Ala Met His
1               5                   10

<210>  175
<211>  17
<212>  PRT
<213>  Artificial sequence

<220>
<223>  Synthesized polypeptide

<400>  175

Ala Ile Ser Ser Ser Gly Gly Ser Thr Tyr Tyr Ala Asp Ser Val Lys
1               5                   10                  15

Gly

<210>  176
<211>  12
<212>  PRT

<213>  Artificial sequence

<220>
<223>  Synthesized polypeptide

<400>  176

Asp Arg Arg Gly Ser His Ala Asp Ala Phe Asp Val
1               5                   10


<210>  177
<211>  14
<212>  PRT
<213>  Artificial sequence

<220>
<223>  Synthesized polypeptide

<400>  177

Thr Gly Thr Ser Ser Asp Val Gly His Lys Phe Pro Val Ser
1               5                   10


<210>  178
<211>  7
<212>  PRT
<213>  Artificial sequence

<220>
<223>  Synthesized polypeptide

<400>  178

Lys Asn Leu Leu Arg Pro Ser
1               5


<210>  179
<211>  10
<212>  PRT
<213>  Artificial sequence

<220>
<223>  Synthesized polypeptide

<400>  179

Gln Ser Tyr Asp Ser Ser Leu Arg Val Val
1               5                   10


<210>  180
<211>  121
<212>  PRT
<213>  Artificial sequence

<220>
<223>  Synthesized polypeptide

<400>  180

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ala Ile Ser Ser Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser
        115                 120

<210>  181
<211>  111
<212>  PRT
<213>  Artificial sequence

<220>
<223>  Synthesized polypeptide

<400>  181

Gln Ser Ala Leu Thr Gln Pro Pro Ser Ala Ser Gly Ser Pro Gly Gln
1               5                   10                  15

Ser Val Thr Ile Ser Cys Thr Gly Thr Ser Ser Asp Val Gly His Lys
            20                  25                  30

Phe Pro Val Ser Trp Tyr Gln Gln Tyr Pro Gly Lys Ala Pro Lys Leu
        35                  40                  45

Leu Ile Tyr Lys Asn Leu Leu Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Gly Ala Asp Tyr Tyr Cys Gln Ser Tyr Asp Ser Ser

85                  90                  95

Leu Arg Val Val Phe Gly Gly Gly Thr Lys Val Thr Val Leu Gly
            100                 105                 110

<210>  182
<211>  247
<212>  PRT
<213>  Artificial sequence

<220>
<223>  Synthesized polypeptide

<400>  182

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ala Ile Ser Ser Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Gln Ser Ala Leu Thr Gln Pro Pro
    130                 135                 140

Ser Ala Ser Gly Ser Pro Gly Gln Ser Val Thr Ile Ser Cys Thr Gly
145                 150                 155                 160

Thr Ser Ser Asp Val Gly Leu Met His Asn Val Ser Trp Tyr Gln Gln
                165                 170                 175

Tyr Pro Gly Lys Ala Pro Lys Leu Leu Ile Tyr Lys Ser Ser Ser Arg
            180                 185                 190

Pro Ser Gly Val Pro Asp Arg Phe Ser Gly Ser Lys Ser Gly Thr Ser
        195                 200                 205

Ala Ser Leu Ala Ile Thr Gly Leu Gln Ala Glu Asp Gly Ala Asp Tyr
    210                 215                 220

Tyr Cys Gln Ser Tyr Asp Ser Ser Leu Arg Val Val Phe Gly Gly Gly
225                 230                 235                 240

Thr Lys Val Thr Val Leu Gly
                245

<210>  183
<211>  10
<212>  PRT
<213>  Artificial sequence

<220>
<223>  Synthesized polypeptide

<400>  183

Gly Phe Thr Phe Ser Ser Tyr Ala Met His
1               5                   10

<210>  184
<211>  17
<212>  PRT
<213>  Artificial sequence

<220>
<223>  Synthesized polypeptide

<400>  184

Ala Ile Ser Ser Ser Gly Gly Ser Thr Tyr Tyr Ala Asp Ser Val Lys
1               5                   10                  15

Gly

<210>  185
<211>  12
<212>  PRT
<213>  Artificial sequence

<220>
<223>  Synthesized polypeptide

<400>  185

Asp Arg Arg Gly Ser His Ala Asp Ala Phe Asp Val
1               5                   10

<210>  186
<211>  14
<212>  PRT
<213>  Artificial sequence

<220>
<223>  Synthesized polypeptide

<400>  186

Thr Gly Thr Ser Ser Asp Val Gly Leu Met His Asn Val Ser
1               5                   10


<210>  187
<211>  7
<212>  PRT
<213>  Artificial sequence

<220>
<223>  Synthesized polypeptide

<400>  187

Lys Ser Ser Ser Arg Pro Ser
1               5


<210>  188
<211>  10
<212>  PRT
<213>  Artificial sequence

<220>
<223>  Synthesized polypeptide

<400>  188

Gln Ser Tyr Asp Ser Ser Leu Arg Val Val
1               5                   10


<210>  189
<211>  121
<212>  PRT
<213>  Artificial sequence

<220>
<223>  Synthesized polypeptide

<400>  189

Gln Val Gln Leu Gln Glu Ser Gly Gly Gly Leu Val Gln Pro Gly Arg
1               5                   10                  15


Ser Leu Arg Leu Ser Cys Ala Ala Ser Gly Phe Thr Phe Ser Ser Tyr
            20                  25                  30


Ala Met His Trp Val Arg Gln Ala Pro Gly Lys Gly Leu Glu Trp Val
        35                  40                  45

Ser Ala Ile Ser Ser Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Phe Asp Val Trp Gly
            100                 105                 110

Gln Gly Thr Leu Val Thr Val Ser Ser
        115                 120

<210>  190
<211>  111
<212>  PRT
<213>  Artificial sequence

<220>
<223>  Synthesized polypeptide

<400>  190

Gln Ser Ala Leu Thr Gln Pro Pro Ser Ala Ser Gly Ser Pro Gly Gln
1               5                   10                  15

Ser Val Thr Ile Ser Cys Thr Gly Thr Ser Ser Asp Val Gly Leu Met
            20                  25                  30

His Asn Val Ser Trp Tyr Gln Gln Tyr Pro Gly Lys Ala Pro Lys Leu
        35                  40                  45

Leu Ile Tyr Lys Ser Ser Ser Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Gly Ala Asp Tyr Tyr Cys Gln Ser Tyr Asp Ser Ser
                85                  90                  95

Leu Arg Val Val Phe Gly Gly Gly Thr Lys Val Thr Val Leu Gly
            100                 105                 110

<210>  191
<211>  247
<212>  PRT

<213>  Artificial sequence

<220>
<223>  Synthesized polypeptide

<400>  191

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ala Ile Ser Ser Ser Gly Arg Ser Thr Tyr Tyr Ala Asp Ser Val
    50                  55                  60

Glu Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Leu Asn Val Trp Gly
            100                 105                 110

Gln Gly Thr Leu Val Thr Val Ser Ser Gly Gly Gly Gly Ser Gly Gly
        115                 120                 125

Gly Gly Ser Gly Gly Gly Gly Ser Gln Ser Ala Leu Thr Gln Pro Pro
    130                 135                 140

Ser Ala Ser Gly Ser Pro Gly Gln Ser Val Thr Ile Ser Cys Thr Gly
145                 150                 155                 160

Thr Ser Ser Asp Val Gly Gly Tyr Asn Tyr Val Ser Trp Tyr Gln Gln
                165                 170                 175

Tyr Pro Gly Lys Ala Pro Lys Leu Leu Ile Tyr Gly Asn Ser Asn Arg
            180                 185                 190

Pro Ser Gly Val Pro Asp Arg Phe Ser Gly Ser Lys Ser Gly Thr Ser
        195                 200                 205

Ala Ser Leu Ala Ile Thr Gly Leu Gln Ala Glu Asp Gly Ala Asp Tyr
    210                 215                 220

Tyr Cys Gln Ser Tyr Asp Ser Ser Leu Arg Val Val Phe Gly Gly Gly
225                 230                 235                 240

Thr Lys Val Thr Val Leu Gly
                245

<210>  192
<211>  10
<212>  PRT
<213>  Artificial sequence

<220>
<223>  Synthesized polypeptide

<400>  192

Gly Phe Thr Phe Ser Ser Tyr Ala Met His
1               5                   10

<210>  193
<211>  17
<212>  PRT
<213>  Artificial sequence

<220>
<223>  Synthesized polypeptide

<400>  193

Ala Ile Ser Ser Ser Gly Arg Ser Thr Tyr Tyr Ala Asp Ser Val Glu
1               5                   10                  15

Gly

<210>  194
<211>  12
<212>  PRT
<213>  Artificial sequence

<220>
<223>  Synthesized polypeptide

<400>  194

Asp Arg Arg Gly Ser His Ala Asp Ala Leu Asn Val
1               5                   10

<210>  195
<211>  14
<212>  PRT
<213>  Artificial sequence

<220>
<223>  Synthesized polypeptide

<400>  195

Thr Gly Thr Ser Ser Asp Val Gly Gly Tyr Asn Tyr Val Ser
1               5                   10

<210>  196
<211>  7
<212>  PRT
<213>  Artificial sequence

<220>
<223>  Synthesized polypeptide

<400>  196

Lys Ser Ser Ser Arg Pro Ser
1               5

<210>  197
<211>  10
<212>  PRT
<213>  Artificial sequence

<220>
<223>  Synthesized polypeptide

<400>  197

Gln Ser Tyr Asp Ser Ser Leu Arg Val Val
1               5                   10

<210>  198
<211>  121
<212>  PRT
<213>  Artificial sequence

<220>
<223>  Synthesized polypeptide

<400>  198

Gln Val Gln Leu Gln Glu Ser Gly Gly Gly Leu Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ser Ala Ile Ser Ser Ser Gly Arg Ser Thr Tyr Tyr Ala Asp Ser Val
    50                  55                  60

Glu Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Arg Gly Ser His Ala Asp Ala Leu Asn Val Trp Gly
            100                 105                 110

Gln Gly Thr Leu Val Thr Val Ser Ser
        115                 120

<210>  199
<211>  111
<212>  PRT
<213>  Artificial sequence

<220>
<223>  Synthesized polypeptide

<400>  199

Gln Ser Ala Leu Thr Gln Pro Pro Ser Ala Ser Gly Ser Pro Gly Gln
1               5                   10                  15

Ser Val Thr Ile Ser Cys Thr Gly Thr Ser Ser Asp Val Gly Gly Tyr
            20                  25                  30

Asn Tyr Val Ser Trp Tyr Gln Gln Tyr Pro Gly Lys Ala Pro Lys Leu
        35                  40                  45

Leu Ile Tyr Gly Asn Ser Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Gly Ala Asp Tyr Tyr Cys Gln Ser Tyr Asp Ser Ser
                85                  90                  95

Leu Arg Val Val Phe Gly Gly Gly Thr Lys Val Thr Val Leu Gly
            100                 105                 110

<210>  200
<211>  243
<212>  PRT
<213>  Artificial sequence

<220>
<223>  Synthesized polypeptide

<400>  200

Gln Val Gln Leu Gln Gln Ser Gly Thr Glu Leu Val Arg Pro Gly Ala
1               5                   10                  15

Ser Val Lys Leu Ser Cys Lys Ala Leu Gly Tyr Thr Phe Thr Asp Tyr
            20                  25                  30

Glu Met His Trp Val Lys Gln Thr Pro Val His Gly Leu Glu Trp Ile
        35                  40                  45

Gly Ala Ile His Pro Gly Ser Gly Asp Thr Ala Tyr Asn Gln Arg Phe
    50                  55                  60

Lys Gly Lys Ala Thr Leu Thr Ala Asp Lys Ser Ser Ser Thr Ala Tyr
65                  70                  75                  80

Met Glu Tyr Ser Ser Leu Thr Ser Glu Asp Ser Ala Val Tyr Tyr Cys
                85                  90                  95

Thr Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Val Val Met Thr Gln Thr Pro Leu Ser Leu Pro Val Ser
    130                 135                 140

Leu Gly Asp Gln Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Lys Leu Leu Ile Tyr Lys Val Ser Asn Arg Phe Ser Gly
            180                 185                 190

Val Pro Asp Arg Phe Ser Gly Arg Gly Ser Gly Thr Asp Phe Thr Leu
        195                 200                 205

Lys Ile Ser Arg Val Glu Ala Glu Asp Leu Gly Val Tyr Phe Cys Ser
    210                 215                 220

Gln Ser Ile Tyr Val Pro Tyr Thr Phe Gly Gly Gly Thr Lys Leu Glu
225                 230                 235                 240

Ile Lys Arg

<210>  201
<211>  5

<212>  PRT
<213>  Artificial sequence

<220>
<223>  Synthesized polypeptide

<400>  201

Asp Tyr Glu Met His
1               5


<210>  202
<211>  17
<212>  PRT
<213>  Artificial sequence

<220>
<223>  Synthesized polypeptide

<400>  202

Ala Ile His Pro Gly Ser Gly Asp Thr Ala Tyr Asn Gln Arg Phe Lys
1               5                   10                  15


Gly




<210>  203
<211>  6
<212>  PRT
<213>  Artificial sequence

<220>
<223>  Synthesized polypeptide

<400>  203

Phe Tyr Ser Tyr Ala Tyr
1               5


<210>  204
<211>  16
<212>  PRT
<213>  Artificial sequence

<220>
<223>  Synthesized polypeptide

<400>  204

Arg Ser Ser Gln Ser Leu Val His Ser Asn Gly Asn Thr Tyr Leu Gln
1               5                   10                  15


<210>  205
<211>  7
<212>  PRT
<213>  Artificial sequence

<220>
<223>  Synthesized polypeptide

<400>  205

Lys Val Ser Asn Arg Phe Ser
1               5


<210>  206
<211>  10
<212>  PRT
<213>  Artificial sequence

<220>
<223>  Synthesized polypeptide

<400>  206

Ser Gln Ser Ile Tyr Val Pro Tyr Thr Phe
1               5                   10


<210>  207
<211>  115
<212>  PRT
<213>  Artificial sequence

<220>
<223>  Synthesized polypeptide

<400>  207

Gln Val Gln Leu Gln Gln Ser Gly Thr Glu Leu Val Arg Pro Gly Ala
1               5                   10                  15


Ser Val Lys Leu Ser Cys Lys Ala Leu Gly Tyr Thr Phe Thr Asp Tyr
            20                  25                  30


Glu Met His Trp Val Lys Gln Thr Pro Val His Gly Leu Glu Trp Ile
        35                  40                  45


Gly Ala Ile His Pro Gly Ser Gly Asp Thr Ala Tyr Asn Gln Arg Phe
    50                  55                  60


Lys Gly Lys Ala Thr Leu Thr Ala Asp Lys Ser Ser Ser Thr Ala Tyr
65                  70                  75                  80


Met Glu Tyr Ser Ser Leu Thr Ser Glu Asp Ser Ala Val Tyr Tyr Cys
                85                  90                  95


Thr Arg Phe Tyr Ser Tyr Ala Tyr Trp Gly Gln Gly Thr Leu Val Thr
            100                 105                 110


Val Ser Ala
        115

<210>  208
<211>  113
<212>  PRT
<213>  Artificial sequence

<220>
<223>  Synthesized polypeptide

<400>  208

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu Gln Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Arg Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Ile Tyr Val Pro Tyr Thr Phe Gly Gly Gly Thr Lys Leu Glu Ile Lys
            100                 105                 110

Arg